(19) Europäisches Patentamt
European Patent Office
Office européen des brevets

(11) **EP 3 209 793 B1**

## (12) FASCICULE DE BREVET EUROPEEN

(45) Date de publication et mention de la délivrance du brevet:
**06.04.2022 Bulletin 2022/14**

(21) Numéro de dépôt: **15791712.1**

(22) Date de dépôt: **23.10.2015**

(51) Classification Internationale des Brevets (IPC):
*C12Q 1/24* *(2006.01)*  *C12N 13/00* *(2006.01)*
*G01N 1/40* *(2006.01)*  *G01N 15/10* *(2006.01)*
*G01N 15/14* *(2006.01)*  *G01N 27/447* *(2006.01)*
*G01N 29/22* *(2006.01)*

(52) Classification Coopérative des Brevets (CPC):
**B01L 3/502753; B01L 3/502776; C12N 1/02; C12N 13/00; C12Q 1/24; G01N 1/40; G01N 15/0255; G01N 27/447;** B01L 3/502715; B01L 2300/0829; B01L 2300/0864; B01L 2300/0867; B01L 2300/0887; B01L 2400/0436; G01N 1/44;  (Cont.)

(86) Numéro de dépôt international:
**PCT/FR2015/052849**

(87) Numéro de publication internationale:
**WO 2016/062975 (28.04.2016 Gazette 2016/17)**

(54) **METHODE ET DISPOSITIFS DE TRAITEMENT D'ECHANTILLONS BIOLOGIQUES**

VERFAHREN UND VORRICHTUNG ZUR BEHANDLUNG VON BIOLOGISCHEN PROBEN

METHOD AND DEVICES FOR TREATING BIOLOGICAL SAMPLES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **24.10.2014 FR 1460253**

(43) Date de publication de la demande:
**30.08.2017 Bulletin 2017/35**

(73) Titulaire: **Biomérieux**
**69280 Marcy-L'Etoile (FR)**

(72) Inventeurs:
• **BROYER, Patrick**
**F-38500 Saint Cassien (FR)**
• **PATEL, Pradip**
**London SW17 7LT (GB)**
• **PAMME, Nicole**
**Beverley HU17 7JL (GB)**
• **BISCEGLIA, Emilie**
**F-69009 Lyon (FR)**

(74) Mandataire: **bioMérieux PI Groupement mandataires**
**bioMérieux**
**69280 Marcy l'Etoile (FR)**

(56) Documents cités:
WO-A1-02/072234    WO-A1-2008/122051
WO-A1-2014/046605    US-A1- 2004 069 717

• THOMAS LAURELL ET AL: "Chip integrated strategies for acoustic separation and manipulation of cells and particles", CHEMICAL SOCIETY REVIEWS, CHEMICAL SOCIETY, LONDON, GB, vol. 36, no. 3, 1 mars 2007 (2007-03-01), pages 492-506, XP008117651, ISSN: 0306-0012, DOI: 10.1039/B601326K
• PER AUGUSTSSON ET AL: "Decomplexing biofluids using microchip based acoustophoresis", LAB ON A CHIP, vol. 9, no. 6, 1 janvier 2009 (2009-01-01), pages 810-818, XP055183122, ISSN: 1473-0197, DOI: 10.1039/B811027A

EP 3 209 793 B1

• LUKÁS KRÁSNÝ ET AL: "Identification of bacteria using mass spectrometry techniques", INTERNATIONAL JOURNAL OF MASS SPECTROMETRY, vol. 353, 1 novembre 2013 (2013-11-01), pages 67-79, XP055209073, ISSN: 1387-3806, DOI: 10.1016/j.ijms.2013.04.016

• A. VAN BELKUM ET AL: "Next-Generation Antimicrobial Susceptibility Testing", JOURNAL OF CLINICAL MICROBIOLOGY, vol. 51, no. 7, 1 juillet 2013 (2013-07-01), pages 2018-2024, XP055209077, ISSN: 0095-1137, DOI: 10.1128/JCM.00313-13

• LEHTINEN J ET AL: "Real-time monitoring of antimicrobial activity with the multiparameter microplate assay", JOURNAL OF MICROBIOLOGICAL METHODS, ELSEVIER, AMSTERDAM, NL, vol. 66, no. 3, 1 septembre 2006 (2006-09-01), pages 381-389, XP027926916, ISSN: 0167-7012 [extrait le 2006-09-01]

(52) Classification Coopérative des Brevets (CPC):
(Cont.)
G01N 2001/4094

## Description

La méthode et les dispositifs selon l'invention présentent un intérêt pour le traitement d'échantillons biologiques, notamment dans le domaine des tests sur des échantillons provenant de sang total, d'hémocultures ou de fluides corporels.

Les procédés de préparations d'échantillons biologiques ou chimiques complexes font appels à des opérations de séparations des particules, cellules ou molécules afin de rendre possible ou de faciliter l'analyse de particules, cellules ou molécules d'intérêts, susceptibles d'être contenues dans l'échantillon. Un objectif de ces procédés de préparations d'échantillons est donc de séparer et/ou de concentrer les particules, cellules ou molécules d'intérêt vis à vis d'éléments non-spécifiques pour permettre par exemple leur capture et/ou leur détection.

Parmi les procédés classiques de séparation de particules, la centrifugation, la filtration, la chromatographie ou l'électrophorèse ont étés largement utilisés pour la préparation d'échantillon biologiques ou chimiques complexes. Cependant, ces méthodes sont souvent fastidieuses à mettre en œuvre et ne permettent pas de traiter de grands volumes d'échantillons. Par exemple, les procédés de filtration obligent de traiter un volume défini d'échantillon puis de procéder à une opération de nettoyage du filtre pour éviter tout bouchage. Cette technique ne permet donc pas de traiter un échantillon biologique ou chimique complexe en flux continu. D'autre part, des techniques classiques de préparation d'échantillon biologique, notamment la lyse chimique ou la lyse sélective, ne permettent pas d'assurer ou peuvent limiter la viabilité des cellules vivantes tels que des bactéries suite à la préparation de l'échantillon, et peuvent donc impacter la qualité des étapes de capture, de remise en croissance et/ou de détection ultérieures.

Des procédés de préparations classiques d'échantillons sanguins de grands volumes, supérieurs à 10mL, et susceptibles de contenir des microorganismes, utilisent pour la plupart une lyse chimique suivi d'une centrifugation. Ces procédés utilisent notamment des détergents comme tampon de lyse sélective des cellules humaines. L'utilisation de détergents est cependant connue pour avoir un impact sur le stress bactérien et entraine un allongement du temps de remis en phase exponentielle des bactéries extraites avec ces procédés. Cette étape est importante et nécessaire pour réaliser une détection des microorganismes mais peut également tuer les microorganismes présents en faible concentration ou rendre leur reprise de croissance moins rapide.

Les procédés d'extraction classiques d'échantillons sanguins par centrifugation, tube à gel ou filtration nécessitent également plusieurs étapes réalisées généralement de manière manuelle et demandant un équipement significatif lié à l'utilisation de plusieurs étapes d'élimination de surnageant et à l'ajout de différents tampons de lavages.

Des procédés de séparation de particules, cellules ou molécules en flux continu utilisant des dispositifs microfluidiques peuvent permettre de traiter des grands volumes d'échantillon et ce en introduisant continuellement l'échantillon dans le dispositif microfluidique. Le principe d'acoustophorèse dans un dispositif microfluidique a par exemple déjà été appliqué pour la séparation de bactérie Escherichia coli dans un échantillon contenant des cellules mononuclées de sang périphérique [1]. Cependant, ce procédé ne peut être employé que sur un échantillon de sang fortement dilué ce qui le rend inapplicable à une analyse d'échantillon sanguins en flux continu.

Des dispositifs et méthodes connus de traitement d'échantillons par acoustophorèse, considérés commes état de l'art le plus proche, sont décrits dans - WO 2008122051 A1; - THOMAS LAURELL ET AL: "Chip integrated stratégies for acoustic séparation and manipulation of cells and particles", CHEMICAL SOCIETY REVIEWS, ol. 36, no. 3, 1 mars 2007 (2007-03-01), pages 492-506, XP008117651, ISSN: 0306-0012; and- PER AUGUSTSSON ET AL: "Decomplexing biofluids using microchip based acoustophoresis", LAB ON A CHIP, vol. 9, no. 6, 1 janvier 2009 (2009-01-01), pages 810-818, XP055183122, ISSN: 1473-0197.

Eu égard aux problématiques présentées ci-dessus, un objectif de la présente invention vise à mettre au point un dispositif et son/ses procédé(s) associé(s) permettant :

- de proposer un procédé simplifié et universel permettant de traiter et d'analyser des échantillons biologiques de différents types, notamment des échantillons de sang total, provenant d'hémocultures, de fluides corporels stériles enrichi en milieu de culture ou de fluides corporels non stériles

- de proposer un procédé de traitement d'échantillon issu d'hémoculture ou un échantillon de fluide corporel stérile n'utilisant pas de tampon de lyse sélective et/ou n'utilisant pas de dilution préalable de l'échantillon avant son introduction dans le dispositif d'acoustophorèse et permettant l'extraction de(s) microorganisme(s) contenus dans l'échantillon

- de proposer une méthode de traitement garantissant la viabilité des microorganismes.

- de proposer un procédé permettant une remise en phase de croissance plus rapide des agents pathogènes présents dans l'échantillon biologique

- de décomplexifier et/ou de concentrer des particules, cellules ou molécules d'intérêt a des débits élevés, notamment des débits supérieur à $1\mu$L/min par canal de séparation.

- de limiter l'obtention d'éléments non-spécifiques susceptibles de fausser le résultat de l'analyse in fine,

- de proposer une méthode de traitement d'échantillon biologique facilitant l'obtention d'une identification de l'espèce microbiologique et/ou d'un profil d'antibiogramme

- de limiter au maximum les risques de contamination de l'échantillon et/ou de l'environnement et/ou du technicien de laboratoire,

- de simplifier le protocole de traitement d'échantillons biologique, afin de réduire le temps opérateur, le nombre de matériels et de consommables utilisés, les risques d'erreurs et améliorer la reproductibilité,

- de proposer des dispositifs et une méthode compatible avec l'ensemble des méthodes d'analyse connues (microbiologie, culture, virologie, bactériologie, immunoessais, métaséquençage, PCR...).

- de proposer plusieurs dispositif microfluidiques, supports et systèmes associés susceptibles de mettre en œuvre des méthodes de traitement d'échantillon biologiques par acoustophorèse, notamment des méthodes selon l'invention

La présente invention concerne une méthode permettant la décomplexification d'échantillons biologiques, notamment d'échantillons sanguins, d'échantillons issus d'hémoculture, ou encore d'échantillons de fluides corporels, permettant ainsi l'analyse de microorganismes contenus ou susceptibles d'être contenus dans l'échantillon biologique.

Afin d'atteindre cet objectif et de palier aux inconvénients des méthodes précitées, l'invention concerne une méthode de traitement d'un échantillon biologique susceptible de contenir une ou des espèce(s) biologique(s) d'intérêt(s) , selon la revendication 1.

Par particules non-spécifiques, on entend des particules pouvant gêner ou limiter la capture et/ou la détection d' espèce(s) biologique(s) d'intérêt. De telles particules peuvent être, à titre d'exemple les éléments figurés du sang ou cellules sanguines telles que des hématies, des globules blancs, des plaquettes, des cellules eucaryotes, des agrégats de lipides ou d'érythrocytes,...

A la suite de cette méthode, au moins un des orifices de sortie du dispositif présente un échantillon enrichi et décomplexifié, c'est à dire que sa concentration en particules non-spécifiques est réduite. Cette étape de décomplexification est donc analogue a une étape de purification dans la mesure où elle facilite la détection et l'analyse ultérieure de ou des espèce(s) biologique(s) d'intérêt(s) présente(s) dans l'échantillon.

A la suite de cette méthode, au moins un des orifices de sortie du dispositif, différent de celui comprenant l'échantillon enrichi et décomplexifié, présente un échantillon concentré, c'est à dire que sa concentration en particules non-spécifiques est augmentée.

En effet, la majorité des particules non-spécifiques est séparée par l'étape de séparation par acoustophorèse, les particules non-spécifiques telles que les éléments figurés du sang présentant un volume et/ou une densité plus importante et/ou une compressibilité moins importante que les espèce(s) biologique(s) d'intérêt, celles-ci sont donc plus sujettes à la pression de radiation acoustique et se retrouvent concentrés dans un des orifices de sortie. Au contraire, les espèce(s) d'intérêt subissent moins l'effet de l'onde acoustique de par leur taille, leur densité et leur compressibilité. Celles-ci vont donc être réparties majoritairement par la circulation du fluide les contenant lors de leur introduction dans le dispositif. En fonction de la géométrie du dispositif et l'orientation de l'onde acoustique dans le canal de séparation, les particules non-spécifiques peuvent notamment être séparées en direction d'un orifice de sortie dont le canal d'acheminement présente un axe semblable et dans le prolongement de celui du canal de séparation. Cette séparation vers un orifice de sortie « central », dont le canal présente un axe semblable à celui du canal de séparation peut être réalisée en appliquant une onde acoustique présentant une nœud de pression sensiblement centré sur l'axe longitudinal du canal de séparation. La répartition des espèce(s) d'intérêt(s), peut, en fonction de leur volume, de leur densité et de leur compressibilité, être équitable entre les orifices de sortie ou concentrée dans au moins un orifice de sortie, différent de celui où se concentre les particules non-spécifiques.

L'étape d'enrichissement par incubation en présence d'un milieu de culture peut employer des milieux de culture spécifiques ou non spécifiques. A titre d'exemple de milieux de culture on peut citer le BHI (« brain heart infusion broth» en langue anglaise). Des milieux de culture en bouteille tel que Bact/ALERT® Standard Aerobic (bioMérieux, ref 256789, France), BacT/ALERT® Standard Anaerobic (bioMérieux, ref 259790, France), BacT/ALERT ®FA FAN®Aerobic (bioMérieux, ref 259791, France), BacT/ALERT® FN FAN® Anaerobic (bioMérieux, ref 259793, France), BacT/ALERT® PF Pediatric FAN® (bioMérieux, ref 259794, France), peuvent également être employés.

Dans le cas de traitement de fluide corporels non stériles, tel que des lavages broncho alvéolaires, l'étape d'incubation n'est pas nécessaire. En effet, la concentration en microorganismes de ces échantillons est comprise environ entre $10^4$ CFU par ml et $10^5$ CFU par ml avec $10^6$ cellules eucaryotes par millilitres. L'intérêt est d'éliminer par la méthode de traitement selon l'invention tout l'ADN humain qui est présent dans un ratio de 20000:1 par rapport à l'ADN microbien. La méthode de préparation permet ainsi la réalisation d'une étape d'analyse de l'ADN microbien à la suite de l'étape de décomplexification, notamment une étape de séquençage ou d'amplification de type PCR (Polymerase Chain Reaction) ou qPCR (quantitative Polymerase Chain Reaction). Pour ces échantillons, l'étape de décomplexification permet ainsi de séparer la flore naturelle des cellules eucaryotes, pour n'analyser que l'ADN microbien lors de l'étape d'analyse. Afin de préparer l'échantillon décomplexifié pour cette étape d'analyse de l'ADN microbien, celui-ci subit une étape de lyse mécanique ou enzymatique suivi d'une étape de purification des acides nucléiques.

L'intérêt est donc un gain de temps vis à vis de protocoles classiques de lyse différentielle et de digestion des acides nucléiques et une automatisation plus facile permettant de s'affranchir d'un besoin en main d'œuvre qualifié. L"étape de décomplexification par acoustophorèse permet éventuellement d'extraire les virus, alors qu'une méthode classique de lyse sélective ne permet pas de récupérer les virus à enveloppe, ceux-ci étant lysés comme les cellules eucaryotes.

Dans le cas de traitement d'un échantillon de sang total, l'étape d'incubation en milieu de culture peut être réduite par 2 à 4 à une température comprise entre 35°C et 37°C, afin d'obtenir une quantité d'espèce(s) biologique(s) d'intérêt(s) comprise environ entre $10e^2$ à $10e^4$ UFC/mL, cette quantité étant suffisante pour déterminer la présence d'une espèce biologique dans l'échantillon. D'autre part, cette étape permet aux espèce(s) biologique(s) d'intérêt présentes dans l'échantillon biologique d'atteindre une phase de croissance exponentielle. Ce stade de croissance permet notamment d'améliorer le rendement d'un test de sensibilité aux antibiotiques réalisé sur l(es)' espèce(s) biologique(s) d'intérêt, le ralentissement ou l'inhibition de leur croissance par l'antibiotique étant plus facilement observable.

UFC signifie Unité Formant Colonie, ou CFU en langue anglaise pour « Colony Forming Unit » .

La réalisation d'une étape de détermination de la présence d'une espèce biologique dans l'échantillon, peut notamment être réalisé par des systèmes commercialisés par la demanderesse tel que le BacT/ALERT® VIRTUO™ (bioMérieux, France). Ce système permet de détecter la présence d'espèce(s) biologique(s) d'intérêt(s) telle que des microorganismes dans des échantillons sanguins quand celle-ci atteint une concentration comprise entre $10e^7$ et $10e^9$ UFC/mL. Pour cela un volume d'échantillon est dispensé dans un bouteille de culture contenant un milieu de culture liquide puis placé dans un incubateur. Différentes types de bouteilles permettent de favoriser la croissance de microorganismes particuliers. La bouteille est surveillée durant l'incubation, celle-ci pouvant durer jusqu'à 72hr, et un signal de croissance positive est délivré si la concentration atteint un seuil déterminé.

Des solutions tampons pouvant être utilisées sont par exemple et de façon non-limitative :

- Des tampons neutres, par exemple de l'eau, une solution de NaCl à 0,85%, Phosphate Buffer Saline (PBS),....
- Des tampons de remise en croissance non-spécifiques (génériques) de microorganismes. A titre d'exemple ces tampons peuvent être de type BHI (« brain heart infusion broth» en langue anglaise), TSB (bouillon Trypticase Soja), BPW (eau peptonée). Ces tampons permettent notamment de placer les microorganismes directement dans un milieu propice à la reprise de croissance avant une étape d'analyse par cytométrie.
- Des tampons de remise en croissance spécifiques de microorganismes. A titre d'exemple ces tampons peuvent être de type : Fraser 1/2, bouillon LX (Listeria Xpress broth ; Ref. 42626 bioMérieux, France), bouillon LMX (Listeria Monocytogenes Xpress broth ; bioMérieux, France) ou bouillon SX (Salmonella Xpress broth ; Ref. 42118 bioMérieux, France)
- Des tampons de densité soit inférieure soit supérieure à la densité de l'échantillon biologique, la densité de l'échantillon est supposée proche de 1. A titre d'exemple ces tampons peuvent comprendre : de la silice colloïdale (silanisée), du Iohexol (Nycodenz®), du Iodixanol (Opti-Pre™), du Ficoll 400, du Dextran 70, du Dextran 200, du Dextran 500, du sucrose, du chlorure de césium, des fluides de perfluorocarbone, des huiles minérales, des huiles de silicone, des huiles pour objectifs de microscope à immersion, du Pluronic® F127, de l'oxyde de Polyethylene, de l'alcool polyvinylique, de l'Hydroxypropylmethyl cellulose, de la gomme de xanthane.
- Un autre exemple de tampon pouvant être utilisé est un tampon isotonique biocompatible tel que du sérum physiologique ajusté avec un ratio volumique de 10% v/v, 5%v/v, préférentiellement 1%v/v de Percoll® (Sigma Alldrich). Le Percoll® comprend des particules de silice de 15 à 30 nm de diamètre enrobée de polyvinyle-pyrrolidone (PVP).

Le dispositif employé avec la méthode selon l'invention comprend au moins trois orifices d'entrée et trois canaux d'acheminements de ces orifices au canal de séparation, et deux solutions tampons différentes sont introduites dans deux orifices d'entrée différent. L'utilisation de deux solutions tampons différentes permet de faciliter le passage de certaines particules ou microorganismes d'un liquide introduit vers un autre durant l'étape de séparation par acoustophorèse, en jouant sur les différences de densité entre les solutions tampons introduites.

De façon plus particulière, l'échantillon biologique est introduit dans un dispositif d'acoustophorèse de façon à être acheminé par au moins deux canaux d'acheminement vers le canal de séparation, le dispositif étant agencé pour obtenir un flux laminaire dans le canal de séparation entre les flux d'échantillon et de solution tampon provenant des canaux d'acheminement.

La méthode de traitement selon l'invention comprend une seconde étape de décomplexification comprenant la réalisation d'une seconde étape de séparation dudit échantillon concentré par acoustophorèse de manière à favoriser un taux de récupération plus important de(s) (l') espèce(s) d'intérêt encore présente(s) dans l'échantillon concentré dans au moins un des orifices de sortie dudit dispositif d'acoustophorèse.

Le taux de récupération des espèces biologiques est défini comme le rapport entre le nombre d'espèces biologiques dans l'orifice de sortie considéré sur le nombre total d'espèces biologiques dans tous les orifices de sorties du dispositif.

Cette seconde étape de décomplexification dudit échantillon biologique par acoustophorèse est réalisée sur la partie d'échantillon résultante à la première séparation et contenant majoritairement des particules non-spécifiques. Cette seconde séparation permet d'augmenter le taux de récupération, ou rendement de collection, des microorganismes présents dans l'échantillon biologique en favorisant une seconde fois la concentration des particules non-spécifiques, tel que des éléments figurés du sang, présents dans l'échantillon concentré, dans un des orifices dudit dispositif d'acoustophorèse. En effet les microorganismes entrainés avec les particules non spécifiques lors de la première séparation sont susceptibles d'être séparés par acoustophorèse et de pouvoir ainsi être collectés suite à cette seconde séparation.

Cette seconde étape de décomplexification par acoustophorèse peut être réalisée en réintroduisant l'échantillon concentré dans unique dispositif d'acoustophorèse comprenant au moins deux canaux de séparation successifs.

Alternativement, cette seconde étape de séparation peut être réalisée par les étapes suivantes :

-   l'introduction de tout ou partie de l'échantillon concentré à la suite de la première étape de séparation dans un orifice d'entrée d'un dispositif d'acoustophorèse, ce dispositif étant identique ou différent du premier dispositif utilisé lors de la première étape de séparation

-   l'introduction d'un tampon dans un second orifice d'entrée dudit dispositif d'acoustophorèse, le tampon étant préférentiellement de différente nature et/ou de différente densité par rapport à l'échantillon biologique

-   lesdits orifices d'entrées étant connectés fluidiquement à au moins deux orifices de sortie par un canal de séparation,

-   le tampon et l'échantillon étant introduit à des débits respectifs aptes à générer un flux laminaire dans le canal de séparation,

-   la réalisation d'une seconde étape de séparation dudit échantillon décomplexifié par acoustophorèse de manière à favoriser un taux de récupération plus important des espèces biologiques encore présentes dans l'échantillon concentré dans au moins un des orifices de sortie dudit dispositif d'acoustophorèse.

De façon avantageuse, la méthode de traitement selon l'invention comprend une seconde étape de décomplexification comprenant la réalisation d'une seconde étape de séparation dudit échantillon décomplexifié par acoustophorèse de manière à favoriser un taux de réjection plus important des éléments non-spécifiques, tel que les éléments figurés du sang, présents dans l'échantillon décomplexifié dans au moins un des orifices de sortie dudit dispositif d'acoustophorèse. En effet une quantité résiduelle de particules non spécifiques sont susceptibles d'être entrainées dans l'échantillon décomplexifié avec les microorganismes lors de la première séparation et peuvent ainsi être séparées et concentrées suite à cette seconde étape de décomplexification.

Le taux de réjection des éléments non-spécifiques est défini comme le rapport entre le nombre d'éléments non-spécifiques dans l'orifice de sortie considéré sur le nombre total d'éléments non-spécifiques dans tous les orifices de sorties du dispositif.

Cette seconde étape de décomplexification par acoustophorèse peut être réalisée en réintroduisant l'échantillon décomplexifié dans unique dispositif d'acoustophorèse comprenant au moins deux canaux de séparation successifs.

Alternativement, cette seconde étape de séparation peut être réalisée par les étapes suivantes :

-   l'introduction de tout ou partie de l'échantillon décomplexifié à la suite de la première étape de séparation dans un orifice d'entrée d'un dispositif d'acoustophorèse, ce dispositif étant identique ou différent du premier dispositif utilisé lors de la première étape de séparation
-   l'introduction d'un tampon dans un second orifice d'entrée dudit dispositif d'acoustophorèse, le tampon étant préférentiellement de différente nature et/ou de différente densité par rapport à l'échantillon biologique
-   lesdits orifices d'entrées étant connectés fluidiquement à au moins deux orifices de sortie par un canal de séparation,
-   le tampon et l'échantillon étant introduit à des débits respectifs aptes à générer un flux laminaire dans le canal de séparation,

- la réalisation d'une seconde étape de séparation dudit échantillon décomplexifié par acoustophorèse de manière à favoriser un taux de réjection plus important des particules non-spécifiques tel que les éléments figurés du sang présentes dans l'échantillon décomplexifié dans au moins un des orifices de sortie dudit dispositif d'acoustophorèse

La méthode de traitement selon l'invention comprend une étape supplémentaire d'identification de l'espèce biologique suite à l'obtention d'un échantillon enrichi et décomplexifié. Cette étape est préférentiellement réalisé par une centrifugation et une concentration en une étape de tout ou partie du culot d'échantillon concentré suivi d'une analyse par spectrométrie de masse. Une technique connue est rapide permettant l'identification rapide est l'analyse par un spectromètre MALDI-TOF d'une fraction du concentrât de microorganismes obtenu après centrifugation et concentration. Cette fraction est par exemple de 1μL. Un appareil pouvant être utilisé pour la réalisation de cette étape est le VITEK® MS commercialisé par la demanderesse. L'intérêt de combiner ce type d'identification avec une étape de séparation par acoustophorèse est de pouvoir disposer d'un échantillon décomplexifié pouvant être utilisé pour l'analyse en spectrométrie de masse. L'échantillon décomplexifié peut pour cela être centrifugé puis remis en suspension dans un tampon adapté tel que de l'eau pure. L'étape de centrifugation pouvant être alternativement réalisé directement en présence d'eau déionisé, éthanol et/ou d'acide formique, de matrice HCCA et d'acétonitrile afin d'extraire directement les protéines des microorganismes à déposer sur la plaque d'analyse MALDI-TOF MS. Alternativement, il est possible de procéder à une seconde étape d'acoustophorèse avec un tampon adapté tel que de l'eau pure, compatible avec l'étape d'analyse par spectrométrie, les microorganismes présents dans l'échantillon décomplexifié sont mélangés au tampon par acoustophorèse et se retrouvent alors dans l'échantillon concentré. Cet échantillon concentré peut être directement utilisé pour une analyse en spectrométrie de masse.

Cette étape d'identification peut également être réalisée par étalement sur un milieu de culture. Des milieux de cultures spécifiques ou non-spécifiques peuvent être employés pour cette étape d'identification. De façon préférentielle, des milieux sélectifs tel que des milieux chromogéniques peuvent être employés. L'intérêt de combiner ce type d'identification avec une étape de séparation par acoustophorèse est de pouvoir disposer d'un échantillon décomplexifié pouvant être directement utilisé pour ensemencer le milieu de culture. L'ensemencement du milieu de culture peut être réalisé sans étape intermédiaire, en prélevant l'échantillon décomplexifié dans un orifice de sortie du dispositif.

De façon avantageuse, la méthode de traitement selon l'invention comprend la réalisation d'un test d'antibiogramme de(s) l'espèce(s) biologique(s) d'intérêt suite à l'obtention d'un échantillon enrichi et décomplexifié, préférentiellement réalisée en cytométrie de flux, par test biochimique ou par imagerie précoce des zones inhibitrices obtenues sur milieu de culture à l'aide de disque d'antibiotique ou de bandelettes antibiotiques Etest® (bioMérieux, France). Cette étape peut être ultérieure à une étape d'identification ou commencée en parallèle de l'étape d'identification. Par exemple, dans la mesure ou le test d'antibiogramme comprend une première étape d'incubation de l'échantillon, celle-ci peut être démarrée avant de connaître le résultat de l'étape d'identification.

La réalisation d'un test d'antibiogramme en cytométrie de flux peut être réalisée de plusieurs méthodes. Une première manière consiste à mettre l'espèce(s) biologique(s) d'intérêt en présence d'un un ou plusieurs antibiotique(s) à des concentrations différentes. L'(es) antibiotique(s) d'intérêt sont avantageusement déterminés par le résultat de l'étape d'identification définie précédemment. A la suite d'une étape de marquage fluorescent, définie ultérieurement, les échantillons obtenus sont aspirés successivement dans un cytomètre afin d'obtenir un profil d'antibiogramme en fonction de la moyenne de fluorescence des microorganismes analysés pour chacune des concentrations d'antibiotique.

Ce test permet donc de caractériser de manière fiable et rapide une réponse de(s) l'espèce(s) d'intérêt à la présence de l'antibiotique, une telle réponse étant classifiée couramment selon l'une des trois affirmations suivantes : susceptible, intermédiaire ou résistant. Par « susceptible », on entend qu'une croissance, voire une survie, des espèce(s) d'intérêt en présence de l'antibiotique est impossible à partir d'une certaine concentration d'antibiotique. Par « intermédiaire », on entend qu'une croissance des espèce(s) d'intérêt en présence de l'antibiotique est compromise, à partir d'une certaine concentration. Par « résistant », on entend qu'une croissance des espèce(s) d'intérêt en présence de l'antibiotique est possible.

Un antibiotique au sens de la présente invention peut être remplacé par un antifongique, un antimycobactérien ou un composé analogue.

La réalisation d'un test d'antibiogramme en cytométrie de flux peut être également réalisée selon une seconde méthode. Pour cela un premier comptage, ou dénombrement de(s) l'espèce(s) d'intérêt présente dans une partie de l'échantillon décomplexifié et enrichi est réalisé en cytométrie de flux. Une seconde partie de l'échantillon décomplexifié et enrichi est mise en présence d'un un ou plusieurs antibiotique(s) à des concentrations différentes pendant un temps donné, par exemple pendant 1hr à 35hr. L'(es) antibiotique(s) d'intérêt sont avantageusement déterminés par le résultat de l'étape d'identification définie précédemment. A la suite de ce temps, un second comptage, ou dénombrement de(s) l'espèce(s) d'intérêt présente dans la seconde partie de l'échantillon en présence de(s) antibiotique(s) est réalisé en cytométrie de flux. En fonction du résultat du comptage et des concentrations en antibiotiques, un profil d'antibiogramme est réalisée. Cette méthode permet de s'affranchir d'une étape de marquage fluorescent.

La réalisation d'un test d'antibiogramme de(s) l'espèce(s) biologique(s) d'intérêt peut également être réalisée en déposant par inondation immédiatement après l'extraction par acoustophorèse l'échantillon décomplexifié sur milieu de culture gélosé (en manuel ou par l'intermédiaire d'un automate) et en incubant ces milieux de culture afin de démarrer la croissance des microorganismes. Un fois l'identification connue, préférentiellement par technique de spectrométrie de masse, les disque d'antibiotiques ou la bandelettes antibiotiques Etest® (bioMérieux, France) adéquates sont sélectionnées et déposées sur la/les boite(s) ensemencées avec l'extrait fourni par le dispositif d'acoustophorèse. Le/les milieux gélosés sont incubés durant 1 à 5h en réalisant un suivi par un système d'imagerie adapté permettant une mesure précoce des zones d'inhibitions et ainsi un rendu rapide du résultat de profil d'antibiogramme par classification en trois catégories Susceptible (S), Intermédiaire (I) ou Résistant (R).

La réalisation d'un test d'antibiogramme de(s) l'espèce(s) biologique(s) d'intérêt peut également être réalisée par des tests biochimiques. Des systèmes commercialisé par la demanderesse tel que l'appareil VITEK® 2 permettent ainsi la réalisation d'antibiogramme en milieu liquide de manière automatisée. Pour cela tout ou partie de l'échantillon enrichi et décomplexifié est introduit dans une carte VITEK® à des fins d'analyse.

De façon avantageuse, la méthode de traitement selon l'invention comprend une étape de mesure de la densité optique de l'échantillon enrichi et décomplexifié pendant ou à la suite d'une étape de séparation par acoustophorèse de manière à obtenir un échantillon d'une densité optique définie, préférentiellement comprise entre 0,025 et 0,5 Mc Farland. La mesure de densité optique peut être réalisée dans l' orifice de sortie ou dans un tube de collecte. Cette étape peut notamment être réalisée en diluant l'échantillon enrichi et décomplexifié à l'aide d'une solution tampon, jusqu'à obtenir une densité optique définie. Un échantillon d'une densité optique comprise entre 0,025 et 0,5 Mc Farland peut notamment être utilisé pour une étape d'ultérieure d'incubation permettant aux microorganismes présents dans l'échantillon d'atteindre une phase exponentielle de croissance.

De façon avantageuse, la méthode de traitement selon l'invention comprend une étape d'incubation de l'échantillon décomplexifié et enrichi, préférentiellement entre 30°C et 37°C, préférentiellement pendant une durée de 1 à 5hr, plus préférentiellement de 2hr, l'incubation pouvant être réalisée directement dans l'orifice ou le tube de collection de sortie. Cette étape peut permet aux espèce(s) biologique(s) d'intérêt(s) présentes dans l'échantillon biologique d'atteindre une phase de croissance exponentielle. Ce stade de croissance permet notamment d'améliorer le rendement d'un test de sensibilité aux antibiotiques réalisé sur l(es)' espèce(s) biologique(s) d'intérêt, le ralentissement ou l'inhibition de leur croissance par l'antibiotique étant plus facilement observable.

Préférentiellement, cette étape d'incubation est réalisée en agitant l'échantillon décomplexifié durant l'incubation. Cette agitation permet par exemple de favoriser l'oxygénation de l'échantillon, augmentant ainsi la vitesse de croissance de microorganismes aérobies.

De façon préférentielle et à la suite de l'étape d'incubation, la méthode de traitement selon l'invention comprend une étape de mesure de la densité optique de l'échantillon enrichi et décomplexifié pendant ou à la suite de l'étape d'incubation, préférentiellement après 2hr d'incubation, de manière à stopper ou prolonger l'étape de d'incubation ou à ajuster la dilution de l'échantillon lorsqu'un seuil de densité optique défini est mesuré. Ledit seuil est préférentiellement compris entre 0,025 et 0,63 Mc Farland, plus préférentiellement entre 0,025 et 0,5 Mc Farland, alternativement entre 0,5 et 0,63 Mc Farland. La mesure de densité optique peut être réalisé dans l' orifice de sortie ou dans un tube de collecte. Cette étape peut notamment être réalisée en diluant l'échantillon enrichi et décomplexifié à l'aide d'une solution tampon, jusqu'à obtenir une densité optique définie. Une densité optique comprise entre 0,025 et 0,63 Mc Farland peut notamment permettre la réalisation d'une étape de détermination sensibilité à un antibiotique par analyse en cytométrie de flux. L'échantillon étant alors suffisamment concentré et l'(es) espèce(s) d'intérêt(s) étant en phase exponentielle de croissance.

De façon avantageuse, la méthode de traitement selon l'invention comprend une étape d'analyse de susceptibilité de l'échantillon à un ou plusieurs antibiotique(s), préférentiellement réalisée en cytométrie de flux ou par test biochimique, l(es) antibiotique(s) étant avantageusement déterminé(s) par le résultat de l'étape d'identification de /des espèce(s) de microorganisme(s) présent(es) dans l'échantillon concentré. Le choix des antibiotiques devant être testés étant restreint par le résultat de l'étape d'identification, l' étape d'analyse de susceptibilité de l'échantillon à un ou plusieurs antibiotique(s) peut être réalisée plus rapidement et avec un nombre minimal de manipulations.

De façon avantageuse, à la suite de l'étape de décomplexification, tout ou partie de l'échantillon enrichi et décomplexifié est transféré dans les puits d'une microplaque ou dans différents puits en connexion fluidique avec au moins un des orifices de sortie contenant tout ou partie de l'échantillon, préférentiellement de 3 à 7 puits, chacun des puits pouvant contenir un antibiotique différent et/ou en concentrations différentes. De façon avantageuse, la réalisation d'une étape d'incubation de l'échantillon enrichi et décomplexifié telle que définie précédemment est réalisée directement dans les puits de la microplaque contenant tout ou partie de l'échantillon. Cette étape d'incubation peut notamment durer environ 1hr à 35°C, sans agitation, de manière à laisser suffisamment l'(es) espèce(s) d'intérêt en présence du/des antibiotique(s) et être ainsi capable d'analyser la poursuite, le ralentissement ou l'arrêt de leur croissance, en fonction des différentes concentration d'antibiotique(s).

De façon avantageuse la réalisation d'une étape de d'ajustement de la densité optique de l'échantillon enrichi et décomplexifié telle que définie précédemment est réalisée directement dans les puits de la microplaque contenant tout ou partie de l'échantillon.

De façon avantageuse, chacun des puits est, après un temps d'incubation égal ou supérieur à 1h en présence de(s) (l) antibiotique(s), aspiré successivement pour être analysé en cytométrie de flux, la réalisation d'une étape de d'ajustement de la densité optique de l'échantillon enrichi et décomplexifié étant réalisé avant l'introduction de(s) (l) antibiotique(s).

De façon avantageuse l'étape d'analyse de susceptibilité de l'échantillon à un ou plusieurs antibiotique(s) est réalisée par l'analyse en cytométrie de flux d'un décalage du signal de fluorescence entre microorganismes sensible, intermédiaire ou résistant aux antibiotiques testés avec l'échantillon enrichi et décomplexifié. Pour cela, un étape de marquage par un marqueur fluorescent non-spécifique ou spécifique de la ou des espèce(s) d'intérêt doit être réalisée. Cette étape peut être réalisée avant l'étape d'incubation, durant l'étape d'incubation ou à la suite de l'étape d'incubation. Cette étape de marquage peut durer environ 15 minutes, à une température de 35°C de manière à laisser suffisamment l'(es) espèce(s) d'intérêt en présence du/des marqueur(s) et de les rendre ainsi détectables.

La réalisation d'une étape de marquage par un marqueur fluorescent non-spécifique ou spécifique de la ou des espèce(s) d'intérêt peut être réalisée par : des marqueurs des acides nucléiques comme l'iodure de propidium, le SYTO9 ou encore le SYBR® Green, des marqueurs de potentiel membranaire tel que le DiBAC ou encore des dérivés de la fluorescéine couplés à des enzymes spécifiques des microorganismes d'intérêt, qui, une fois clivés à l'intérieur du microorganisme deviennent fluorescents.

A titre d'exemple, d'autres marqueurs peuvent être utilisés parmi la liste ci-dessous :

- des marqueurs des acides nucléiques : TOTO-3, SYTOX Green, Ethidium Bromide, Hoechst 33258/33342, SYTO 13, Mithramycin, Pyronine Y

- des marqueurs protéiques : FITC, Texas Red (sulforhodamine isothiocyanate), Oregon Green isothiocyanate,

- des marqueurs de fonctions cellulaires : Indo-1, Fura-2, Fluor-3,

- des marqueurs dépendants du PH : BCECF, SNARF-1, DIOC6(3),

- des marqueurs de potentiel membranaire : Oxonol, [DiBAC4(3)], Rhodamine 123, Fun-1,

- des marqueurs lipophiles : Nile Red,

- des lectines couplées à des marqueurs fluorescents

- des oligonucléotides couplés à des marqueurs fluorescents

- des substrats couplés à des fluorochromes

- des anticorps couplés à des fluorochromes.

Cette étape de marquage peut être réalisée en ligne, directement à la suite de l'étape de décomplexification, par la mise en contact de l'échantillon décomplexifié avec une solution contenant des marqueurs fluorescents. Cette mise en contact peut être réalisée directement dans l'orifice de sortie contenant l'échantillon décomplexifié.

A la suite de cette étape de marquage, l'étape d'analyse en cytométrie de flux vise à détecter la présence dudit marqueur fluorescent. Cette étape est préférentiellement réalisée en ligne, directement à la suite de l'étape de marquage. Cette étape peut être réalisée en prélevant l'échantillon décomplexifié dans l'orifice de sortie et en l'introduisant dans un cytomètre de flux ou en l'acheminant directement par un dispositif microfluidique. Avantageusement, la solution tampon introduite dans le dispositif pour la réalisation de l'étape de séparation par acoustophorèse peut être compatible avec une étape d'analyse par cytométrie de flux. De façon préférentielle, cette solution tampon peut comprendre des marqueurs fluorescents afin de réaliser directement le marquage de(s) l' espèce(s) d'intérêt contenue(s) dans l'échantillon biologique.

On entend par échantillon biologique un échantillon de sang total ou un de ces dérivé tel que du sérum, un échantillon issu d'une hémoculture, ou encore un échantillon de fluide corporel stérile ou non stérile, liquide ou visqueux susceptible de contenir une ou plusieurs espèce(s) d'intérêt, tel que des microorganismes. Un échantillon biologique peut également présenter une matrice solide ou semi-solide, en suspension dans un liquide tel qu'une solution tampon. A titre d'exemple de fluides corporels stériles on peut citer des urines, du liquide céphalo rachidien des prélèvements synoviaux. A titre d'exemple de fluides corporels non-stériles on peut citer des lavages broncho alvéolaires.

Par échantillon issu d'une hémoculture, on entend un échantillon sanguin, mis au contact d'un milieu de culture et incubé pendant un laps de temps de manière à favoriser la croissance de microorganismes susceptibles d'être présent dans l'échantillon sanguin. Par hémoculture positive, on entend un échantillon sanguin mis au contact d'un milieu de culture et incubé pendant un laps de temps à l'issu duquel la présence d'un microorganisme dans l'échantillon sanguin est avérée. Cette présence peut généralement être confirmée quand la concentration en microorganismes dans le mélange atteint un seuil, généralement compris entre $10e^7$ et $10e^9$ UFC/mL. Des systèmes commercialisés par la demanderesse tel que le BacT/ALERT® VIRTUO™ sont susceptibles de fournir des hémocultures positives. Ce système permet de détecter la présence de microorganismes dans des échantillons sanguins. Pour cela un volume d'échantillon est dispensé dans un bouteille de culture contenant un milieu de culture liquide puis placé dans un incubateur. Différentes types de bouteilles permettent de favoriser la croissance de microorganismes particuliers. La bouteille est surveillé durant l'incubation, celle-ci pouvant durer jusqu'à 72hr, et un signal de croissance positive est délivré si la concentration atteint un seuil déterminé.

Au sens de la présente invention, le terme microorganisme recouvre les bactéries à Gram positive ou à Gram négative, les levures, les moisissures, les amibes et plus généralement, les organismes unicellulaires, invisibles à l'œil nu, qui peuvent être manipulés et multipliés en laboratoire et pouvant représenter des agents pathogènes, notamment pour l'homme.

Selon un mode préféré de réalisation de l'invention, le microorganisme est une bactérie, à Gram négatif ou positif, une levure, ou une moisissure.

A titre d'exemple de bactéries à Gram positive, on peut citer les bactéries des genres suivants : *Enterococcus, Streptococcus, Lactobacillus, Bifidobacterium, Staphylococcus, Bacillus, Listeria, Clostridium, Mycobacteria, Nocardia, Corynebacteria, Micrococcus* et *Deinococcus.*

A titre d'exemple de bactéries à Gram négative, on peut citer les bactéries des genres suivants : *Escherichia,* notamment *Escherichia Coli O157:H7, Enterobacter, Klebsiella, Salmonella, Proteus, Serratia, Campylobacter*

A titre d'exemple de levures, on peut citer les genres suivants : *Candida, Cryptococcus, Saccharomyces* et *Trichosporon.*

A titre d'exemple de moisissures, on peut citer les genres suivants : *Aspergillus, Penicillium, Cladosporium.*

**Brève description des dessins**

L'invention, sa fonctionnalité, ses applications ainsi que ses avantages seront mieux appréhendés à la lecture de la présente description détaillée qui suit, faite en référence aux figures, dans lesquelles :

- la figure 1 représente une vue de dessus d'un dispositif microfluidique de l'état de l'art, susceptible de mettre en œuvre le procédé selon l'invention,

- la figure 2a représente une vue en coupe longitudinale du dispositif microfluidique de l'état de l'art selon le plan de coupe A-A de la figure 1

- la figure 2b représente une section transversale du canal de séparation 50 selon le plan de coupe B-B de la figure 1

- la figure 3 représente une vue schématique d'un système permettant la mise en œuvre d'une méthode selon la présente invention utilisant un dispositif microfluidique selon l'état de l'art

- la figure 4 représente une vue de dessus d'un premier dispositif microfluidique à deux orifices d'entrée , non couvert par la présente invention.

- la figure 5a représente une vue en coupe B-B du dispositif microfluidique selon la figure 4

- la figure 5b représente un mode de réalisation d'un orifice d'entrée vue en coupe A-A d'un orifice d'entrée du dispositif microfluidique selon la figure 4.

- la figure 6a représente une vue de dessus d'un second dispositif microfluidique à deux orifices d'entrée , non couvert par la présente invention.

- la figure 7 6b représente une vue de dessus d'un troisième dispositif microfluidique à deux orifices d'entrée , non couvert par la présente invention.

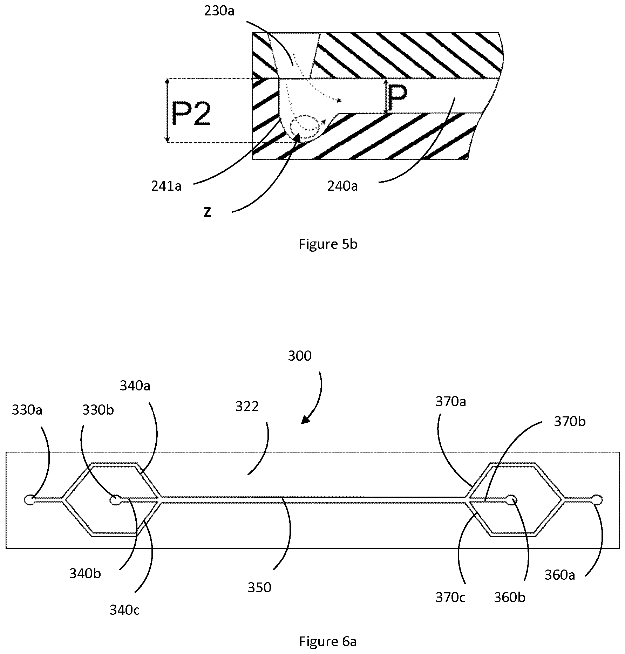

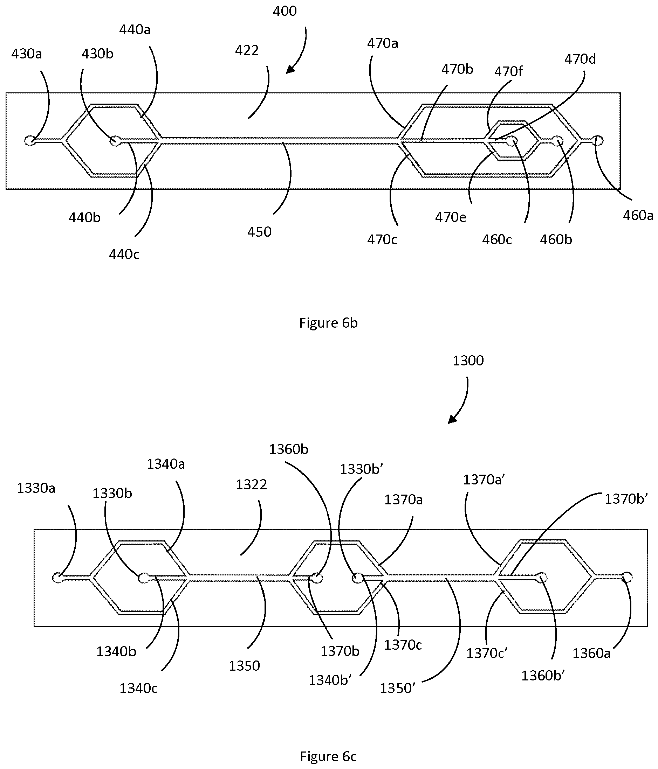

- la figure 6c représente une vue de dessus d'un quatrième dispositif microfluidique à trois orifices d'entrée selon l'invention, susceptible de mettre en œuvre le procédé selon l'invention,

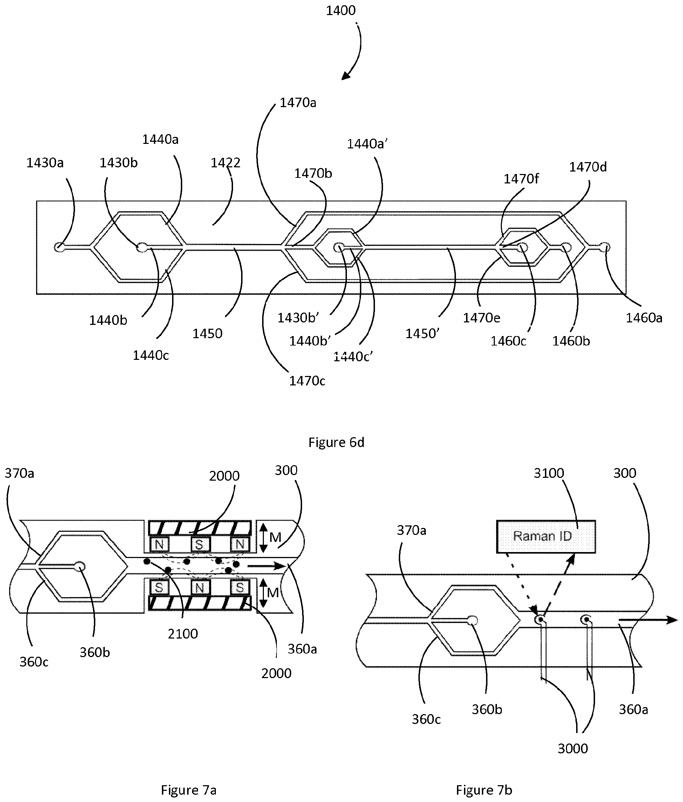

- la figure 6d représente une vue de dessus d'un cinquième dispositif microfluidique à trois orifices d'entrée selon l'invention, susceptible de mettre en œuvre le procédé selon l'invention,

- la figure 7a représente une première alternative de réalisation des orifices de sortie des dispositifs selon l'invention

- la figure 7a représente une seconde alternative de réalisation des orifices de sortie des dispositifs selon l'invention

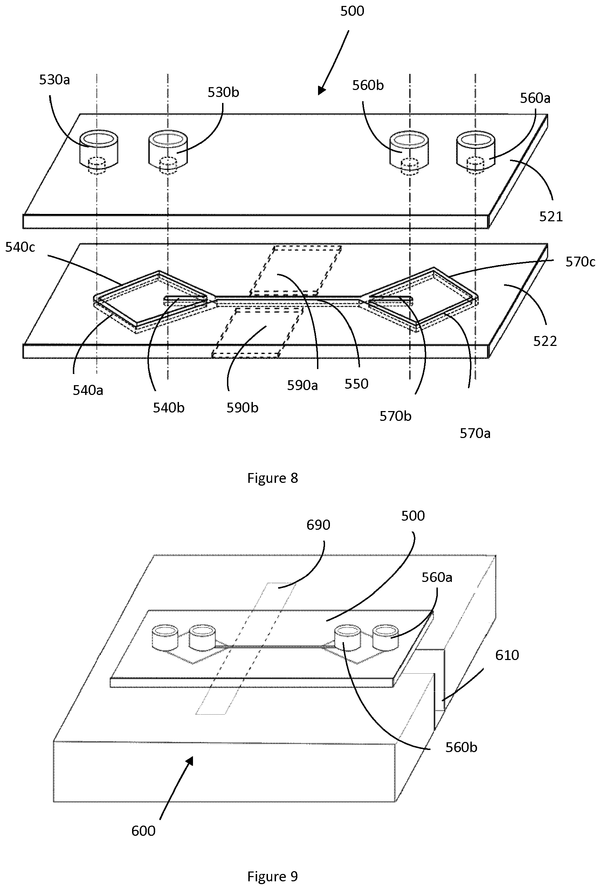

- la figure 8 représente une vue en perspective d'un sixième dispositif microfluidique à deux orifices d'entrée , non couvert par la présente invention.

- la figure 9 représente une vue en perspective d'un premier support pour dispositifs microfluidiques , non couvert par la présente invention.

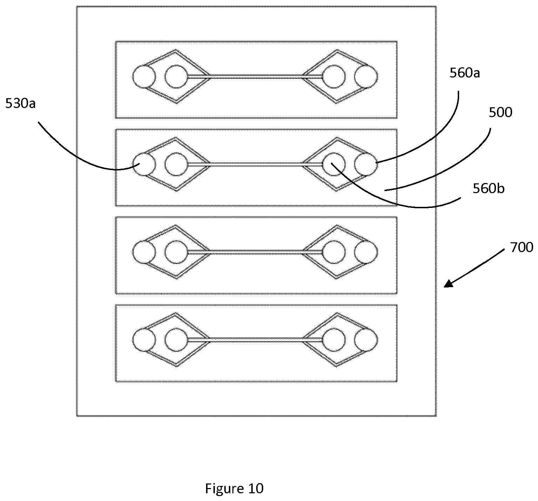

- la figure 10 représente une vue en perspective d'un second support pour dispositifs microfluidiques non couvert par la présente invention,

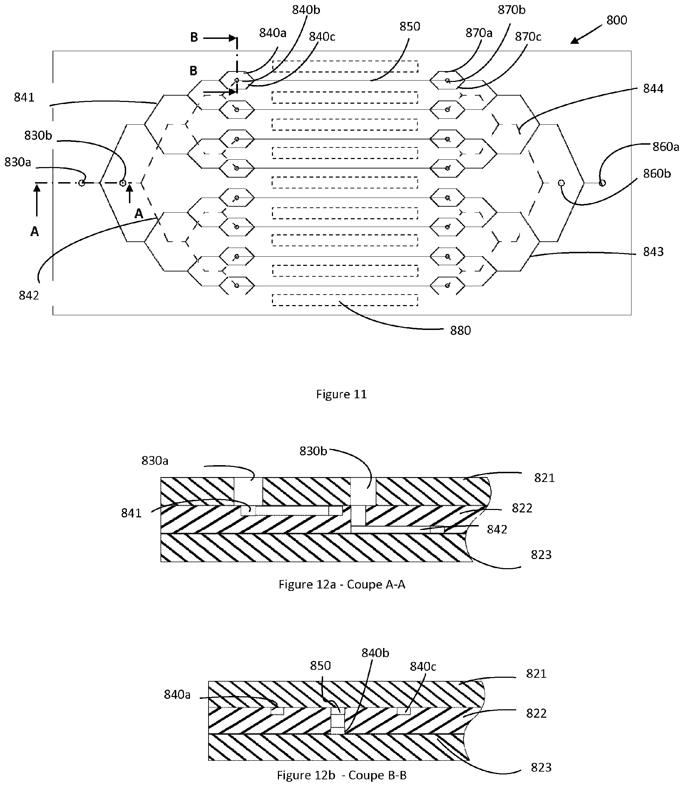

- la figure 11 représente unde vue de dessus d'un cinquième dispositif microfluidique à deux orifices non couvert par la présente invention,

- la figure 12a représente une coupe partielle selon le plan A-A de la figure 11

- la figure 12b représente une coupe partielle selon le plan B-B de la figure 11

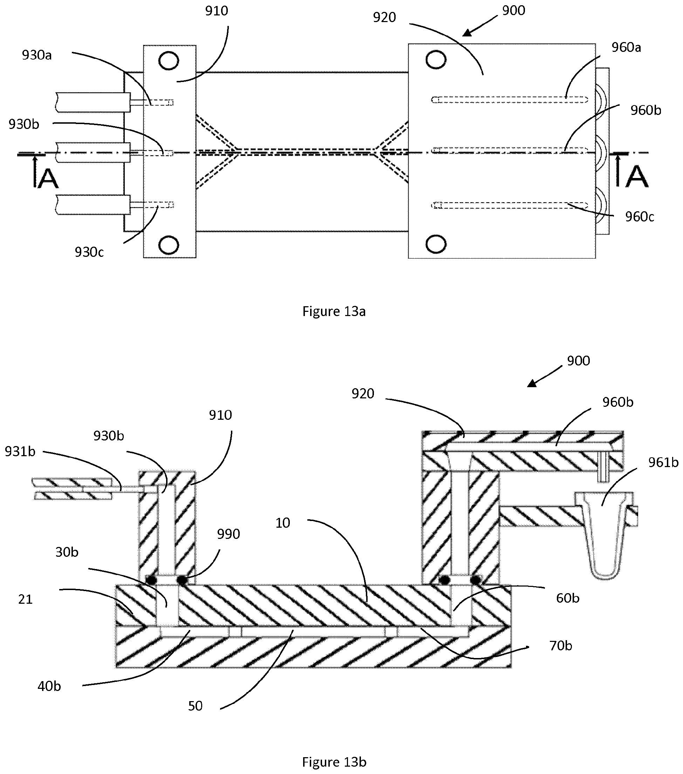

- la figure 13a représente un dispositif de connexion selon l'invention en vue de dessus

- la figure 13b représente le dispositif de connexion selon la coupe A-A de la figure 13a

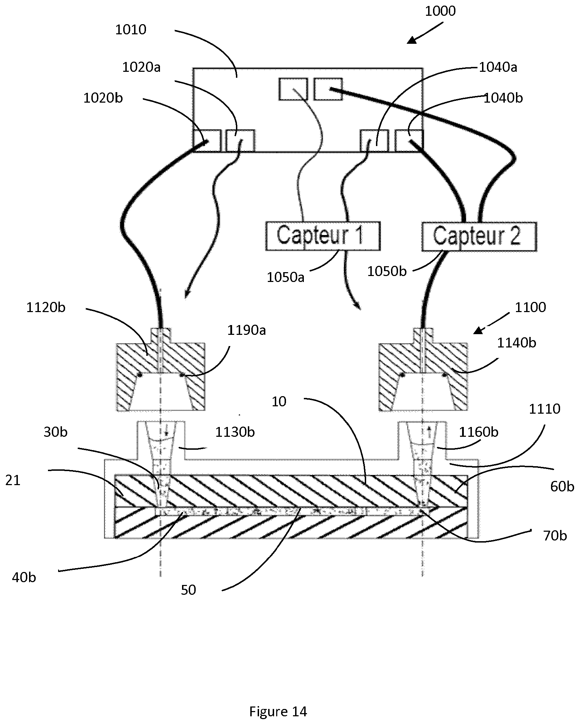

- la figure 14 représente une vue schématique d'un système de régulation d'air permettant la mise en œuvre d'une méthode non couverte par la présente invention,

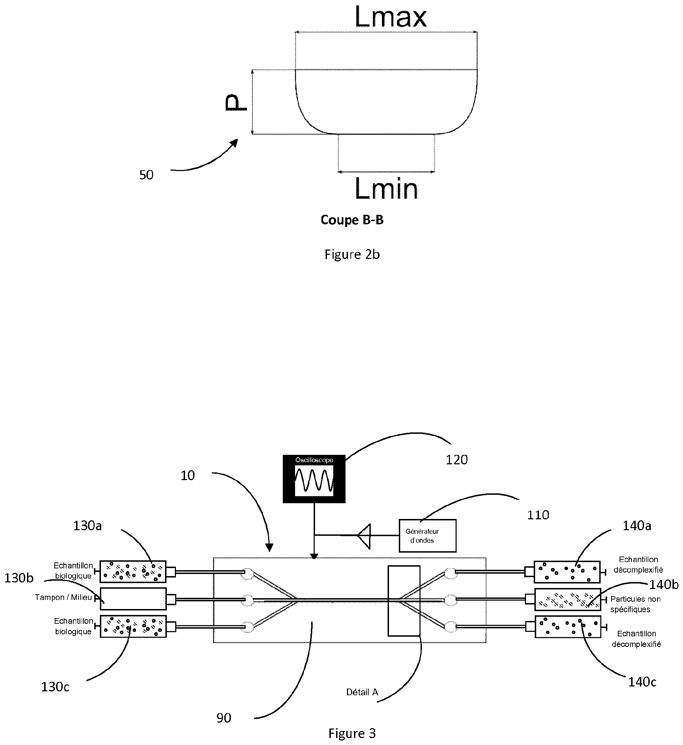

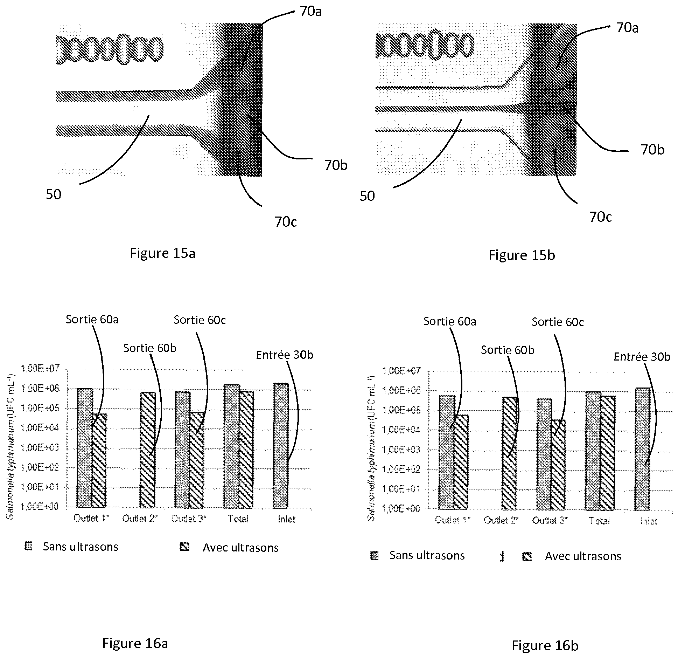

- les figure 15a et 15b est une photographie un vue de dessus d'une partie de dispositif microfluidique du système selon le détail A de la figure 3, non couvert par la présente invetion,

- les figures 16a et 16b illustrent les résultats d'opérations de dénombrement de Salmonelles suite à la méthode de traitement selon l'invention

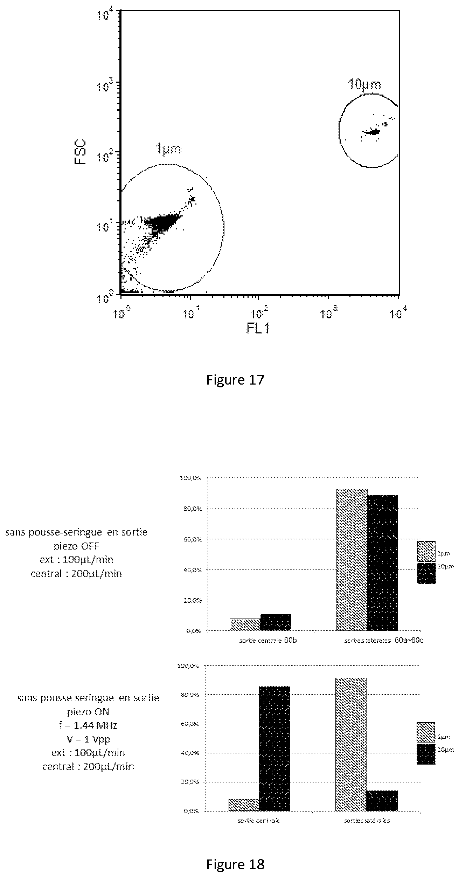

- la figure 17 illustre la capacité d'un cytomètre à distinguer des billes de 1$\mu$m et de 10$\mu$m

- la figure 18 illustre le taux de récupération de billes fluorescentes de 1$\mu$m et de 10$\mu$m suite à la méthode de traitement selon l'invention

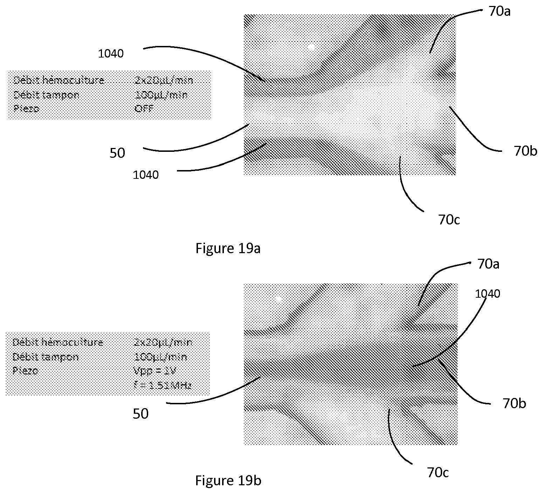

- les figures 19a et 19b illustrent l'effet des ondes acoustiques sur la focalisation des globules rouges d'une hémoculture pendant la méthode de traitement selon l'invention

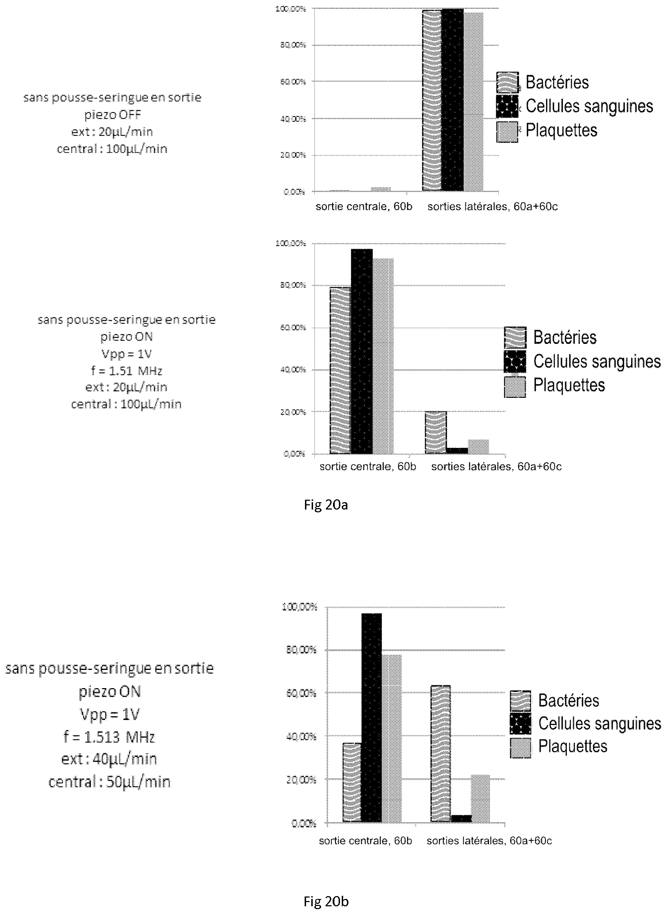

- la figure 20a illustre le taux de réjection des cellules sanguines, l'échantillon d'hémoculture étant introduit sans dilution préalable à un débit de 20$\mu$L/min.

- la figure 20b illustre le taux de réjection des cellules sanguines, l'échantillon d'hémoculture étant introduit sans dilution préalable à un débit de 40$\mu$L/min

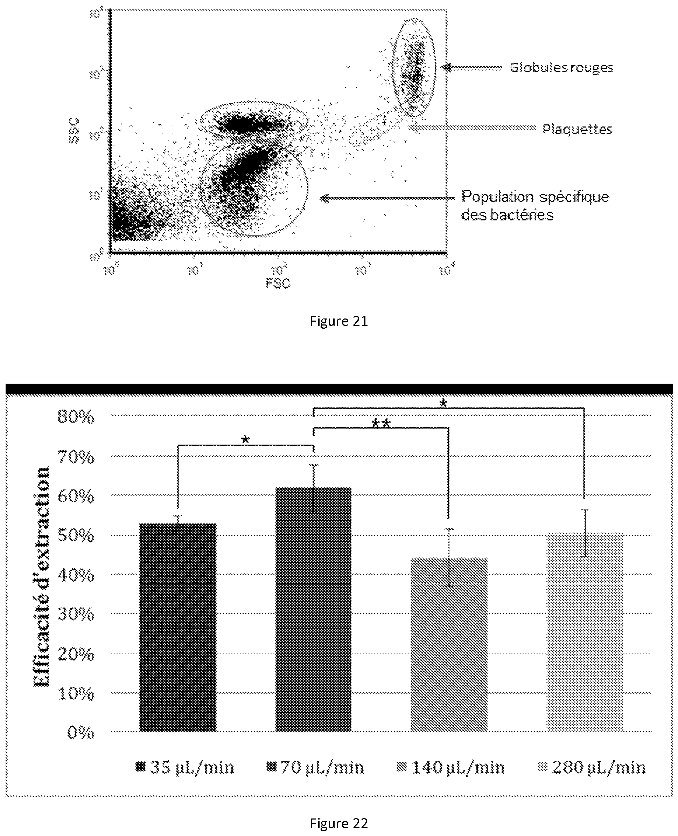

- la figure 21 illustre le résultat de l'analyse en cytométrie de flux (scattergramme) d'un échantillon décomplexifié par la méthode de traitement selon l'invention

- la figure 22 est un diagramme illustrant l'impact du débit de tampon extracteur sur l'efficacité d'extraction.

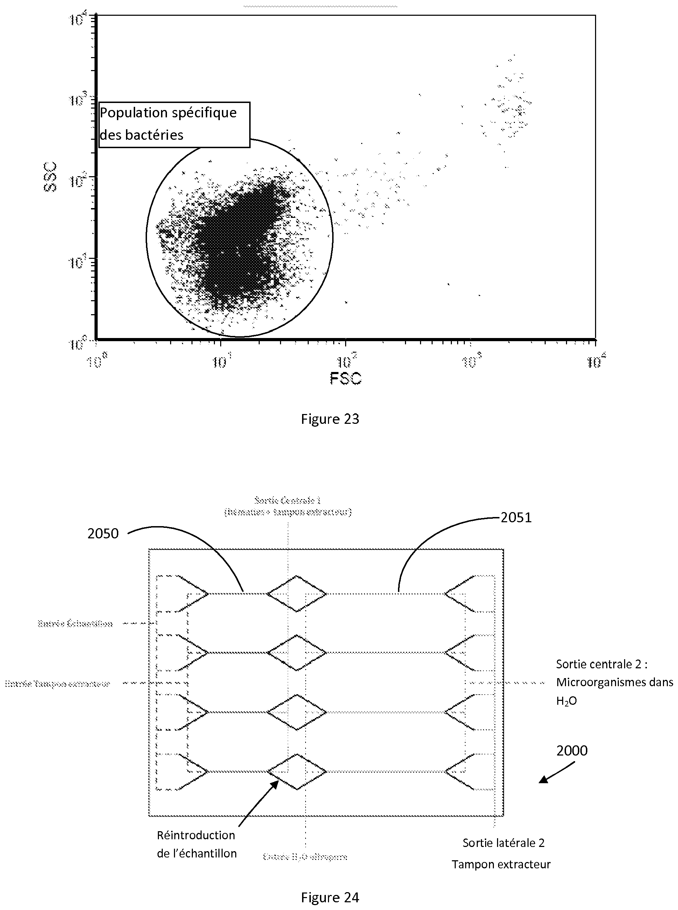

- la figure 23 illustre le résultat de l'analyse en cytométrie de flux (scattergramme) d'un échantillon décomplexifié par la méthode de traitement selon l'invention

- la figure 24 illustre un dispositif susceptible de mettre en œuvre le procédé selon l'invention, comprenant quatre paires de deux canaux de

séparation successifs et permettant le changement du tampon utilisé entre les deux étapes de séparation

La description détaillée ci-après a pour but d'exposer l'invention de manière suffisamment claire et complète, notamment en référence aux figures susvisées, mais ne doit en aucun cas être regardée comme limitant l'étendue de la protection aux modes de réalisation particuliers objets desdites figures.

Des procédés de séparation de particules, cellules ou molécules en flux continu utilisant des dispositifs microfluidiques peuvent permettre de traiter des grands volumes d'échantillon et ce en introduisant continuellement l'échantillon dans le dispositif microfluidique. Un autre intérêt de ces techniques et leur potentiel d'intégration en amont ou en aval d'une étape de capture ou d'analyse de l'échantillon, assurant ainsi un rôle d'aiguillage et/ou de filtre dans une chaine de traitement et d'analyse d'un échantillon biologique ou chimique complexe.

Un certain nombre de forces ont été utilisées avec succès dans des dispositifs microfluidiques, y compris les forces d'inertie, électriques, magnétiques ainsi que les forces mécaniques de contact. Parmi ces diverses forces appliquées pour la séparation de particules, cellules ou molécules dans des dispositifs microfluidiques, les forces acoustiques générées à partir d'ondes ultrasonores ont également été largement utilisées pour la séparation de particules de taille micrométrique en suspension, pour les séparer de leur milieu et/ou d'autres particules. Cette technique, dite d'acoustophorèse, permet la séparation non destructive et sans marqueur, uniquement sur la base de la taille, la densité et la compressibilité de particules, cellules ou molécules.

L'acoustophorèse consiste en l'application d'une onde acoustique stationnaire sur un ou plusieurs canaux d'un dispositif microfluidique, présentant ainsi un profil de pression immobile et disposé transversalement vis à vis du canal visé. Le profil de pression des ondes acoustiques stationnaires appliquées varie ainsi entre des zones de haute pression, appelées des nœuds (« nodes » en langue anglaise), et des zones de basse pression, appelées anti nœuds (« anti-nodes » en langue anglaise).

De façon classique, plusieurs fluides de densités différentes sont introduits par des canaux d'acheminement dans un dispositif microfluidique de façon à s'écouler de façon laminaire (donc sans mélange) dans le canal de séparation, en regard d'un transducteur ultrasonore piézoélectrique. Quand le transducteur n'est pas excité par un signal de commande, les fluides introduits s'échappent du canal de séparation sans observer de mélange ni de migration d'un fluide vers l'autre. En appliquant un signal de commande au transducteur, les particules présentes dans les différents fluides vont subir la force acoustique ainsi générée et se déplacer vers les nœuds de pression ou les anti-nœuds en fonction de leur taille, leur densité et leur compressibilité. La force acoustique s'appelle également pression de radiation acoustique. La densité des fluides introduits ainsi que leur compressibilité influencent également. Cette migration des particules dans le canal de séparation permet donc de favoriser leur concentration dans certain canaux d'acheminement et vers les orifices de sorties du dispositif microfluidique, en aval de canal de séparation.

L'amplitude de la radiation acoustique générée par le transducteur est proportionnelle à celle du signal de commande appliqué, cependant, les effets maximums de la force acoustique sont obtenus à partir d'un même signal de commande quand la fréquence et l'amplitude de celui-ci font entrer le dispositif microfluidique en résonance. Pour une configuration à un seul nœud de pression, cette fréquence de résonance est dépendante de la largeur du canal de séparation ainsi que du matériau dans lequel est fabriqué le dispositif. Des matériaux classiques pouvant être employés sont le verre ou le silicium, ceux-ci présentant des surfaces idéales de réflexion des ondes acoustique.

La pression de radiation dû à l'onde acoustique influence majoritairement les particules de taille supérieures à $2\mu$m. Cette pression étant directement proportionnelle au volume des particules, un changement mineur du rayon de la particule diminue ou augmente rapidement son impact sur celle-ci.

Une autre force est également créée par une onde ultrasonore stationnaire dans un canal contenant une suspension de microparticules. Cette force est dûe à la diffusion et la réflexion de l'onde acoustique dans le fluide et sur les particules. La force de diffusion acoustique est relativement faible et affecte surtout les particules inférieures à $2\mu$m.

Un dispositif de séparation par acoustophorèse peut donc être créé par l'utilisation d'un transducteur acoustique ultrasonore en regard d'un surface de réflexion ou d'un second transducteur de manière à établir une onde stationnaire résonante dans le canal de séparation.

Un dispositif microfluidique comprend ainsi au moins deux orifices d'entrée, un canal de séparation et au moins deux orifices de sortie. Les orifices d'entrée débouchent vers le canal de séparation, tandis que le canal de séparation débouche vers les orifices de sortie. Le dispositif est agencé pour qu'un transducteur à ultrasons puisse être intégré dans ou accolé à une paroi dudit canal de séparation. Le transducteur à ultrasons ainsi intégré ou accolé est apte à transmettre des oscillations mécaniques en ondes acoustiques multiples agissant sur le contenu du canal de séparation.

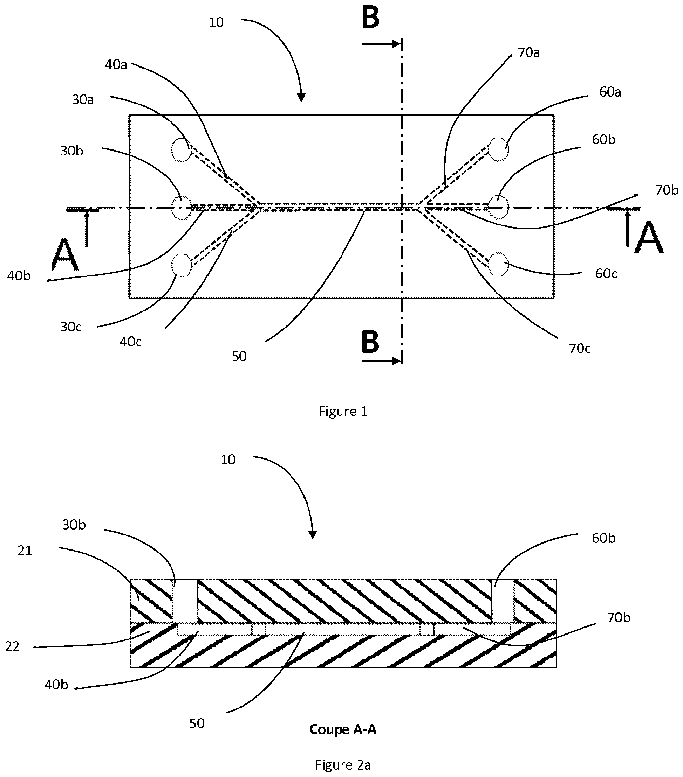

Comme représenté sur les figure 1 et figure 2a, un dispositif microfluidique 10 de l'art antérieur comprend deux parties fluidiques 21 et 22, la partie 21 étant également appelée plaque de recouvrement et la partie 22 étant également appelé séparateur. Le séparateur et la plaque de recouvrement sont sensiblement plans, et assemblés entre eux de manière à réaliser des canaux fluidiques. Le séparateur est microthermiquement lié à la plaque de recouvrement pour réaliser un dispositif 10 apte à être observé par un dispositif d'analyse optique tel qu'un microscope.

Le dispositif se compose de trois orifices d'entrées 30a, 30b, 30c et de trois orifices de sorties 60a, 60b, 60c reliés par un canal de séparation 50 rectiligne, d'une longueur de 35 mm, permettant, lors du fonctionnement du système, aux cellules, particules ou molécules de devenir acoustiquement concentrées dans le canal d'acheminement 70b, dont l'axe est identique et dans le prolongement de l'axe du canal de séparation 50. Les cellules, particules ou molécules sont acoustiquement concentrées dans le canal central en fonction de leur densité, leur taille et leur compressibilité.

Les trois orifices d'entrée 30a, 30b, 30c, communiquent avec le canal de séparation 50 par le biais de canaux d'acheminement respectivement 40a, 40b, 40c. Le canal de séparation 50 communique également avec les trois orifices de sortie 60a, 60b, 60c, par le biais de canaux d'acheminement, respectivement 70a, 70b, 70c.

Le séparateur 22 comprend une plaquette de verre de 1mm d'épaisseur revêtue d'une couche de chrome et d'une couche de résine photosensible. Après développement de la couche photosensible, le verre est gravé par de l'acide fluorhydrique (HF) produisant des canaux d'acheminement et de séparation d'une profondeur de 125 $\mu$m, d'une largeur inférieure, au fond des canaux, de 375 $\mu$m, et d'une largeur supérieure de 625 $\mu$m dans le plan du séparateur en contact avec la plaque de recouvrement. La plaque de recouvrement 21 est percée pour réaliser les orifices d'entrée et de sorties puis collée thermiquement avec le séparateur afin de réaliser un assemblage étanche.

Les canaux d'acheminement des orifices d'entrée 40a, 40b, 40c présentent un angle de respectivement de 45°, 0° et moins 45° par rapport au canal de séparation dans le plan du séparateur, de manière à ralentir le débit des fluides provenant des canaux d'acheminement présentant un angle de 45 ° ou -45° ,40a , 40c, par rapport au canal de séparation et de favoriser ainsi l'apparition d'un flux laminaire dans le canal de séparation.

Le profil type d'un canal d'acheminement ou de séparation est représenté sur la figure 2b. Les canaux présentent une forme de D dans le plan perpendiculaire à l'axe du canal considéré, la largeur la plus importante étant située dans le plan en contact avec la plaque de recouvrement quand le dispositif est assemblé. Ils présentent ainsi une largeur inférieure « Lmin », au fond des canaux, une largeur supérieure « Lmax » dans le plan du séparateur en contact avec la plaque de recouvrement quand le dispositif est assemblé ainsi qu'une profondeur « P ».

Ce dispositif de l'art antérieur est susceptible de mettre en œuvre des procédés de traitement d'échantillons biologiques par acoustophorèse. Pour cela, tout ou partie de l'échantillon biologique à traiter, est introduit dans les orifices d'entrée 30a et 30c du dispositif 10. Une solution tampon est introduite dans l'orifice d'entrée 30b, le tampon et l'échantillon étant introduits à des débits respectifs aptes à générer un flux laminaire dans le canal de séparation. Un transducteur ultrasonore, tel qu'un transducteur piézoélectrique, accolé au canal de séparation est alors activé par un signal de commande, de manière à réaliser une étape de séparation dudit échantillon biologique par acoustophorèse. Cette séparation permet de favoriser la concentration des particules non-spécifiques dans l'échantillon, dans l'orifice de sortie 60b dudit dispositif d'acoustophorèse. L'échantillon décomplexifié étant obtenu dans les orifices de sortie 60a et 60c. L'échantillon concentré étant obtenu dans l'orifice de sortie 60b.

Ci-après sont décrit différents dispositifs microfluidiques permettant le traitement d'échantillons biologiques par acoustophorèse. Ces différents dispositifs sont également susceptibles de mettre en œuvre le procédé selon l'invention de façon avantageuse. Ces dispositif sont particulièrement intéressants à mettre en œuvre avec le procédé selon l'invention car ils permettent d'atteindre des débits de préparation d'échantillon plus élevés que des dispositifs de l'art antérieur tout en assurant un taux de décomplexification égal ou supérieur à ceux observé dans l'art antérieur pour des échantillons biologique tel que des échantillons sanguins primaires ou les fluides corporels stériles incubés en milieu de culture.

Les orifices d'entrée et la forme des canaux d'acheminements communiquant avec les orifices d'entrée peuvent avantageusement être optimisés afin d'accepter une sédimentation des particules non spécifiques, agrégats (de lipides ou d'érythrocytes) ou débris aux entrées du dispositif microfluidique sans risque de bouchage des canaux du dispositif. A ce titre, la partie de chaque canal d'acheminement en regard de chaque orifice d'entrée susceptible de recevoir l'échantillon biologique peut présenter une cavité d'une profondeur plus importante que la profondeur générale du canal d'acheminement. Avantageusement, la cavité en regard de l'orifice d'entrée est deux à trois fois plus profonde que le canal d'acheminement connecté audit orifice. Cette cavité permet de créer une zone de vitesse ralentie ou nulle de l'échantillon introduit, favorisant la sédimentation des particules non-spécifiques. Le dispositif ainsi formé présente une meilleure tolérance aux bouchages liés à l'introduction de particules non-spécifiques de taille et de densité importantes.

Pour chacun de ces modes de réalisation, le séparateur peut être réalisé en silicium, ceramique ou en verre, les canaux étant obtenus par gravure chimique ou physique (e.g. sablage). Le séparateur peut également être réalisé en matériau polymère, souple et fin tel que du polydiméthylsiloxane (PDMS), du Polypropylene ou un bicomposé Polymère / Silicone (par exemple un Polystyrène avec un film souple de silicone ou de PDMS afin d'assurer un couplage sans air des transducteurs piézoélectriques sur la paroi du dispositif), les canaux étant alors obtenu par moulage. L'avantage de l'utilisation d'un matériau polymère, souple et fin est de pouvoir réaliser un dispositif à très bas coût, permettant son utilisation comme consommable en routine dans des procédé de préparation d'échantillons biologiques par acoustophorèse tel que le procédé selon l'invention. Un autre avantage étant de ne pas nécessiter de gel de couplage ou de colle entre le/les transducteurs piézoélectrique(s) et les parois du canal de séparation.

Pour chacun de ces modes de réalisation, chaque orifice, d'entrée ou de sortie, peut former un réservoir de manière à pouvoir réaliser des opérations de stockage d'un échantillon ainsi que des opérations de dépose et de pipetage. En accolant un moyen de chauffage à un orifice, une étape d'incubation de l'échantillon peut également être réalisée. De façon avantageuse, un orifice peut être réalisé dans un matériau transparent ou translucide, de manière à pouvoir réaliser directement une lecture de la densité optique de l'échantillon qu'il contient.

Pour chacun de ces modes de réalisation, chaque dispositif peut être utilisé seul ou en parallèle de manière à pouvoir traiter un volume d'échantillon biologique à un débit plus important. En effet, si on augmente le débit d'introduction des fluides dans un même canal de séparation, le taux de décomplexification de l'échantillon peut décroitre rapidement, les particules n'ayant pas le temps d'être séparées par l'onde acoustique dû à un temps de résidence trop court dans le dispositif. D'autre part, si la taille des canaux, notamment du canal de séparation est augmentée, dans l'espoir de pouvoir également augmenter le débit de traitement, l'amplitude d'excitation (du signal de commande) du/des transducteur(s) piézoélectrique(s) nécessaire à une bonne séparation des particules devra être bien plus importante puisque la fréquence de résonance sera réduite et par conséquence la pression de radiation sur les particules à focaliser au centre du canal de séparation. Cependant, en excitant le transducteur à ultrasons à une plus forte amplitude, celui-ci peut provoquer un échauffement local dans le canal de séparation. Cet échauffement n'est pas souhaitable car celui-ci peut engendrer une dégradation du dispositif ainsi que de la viabilité des particules, cellules, ou molécules d'intérêt présentes dans le canal de séparation. D'autre part, cet échauffement peut engendrer un changement de densité du tampon utilisé ce qui peut modifier la propagation des ondes acoustiques dans le canal et perturber l'étape de séparation. De ce fait les dispositifs utilisés seul ou en parallèle, permettent d'obtenir des débits de séparation / décomplexification égaux ou supérieurs à ceux de l'art antérieur tout en garantissant la viabilité des particules, cellules, ou molécules d'intérêt traitées.

Ainsi les canaux de séparation des différents dispositifs présentent une longueur pouvant être comprise entre 35mm et 80mm. Les largeurs inférieures du canal de séparation sont par exemple comprises entre $300\mu m$ et $375\mu m$, les largeurs supérieures sont par exemple comprises entre et $550\mu m$ et $625\mu m$. La profondeur du canal de séparation peut être comprise entre $100\mu m$ et $150\mu m$, préférentiellement de $125\mu m$. Les différents dispositifs sont adaptés pour être utilisés avec des signaux de commandes de(s) transducteur(s) ultrasonore(s) accolé(s) présentant une fréquence comprise entre 300kHz et 10MHz, préférentiellement de 1,3MHz alternativement de 1,44MHz pour la création d'un seul nœud de pression au centre du canal. Ces différentes valeurs de fréquence permettent d'obtenir une focalisation centrale (un seul nœud de pression dans le canal de séparation) présentant des particules non-spécifiques focalisées dans l'orifice de sortie dans le prolongement de l'axe du canal de séparation ou de privilégier la concentration de ces particules non-spécifiques dans un des orifices de sortie. Différentes fréquences de résonance peuvent être observées pour un même dispositif du fait de l'apparition de plusieurs nœuds de pression dans la largeur du canal de séparation en multiple du quart de la longueur d'onde ($n\lambda/4$). Des variations légères d'environ 30kHz autour de la fréquence de résonance peuvent également permettre d'obtenir une meilleure résonance du dispositif, cette fréquence étant dépendante de la qualité de réalisation des canaux du dispositif.

L'amplitude du signal de commande est comprise entre 0,1V et 100V, préférentiellement de 38V. Pour des amplitudes du signal de commande supérieures à 38V, il peut être souhaitable d'utiliser un dispositif de refroidissement accolé au(x) transducteur(s) à ultrasons pour empêcher une dégradation du dispositif et/ou une atteinte à la viabilité des particules, cellules, ou molécules présentent dans le canal de séparation. Des blocs Peltier ou des ventilateurs peuvent constituer de tels dispositifs de refroidissement. Avantageusement, le dispositif de refroidissement est asservi en température afin de réguler la température à proximité du transducteur ultrasonore.

Pour chacun de ces modes de réalisation, les canaux d'acheminement des orifices d'entrée utilisés pour l'introduction de l'échantillon biologique présentent un angle compris entre 30 et 60°, préférentiellement de 45° par rapport au canal de séparation dans le plan du séparateur. Cet angle peut aisément être adapté par l'homme du métier de manière à ralentir plus ou moins le débit d'introduction du fluide dans le canal de séparation, et assurer ainsi l'apparition d'un écoulement laminaire des fluides dans ce canal. Ces canaux d'acheminement sont appelés canaux latéraux car ils permettent d'introduire le fluide en direction des parois du canal de séparation.

Pour chacun de ces modes de réalisation, les canaux d'acheminement vers les orifices de sortie utilisé pour récupérer ou réintroduire l'échantillon décomplexifié présentent un angle compris entre 30 et 60°, préférentiellement de 45° par rapport au canal de séparation dans le plan du séparateur. Cet angle peut aisément être adapté par l'homme du métier de manière à ralentir plus ou moins le débit d'aspiration du fluide hors du canal de séparation, et assurer ainsi l'apparition d'un écoulement laminaire des fluides dans ce canal. Ces canaux d'acheminement sont également appelés canaux latéraux car ils permettent d'aspirer le fluide circulant le long des parois du canal de séparation.

Les canaux d'acheminements dont l'(les) axes est (sont) identique(s) à l'axe du canal de séparation sont appelés canaux centraux. Ces canaux transportent l'échantillon concentré.

D'autre part les sections des canaux d'acheminement des orifices d'entrée et de sortie peuvent être adaptées.

De façon particulière, les sections des canaux d'acheminement des orifices de sortie latérales (dans lesquels on retrouve l'échantillon décomplexifié) par rapport à la section du canal d'acheminement de l'orifice de sortie central (dans lequel on retrouve un maximum de particules non-spécifiques) peuvent être adaptées afin de garantir une meilleure focalisation au niveau de l'embranchement de sortie entre ces canaux. Dans cette zone, à la jonction du canal de séparation et des canaux d'acheminement vers les orifices de sortie, la résonance est peu effective (car il n'y a plus de parois latérales à l'embranchement) durant un temps transitoire court. Ceci conduit à une perte partielle de focalisation des particules non-spécifiques. Dans le cas du dispositif illustré sur la figure 1, l'ajustement des rapports des sections entre les canaux 70a ou 70c et 70b peut ainsi permettre de minimiser les pertes de microorganismes ou d'améliorer le taux de concentration des particules non-spécifiques ou débris dans le canal d'acheminement central 70b.

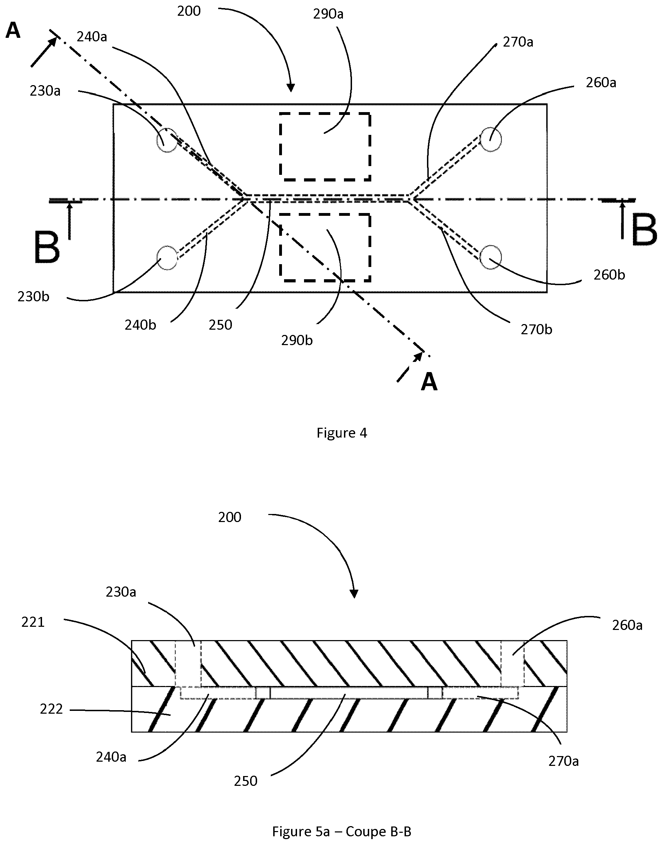

Un premier mode de réalisation d'un dispositif est représenté sur les figures 4 et 5a. Ce dispositif 200 comprend deux orifices d'entrée 230a, 230b, un canal de séparation 250 et au moins deux orifices de sorties 260a, 260b. Le dispositif 200 comprend deux parties fluidiques, un séparateur 222 et une plaque de recouvrement 221, sensiblement planes. Ces deux parties sont assemblées de façon hermétique. Les orifices d'entrée débouchent vers le canal de séparation, tandis que le canal de séparation débouche vers les orifices de sortie par le biais de canaux d'acheminement. Ainsi, le canal de séparation 250 débouche vers l'orifice 230a via le canal d'acheminement 240a, vers l'orifice 230b via le canal d'acheminement 240b, vers l'orifice 260a via le canal d'acheminement 270a, vers l'orifice 260b via le canal d'acheminement 270b. Dans une première alternative de réalisation de ce premier mode de réalisation, le dispositif est agencé pour qu'au moins un transducteur à ultrasons puisse être intégré dans ou accolé à une paroi dudit canal de séparation. Le transducteur à ultrasons ainsi intégré ou accolé est apte à transmettre des oscillations mécaniques en ondes acoustiques multiples pouvant agir sur le contenu du canal de séparation.

Dans une seconde alternative de réalisation de ce premier mode de réalisation, le dispositif comprend deux évidements 290a, 290b, réalisés le long du canal de séparation 250, dans le séparateur 222, et susceptibles de recevoir chacun un transducteur à ultrasons. Ce mode de réalisation permet l'obtention d'une onde stationnaire dans un séparateur réalisé dans un matériau peu réfléchissant aux ondes acoustiques, le matériau étant souple et fin tel que des matériaux plastiques, polymères, silicones. Un silicone préférentiellement utilisé est le Polydiméthylsiloxane (PDMS). Préférentiellement, chaque transducteur à ultrasons comprend une lame ¼ onde adaptatrice dont l'impédance acoustique est calculée pour minimiser les pertes d'énergie, et donc l'échauffement, entre le transducteur à ultrasons et les parois du dispositif. Cette lame de résine recouvrant le/les transducteur(s) piézoélectrique(s) (réalisée par coulage et polymérisation), est définie pour avoir une impédance acoustique intermédiaire entre l'impédance acoustique du matériau utilisé pour la réalisation du dispositif, et l'impédance acoustique du(es) transducteur(s) piézoélectrique(s).

Préférentiellement la plaque de recouvrement 221 est réalisée en Polydiméthylsiloxane (PDMS) ou en matériau plastique moulé tel que du Polycarbonate (PC), poly(méthacrylate de méthyle) (PMMA), polypropylène (PP), PolyStyrene (PS), Acrylonitrile butadiène styrène (ABS), Copolymère d'Oléfine Cyclique (COC), Polymère d'Oléfine Cyclique (COP), Polyoxyméthylène (POM).

Ce dispositif 200 est susceptible de mettre en œuvre des procédés de préparation d'échantillons biologiques par acoustophorèse .

Pour cela, tout ou partie de l'échantillon biologique à traiter, est introduit dans l'orifice d'entrée 230a du dispositif 200. Une solution tampon est introduite dans l'orifice d'entrée 230b, le tampon et l'échantillon étant introduits à des débits respectifs aptes à générer un flux laminaire dans le canal de séparation. Un transducteur ultrasonore, tel qu'un transducteur piézoélectrique, accolé au canal de séparation est alors activé par un signal de commande, de manière à réaliser une étape de séparation dudit échantillon biologique par acoustophorèse. Cette séparation permet de favoriser la concentration des particules non-spécifiques, tel que des éléments figurés du sang, présents dans l'échantillon, dans l' orifice de sortie 260b dudit dispositif d'acoustophorèse. L'échantillon décomplexifié étant obtenu dans l'orifice de sortie 260a. Les particules non spécifiques sont ainsi transférées dans la solution tampon introduite dans l'entrée 230b. L'échantillon concentré étant ainsi obtenu dans l'orifice de sortie 260b.

Une alternative de réalisation de l'orifice d'entrée 230a est présentée sur la figure 5b. L'orifice d'entrée 230a présente ici une forme tronconique mais peut également être cylindrique en fonction du moyen d'introduction choisi. La forme du canal d'acheminement 240a, en regard de l'orifice 230a est modifiée de manière à comprendre une cavité 241a de profondeur plus importante que la profondeur générale du canal d'acheminement 240a. Cette cavité présente ainsi une profondeur P2 avec P2 comprise entre deux et trois fois la profondeur P du canal d'acheminement. Cette cavité permet de créer une zone de vitesse ralentie ou nulle (Z) de l'échantillon introduit, favorisant la sédimentation des particules non-spécifiques contenues dans l'échantillon. Le dispositif ainsi formé présente une meilleure tolérance aux bouchages liés à l'introduction de particules non-spécifiques de taille et de densité importantes.

Comme représenté sur la figure un second mode de réalisation d'un dispositif 300 comprenant deux parties fluidiques, un séparateur 322 et une plaque de recouvrement (non représentée), ces deux parties étant sensiblement planes. Ces deux parties sont assemblées de façon hermétique. Des canaux sont réalisés dans le séparateur 322. Le dispositif 300 comprend deux orifices d'entrée 330a, 330b, en communication fluidique avec au moins un canal de séparation 350 par le biais de canaux d'acheminement respectivement 340a, 340c pour l'orifice 330a et 340b pour l'orifice 330b. Le canal de séparation 350 est également en communication fluidique avec deux orifices de sortie 360a, 360b, par le biais de canaux d'acheminement, respectivement 370a et 370c pour l'orifice 360a et 370b pour l'orifice 360b. Cette configuration permet de simplifier la connexion fluidique car elle présente uniquement deux orifices d'entrée et deux orifices de sortie. De même, la collecte de l'échantillon décomplexifié peut être réalisée dans un orifice de sortie ou un tube de collection unique. D'autre part, les canaux d'acheminement 370a et 370c vers l'orifice de sortie 360a étant directement connectés par gravure des canaux sur le dispositif, l'équilibrage des pertes de charge au niveau de l'embranchement des canaux d'acheminement vers les orifices de sortie est significativement améliorée. Une différence minime de perte de charge (liée à une différence de connexion des tubes de sortie sur le dispositif) pouvant entraîner une déviation de la focalisation et ainsi une dégradation importante des performances de décomplexification.

Le dispositif 300 est agencé pour qu'un transducteur à ultrasons, non représenté, puisse être intégré dans ou accolé à une paroi dudit canal de séparation 350. Le transducteur à ultrasons ainsi intégré ou accolé est apte à transmettre des oscillations mécaniques en ondes acoustiques multiples pouvant agir sur le contenu du canal de séparation et y générer une onde acoustique stationnaire. Un générateur de courant alternatif associé à un amplificateur de signal, non représentés, peut être connecté électriquement au transducteur afin de générer un signal de commande du transducteur dont la fréquence, la forme d'onde et l'amplitude sont connues.

Dans un mode de réalisation particulier du dispositif 300, le séparateur 322 est réalisé dans une plaquette de verre de 1mm d'épaisseur revêtue d'une couche de chrome et d'une couche de résine photosensible. Après développement de la couche photosensible, le verre est gravé par de l'acide fluorhydrique (HF). Les canaux d'acheminement 340a et 340c, 370a et 370c présentent une profondeur P de 125 $\mu$m, une largeur inférieure Lmin, au fond des canaux, de 300 $\mu$m, et d'une largeur supérieure Lmax de 550 $\mu$m dans le plan du séparateur en contact avec la plaque de recouvrement. Les canaux d'acheminement 340b et 370b ainsi que le canal de séparation 350 présentent une profondeur P de 125 $\mu$m, une largeur inférieure Lmin, au fond des canaux, de 375 $\mu$m, et une largeur supérieure Lmax de 625$\mu$m dans le plan du séparateur en contact avec la plaque de recouvrement. La plaque de recouvrement est percée pour réaliser les orifices d'entrée et de sortie puis collée thermiquement avec le séparateur afin de réaliser un assemblage étanche. Les canaux d'acheminement des orifices d'entrée 340a, 340b, présentent un angle de respectivement de 45°, 0° et moins 45° par rapport au canal de séparation 350 dans le plan du séparateur, de manière à ralentir le débit des fluides provenant des canaux d'acheminement présentant un angle de 45 ° ou -45° ,340a, 340b, par rapport au canal de séparation. Le canal de séparation 350 rectiligne, présente une longueur de 80 mm, permettant, aux cellules, particules ou molécules de devenir acoustiquement concentrés dans le canal d'acheminement 370b lors du fonctionnement du système. L'axe du canal d'acheminement 370b est identique à l'axe du canal de séparation. Les cellules, particules ou molécules sont acoustiquement concentrées dans le canal central 370b, en fonction de leur densité, leur taille et leur compressibilité.

Alternativement, le séparateur 322 du dispositif 300 comprend deux évidements (non représentés) réalisés le long du canal de séparation 350, et susceptibles de recevoir chacun un transducteur à ultrasons (non représentés). Ce mode de réalisation permet l'obtention d'une onde stationnaire dans le séparateur 322 si celui-ci réalisé dans un matériau peu réfléchissant aux ondes acoustiques, le matériau étant souple et fin tel que des matériaux plastiques, polymères, silicones. Un silicone préférentiellement utilisé est le Polydiméthylsiloxane (PDMS). Préférentiellement, chaque transducteur à ultrasons comprend une lame ¼ onde adaptatrice dont l'impédance acoustique est calculée pour minimiser les pertes d'énergie, et donc l'échauffement, entre le transducteur à ultrasons et les parois du dispositif.

Préférentiellement la plaque de recouvrement (non représentée) est réalisée en PDMS ou en matériau plastique moulé tel que du Polycarbonate (PC), poly(méthacrylate de méthyle) (PMMA), polypropylène (PP), PolyStyrene (PS), Acrylonitrile butadiène styrène (ABS), Copolymère d'Oléfine Cyclique (COC), Polymère d'Oléfine Cyclique (COP), Polyoxyméthylène (POM).

Ce dispositif 300 est susceptible de mettre en œuvre des procédés de préparation d'échantillons biologiques par acoustophorèse. Pour cela, tout ou partie de l'échantillon biologique à traiter, est introduit dans l'orifice d'entrée 330a du dispositif 300. Une solution tampon est introduite dans l'orifice d'entrée 330b, le tampon et l'échantillon étant introduits à des débits respectifs aptes à générer un flux laminaire dans le canal de séparation. Un transducteur ultrasonore, tel qu'un transducteur piézoélectrique, accolé au canal de séparation est alors activé par un signal de commande, de manière à réaliser une étape de séparation dudit échantillon biologique par acoustophorèse. Cette séparation permet de favoriser la concentration des particules non-spécifiques, tel que des éléments figurés du sang, présents dans l'échantillon, dans l'orifice de sortie 360b dudit dispositif d'acoustophorèse. L'échantillon décomplexifié étant obtenu dans l'orifice de sortie 360a. L'échantillon concentré étant obtenu dans l'orifice de sortie 360b.

Comme représenté sur la figure 6b, un dispositif 400 comprenant deux parties fluidiques, un séparateur 422 et une plaque de recouvrement (non représentée), ces deux parties étant sensiblement planes. Ces deux parties sont assemblées de façon hermétique. Des canaux fluidiques sont réalisés dans le séparateur 422. Le dispositif 400 comprend deux orifices d'entrée 430a, 430b, en communication fluidique avec au moins un canal de séparation 450 par le biais de canaux d'acheminement respectivement 440a, 440c, pour l'orifice 430a et 440b pour l'orifice 430b. Le canal de séparation 450 est également en communication fluidique avec trois orifices de sortie 460a, 460b, 460c, par le biais de canaux d'acheminement, respectivement 470a et 470c pour l'orifice 460a , 470b et 470d pour l'orifice 460c ; 470b, 470f et 470e pour l'orifice de sortie 460b. Le dispositif 400 est agencé pour qu'un transducteur à ultrasons, non représenté, puisse être intégré dans ou accolé à une paroi dudit canal de séparation 450. Le transducteur à ultrasons ainsi intégré ou accolé est apte à transmettre des oscillations mécaniques en ondes acoustiques multiples pouvant agir sur le contenu du canal de séparation et y générer une onde acoustique stationnaire. Un générateur de fonction associé à un amplificateur de signal, non représentés, peut être connecté électriquement au transducteur afin de générer un signal de commande du transducteur dont la fréquence, la forme d'onde et l'amplitude sont connues. Le canal d'acheminement 470b, disposé dans le prolongement du canal de séparation 450, permet la réalisation d'une seconde étape de séparation des particules non-spécifiques pouvant subsister dans l'échantillon biologique.

Dans un mode de réalisation particulier du dispositif 400, Le séparateur 422 est réalisé dans une plaquette de verre de 1mm d'épaisseur revêtue d'une couche de chrome et d'une couche de résine photosensible. Après développement de la couche photosensible, le verre est gravé par de l'acide fluorhydrique (HF). Les canaux d'acheminement 440a, 440c, 470a, 470c, 470e et 470f présentent une profondeur P de 125 $\mu$m, une largeur inférieure Lmin, au fond des canaux, de 300 $\mu$m, et une largeur supérieure Lmax de 550 $\mu$m dans le plan du séparateur en contact avec la plaque de recouvrement. Les canaux d'acheminement 440b, 470b et 470d ainsi que le canal de séparation 450 présentent une profondeur P de 125 $\mu$m, une largeur inférieure Lmin, au fond des canaux, de 375 $\mu$m, et une largeur supérieure Lmax de 625$\mu$m dans le plan du séparateur en contact avec la plaque de recouvrement. La plaque de recouvrement est percée pour réaliser les orifices d'entrée et de sortie puis collée thermiquement avec le séparateur afin de réaliser un assemblage étanche. Les canaux d'acheminement des orifices d'entrée 440a, 440b, 440c, présentent un angle de respectivement de 45°, 0° et moins 45° par rapport au canal de séparation 450 dans le plan du séparateur, de manière à ralentir le débit des fluides provenant des canaux d'acheminement présentant un angle de 45 ° ou - 45° ,440a, 440c, par rapport au canal de séparation. Le canal de séparation 450 rectiligne, présente une longueur de 80 mm, permettant, lors du fonctionnement du système, aux cellules, particules ou molécules de devenir acoustiquement concentrées dans le canal d'acheminement 470b puis 470d, dont les axes sont identiques à l'axe du canal de séparation.

Alternativement, le séparateur 422 du dispositif 400 comprend deux évidements (non représentés) réalisés le long du canal de séparation 450, et susceptibles de recevoir chacun un transducteur à ultrasons (non représenté). Ce mode de réalisation permet l'obtention d'une onde stationnaire dans le séparateur 422 si celui-ci réalisé dans un matériau peu réfléchissant aux ondes acoustiques, le matériau étant souple et fin tel que des matériaux plastiques, polymères, silicones. Un silicone préférentiellement utilisé est le Polydiméthylsiloxane (PDMS). Préférentiellement, chaque transducteur à ultrasons comprend une lame ¼ onde adaptatrice dont l'impédance acoustique est calculée pour minimiser les pertes d'énergie, et donc l'échauffement, entre le transducteur à ultrasons et les parois du dispositif.

Préférentiellement la plaque de recouvrement (non représentée) est réalisée en PDMS ou en matériau plastique moulé tel que du Polycarbonate (PC), poly(méthacrylate de méthyle) (PMMA), polypropylène (PP), PolyStyrene (PS), Acrylonitrile butadiène styrène (ABS), Copolymère d'Oléfine Cyclique (COC), Polymère d'Oléfine Cyclique (COP), Polyoxyméthylène (POM).

Ce dispositif 400 est susceptible de mettre en œuvre des procédés de préparation d'échantillons biologiques par acoustophorèse. Pour cela, tout ou partie de l'échantillon biologique à traiter, est introduit dans l'orifice d'entrée 430a du dispositif 400. Une solution tampon est introduite dans l'orifice d'entrée 430b, le tampon et l'échantillon étant introduits à des débits respectifs aptes à générer un flux laminaire dans le canal de séparation. Un transducteur ultrasonore, tel qu'un transducteur piézoélectrique, accolé au canal de séparation est alors activé par un signal de commande, de manière à réaliser une étape de séparation dudit échantillon biologique par acoustophorèse. Cette séparation permet de favoriser la concentration des particules non-spécifiques, tel que des éléments figurés du sang, présents dans l'échantillon, dans le canal d'acheminement 470b dudit dispositif d'acoustophorèse. L'échantillon décomplexifié est obtenu dans l'orifice de sortie 460a. Une seconde étape de séparation dudit échantillon biologique par acoustophorèse est réalisé sur la partie résultante de la première séparation, l'échantillon concentré, dans le canal 470b. Cette séparation permet de favoriser la concentration des particules non-spécifiques, tel que des éléments figurés du sang, présents dans l'échantillon concentré, dans l'orifice de sortie 460c dudit dispositif d'acoustophorèse. L'échantillon décomplexifié une seconde fois est obtenu dans l'orifice de sortie 460b. La collection et le mélange des échantillons décomplexifiés issus des orifices de sortie 460a et 460b permettent d'augmenter le rendement de collection des microorganismes présents dans l'échantillon biologique. En effet les microorganismes entrainés avec les particules non spécifiques lors de la première séparation dans le canal 470b sont susceptibles d'être séparés par acoustophorèse dans ce canal 470b et de pouvoir ainsi être collectés suite à cette seconde séparation dans l'orifice 460b.

La position relative et la longueur du canal de seconde séparation 470b vis-à-vis du canal de séparation principal 450 est choisie en fonction des applications, des performances et des débits souhaités.

Comme représenté sur la figure 6c, l'invention concerne un dispositif 1300 comprenant deux parties fluidiques, un séparateur 1322 et une plaque de recouvrement (non représentée), ces deux parties étant sensiblement planes. Ces deux parties sont assemblées de façon hermétique. Des canaux sont réalisés dans le séparateur 1322. Le dispositif 1300 comprend deux orifices d'entrée 1330a, 1330b, en communication fluidique avec un premier canal de séparation 1350 par le biais de canaux d'acheminement respectivement 1340a, 1340c pour l'orifice 1330a et 1340b pour l'orifice 1330b. Le canal de séparation 1350 est en communication fluidique avec un orifice de sortie 1360b, par le biais d'un canal d'acheminement 1370b. Le canal de séparation 1350 est également en communication fluidique avec un second canal de séparation 1350' par le biais de deux canaux d'acheminements 1370a et 1370c. Le second canal de séparation 1350' est en communication fluidique avec un orifice de sortie 1360b', par le biais d'un canal d'acheminement 1370b' et en communication fluidique avec un orifice de sortie 1360a, par le biais deux canaux d'acheminements 1370a' et 1370c'.

Le dispositif 1300 comprend également un orifice d'entrée 1330b', en communication fluidique avec le second canal de séparation 1350' via un canal d'acheminement 1340b'.

Le dispositif 1300 est agencé pour qu'un ou plusieurs transducteur(s) à ultrasons, non représenté(s), puisse être intégré dans ou accolé à une paroi desdits canaux de séparation 1350 et 1350'. Le(s) transducteur(s) à ultrasons ainsi intégré(s) ou accolé(s) est(sont) apte(s) à transmettre des oscillations mécaniques en ondes acoustiques multiples pouvant agir sur le contenu des canaux de séparation et y générer une onde acoustique stationnaire. Un générateur de courant alternatif associé à un amplificateur de signal, non représentés, peut être connecté électriquement au(x) transducteur(s) afin de générer un signal de commande dont la fréquence, la forme d'onde et l'amplitude sont connues.

Cette configuration permet d'améliorer la pureté de l'échantillon décomplexifié. En effet, après avoir concentré les particules non-spécifiques vers l'orifice de sortie 1360b, l'échantillon décomplexifié est injecté dans le second canal de séparation 1350' via les canaux 1370a et 1370c. L'échantillon décomplexifié est alors séparé une seconde fois des particules non-spécifiques pouvant être toujours présentes, celles-ci se retrouvant dans l'orifice 1360b'. L'échantillon décomplexifié une seconde fois est obtenu dans l'orifice de sortie 1360a.

Dans un mode de réalisation particulier du dispositif 1300, le séparateur 1322 est réalisé dans une plaquette de verre de 1mm d'épaisseur revêtue d'une couche de chrome et d'une couche de résine photosensible. Après développement de la couche photosensible, le verre est gravé par de l'acide fluorhydrique (HF). Les canaux d'acheminement 1340a et 1340c, 1370a, 1370c, 1370a' et 1370c' présentent une profondeur P de 125 $\mu$m, une largeur inférieure Lmin, au fond des canaux, de 300 $\mu$m, et d'une largeur supérieure Lmax de 550 $\mu$m dans le plan du séparateur en contact avec la plaque de recouvrement. Les canaux d'acheminement 1340b, 1340b', 1370b et 1370b' ainsi que les canaux de séparation 1350 et 1350' présentent une profondeur P de 125 $\mu$m, une largeur inférieure Lmin, au fond des canaux, de 375 $\mu$m, et une largeur supérieure Lmax de 625 $\mu$m dans le plan du séparateur en contact avec la plaque de recouvrement. La plaque de recouvrement est percée pour réaliser les orifices d'entrée et de sortie puis collée thermiquement avec le séparateur afin de réaliser un assemblage étanche.

Les canaux d'acheminement des orifices d'entrée 1340a, 1340b, présentent un angle de respectivement de 45°, 0° et moins 45° par rapport au canal de séparation 1350 dans le plan du séparateur, de manière à ralentir le débit des fluides provenant des canaux d'acheminement présentant un angle de 45 ° ou -45°, 1340a, 1340c, par rapport au canal de séparation 1350. Le canal de séparation 1350 rectiligne, présente une longueur de 19,5 mm, permettant aux cellules, particules ou molécules de devenir acoustiquement concentrés dans le canal d'acheminement 1370b lors du fonctionnement du système. L'axe du canal d'acheminement 1370b est identique à l'axe du canal de séparation.

Les canaux d'acheminement 1370a, 1370b, 1340b' présentent un angle de respectivement de 45°, 0° et moins 45° par rapport au canal de séparation 1350' dans le plan du séparateur, de manière à ralentir le débit des fluides provenant des canaux d'acheminement présentant un angle de 45 ° ou - 45°, 1340a, 1340c, par rapport au second canal de séparation 1350'. Le second canal de séparation 1350' rectiligne, présente une longueur de 19,5 mm, permettant aux cellules, particules ou molécules de devenir acoustiquement concentrés dans le canal d'acheminement 1370b' lors du fonctionnement du système. L'axe du canal d'acheminement 1370b' est identique à l'axe du canal de séparation 1350'. Les cellules, particules ou molécules sont acoustiquement concentrées dans le canal central 1370b', en fonction de leur densité, leur taille et leur compressibilité.

Alternativement, le séparateur 1322 du dispositif 1300 comprend quatre évidements (non représentés) réalisés le long des canaux de séparation 1350 et 1350', et susceptibles de recevoir chacun un transducteur à ultrasons (non représentés). Ce mode de réalisation permet l'obtention d'une onde stationnaire dans le séparateur 1322 si celui-ci est réalisé dans un matériau peu réfléchissant aux ondes acoustiques, le matériau étant souple et fin tel que des matériaux plastiques, polymères, silicones. Un silicone préférentiellement utilisé est le Polydiméthylsiloxane (PDMS). Préférentiellement, chaque transducteur à ultrasons comprend une lame ¼ onde adaptatrice dont l'impédance acoustique est calculée pour minimiser les pertes d'énergie, et donc l'échauffement, entre le transducteur à ultrasons et les parois du dispositif.

Préférentiellement la plaque de recouvrement (non représentée) est réalisée en PDMS ou en matériau plastique moulé tel que du Polycarbonate (PC), poly(méthacrylate de méthyle) (PMMA), polypropylène (PP), Polystyrène (PS), Acrylonitrile butadiène styrène (ABS), Copolymère d'Oléfine Cyclique (COC), Polymère d'Oléfine Cyclique (COP), Polyoxyméthylène (POM).

Ce dispositif 1300 est susceptible de mettre en œuvre des procédés de préparation d'échantillons biologiques par acoustophorèse tel que le procédé selon l'invention. Pour cela, tout ou partie de l'échantillon biologique à traiter, est introduit dans l'orifice d'entrée 1330a du dispositif 1300. Une solution tampon est introduite dans les orifices d'entrée 1330b et 1330b', les tampons et l'échantillon étant introduits à des débits respectifs aptes à générer un flux laminaire dans les canaux de séparations 1350 et 1350'. Un ou des transducteur(s) ultrasonore(s), accolé(s) aux canaux de séparation est (sont) alors activé(s) par un signal de commande, de manière à réaliser deux étapes successives de séparation dudit échantillon biologique par acoustophorèse. Cette séparation permet de favoriser une première fois la concentration des particules non-spécifiques, présents dans l'échantillon, dans l'orifice de sortie 1360b. A la suite de cette première séparation, un seconde séparation permet de favoriser une seconde fois la concentration des particules non-spécifiques, tel que des débris alimentaires toujours présentes dans l'échantillon décomplexifié en provenance des canaux 1370a et 1370c dans l'orifice de sortie 1360b'. L'échantillon décomplexifié une seconde fois et présentant une pureté améliorée étant obtenu dans l'orifice de sortie 1360a.

La position relative et la longueur du canal de seconde séparation 1350' vis-à-vis du canal de séparation principal 1350' est choisie en fonction des applications, des performances et des débits souhaités. Avantageusement, deux solutions tampons de type ou de densité différentes sont introduites dans les orifices d'entrée 1330b et 1330b'.

Comme représenté sur la figure 6d, l'invention concerne également un dispositif 1400 comprenant deux parties fluidiques, un séparateur 1422 et une plaque de recouvrement (non représentée), ces deux parties étant sensiblement planes. Ces deux parties sont assemblées de façon hermétique. Des canaux fluidiques sont réalisés dans le séparateur 1422. Le dispositif 1400 comprend deux orifices d'entrée 1430a, 1430b, en communication fluidique avec un premier canal de séparation 1450 par le biais de canaux d'acheminement respectivement 1440a, 1440c, pour l'orifice 1430a et 1440b pour l'orifice 1430b.

Le premier canal de séparation 1450 est également en communication fluidique avec un orifice de sortie 1460a, par le biais de canaux d'acheminement 1470a et 1470c.

Le premier canal de séparation 1450 est enfin en communication fluidique avec un second canal de séparation 1450', par le biais de canaux d'acheminement 1440a' et 1440c'.

Le dispositif 1400 comprend également un orifice d'entrée 1430b', en communication fluidique avec le second canal de séparation 1450 par le canal d'acheminement 1440b'.

Le second canal de séparation 1450' est également en communication fluidique avec deux orifices de sortie 1460b, 1460c, par le biais de canaux d'acheminement, respectivement 1470d pour l'orifice 1460c ; 1470e et 1470f pour l'orifice de sortie 1460b.

Le dispositif 1400 est agencé pour qu'un ou plusieurs transducteur(s) à ultrasons, non représenté(s), puisse être intégré(s) dans ou accolé(s) à une paroi desdits canaux de séparation 1450 et 1450'. Le(s) transducteur(s) à ultrasons ainsi intégré(s) ou accolé(s) est(sont) apte(s) à transmettre des oscillations mécaniques en ondes acoustiques multiples pouvant agir sur le contenu des canaux de séparation et y générer une onde acoustique stationnaire. Un générateur de courant alternatif associé à un amplificateur de signal, non représentés, peut être connecté électriquement au(x) transducteur(s) afin de générer un signal de commande dont la fréquence, la forme d'onde et l'amplitude sont connues.

Dans un mode de réalisation particulier du dispositif 1400, le séparateur 1422 est réalisé dans une plaquette de verre de 1mm d'épaisseur revêtue d'une couche de chrome et d'une couche de résine photosensible. Après développement de la couche photosensible, le verre est gravé par de l'acide fluorhydrique (HF). Les canaux d'acheminement 1440a, 1440c, 1470a, 1470c, 1470e et 1470f présentent une profondeur P de 125 $\mu$m, une largeur inférieure Lmin, au fond des canaux, de 300 $\mu$m, et une largeur supérieure Lmax de 550 $\mu$m dans le plan du séparateur en contact avec la plaque de recouvrement. Les canaux d'acheminement 1440b, 1470b et 1470d ainsi que les canaux de séparation 1450 et 1450' présentent une profondeur P de 125 $\mu$m, une largeur inférieure Lmin, au fond des canaux, de 375 $\mu$m, et une largeur supérieure Lmax de 625$\mu$m dans le plan du séparateur en contact avec la plaque de recouvrement. La plaque de recouvrement est percée pour réaliser les orifices d'entrée et de sortie puis collée thermiquement avec le séparateur afin de réaliser un assemblage étanche. Les canaux d'acheminement 1440a, 1440c, présentent un angle de respectivement de 45° et moins 45° par rapport au canal de séparation 1450 dans le plan du séparateur, de manière à ralentir le débit des fluides provenant de ces canaux d'acheminement. De la même manière, les canaux d'acheminement 1440a', 1440c', présentent un angle de respectivement de 45° et moins 45° par rapport au canal de séparation 1450' dans le plan du séparateur.

Les canaux de séparation 1450 et 1450' rectilignes, présente une longueur de 22,5 mm, permettant, lors du fonctionnement du système, aux cellules, particules ou molécules de devenir acoustiquement concentrées dans le canal d'acheminement 1470b puis 1470d, dont les axes sont identiques aux axes des canaux de séparation.

Alternativement, le séparateur 1422 du dispositif 1400 comprend quatre évidements (non représentés) réalisés le long des canaux de séparation 1450 et 1450', et susceptibles de recevoir chacun un transducteur à ultrasons (non représentés). Ce mode de réalisation permet l'obtention d'une onde stationnaire dans le séparateur 1422 si celui-ci réalisé dans un matériau peu réfléchissant aux ondes acoustiques, le matériau étant souple et fin tel que des matériaux plastiques, polymères, silicones. Un silicone préférentiellement utilisé est le Polydiméthylsiloxane (PDMS). Préférentiellement, chaque transducteur à ultrasons comprend une lame ¼ onde adaptatrice dont l'impédance acoustique est calculée pour minimiser les pertes d'énergie, et donc l'échauffement, entre le transducteur à ultrasons et les parois du dispositif.

Préférentiellement la plaque de recouvrement (non représentée) est réalisée en PDMS ou en matériau plastique moulé tel que du Polycarbonate (PC), poly(méthacrylate de méthyle) (PMMA), polypropylène (PP), Polystyrène (PS), Acrylonitrile butadiène styrène (ABS), Copolymère d'Oléfine Cyclique (COC), Polymère d'Oléfine Cyclique (COP), Polyoxyméthylène (POM).

Ce dispositif 1400 est susceptible de mettre en œuvre des procédés de préparation d'échantillons biologiques par acoustophorèse tel que le procédé selon l'invention. Pour cela, tout ou partie de l'échantillon biologique à traiter, est introduit dans l'orifice d'entrée 1430a du dispositif 1400. Une solution tampon est introduite dans l'orifice d'entrée 1430b, le tampon et l'échantillon étant introduits à des débits respectifs aptes à générer un flux laminaire dans le canal de séparation 1450. Un transducteur ultrasonore, tel qu'un transducteur piézoélectrique, accolé au canal de séparation 1450 est alors activé par un signal de commande, de manière à réaliser une étape de séparation dudit échantillon biologique par acoustophorèse. Cette séparation permet de favoriser la concentration des particules non-spécifiques, présents dans l'échantillon, dans le canal d'acheminement 1470b dudit dispositif d'acoustophorèse. L'échantillon décomplexifié est obtenu dans l'orifice de sortie 1460a. Une seconde étape de séparation dudit échantillon concentré par acoustophorèse est également réalisée sur la partie résultante à la première séparation dans le canal 1450'. Pour cela un second tampon propre est introduit simultanément dans l'orifice d'entrée 1430b' afin d'extraire les espèces d'intérêt résiduelle toujours présente dans l'échantillon concentré. Cette séparation permet de favoriser la concentration des particules non-spécifiques, toujours présents dans l'échantillon concentré, dans l'orifice de sortie 1460c dudit dispositif d'acoustophorèse. L'échantillon concentré et alors décomplexifié une seconde fois puis obtenu dans l'orifice de sortie 1460b. La collection et le mélange des échantillons décomplexifiés issus des orifices de sortie 1460a et 1460b permettent d'augmenter le rendement de collection des microorganismes présents initialement dans l'échantillon biologique. En effet les microorganismes entraînés avec les particules non spécifiques lors de la première séparation vers le canal 1450' via les canaux 1440a' et 1440c' sont susceptibles d'être séparés par acoustophorèse dans ce canal 1450' et de pouvoir ainsi être collectés suite à cette seconde séparation dans l'orifice 1460b. Le rendement d'extraction des microorganismes et ainsi amélioré.

La position relative et la longueur du canal de seconde séparation 1450' vis-à-vis du canal de séparation principal 1450' est choisie en fonction des applications, des performances et des débits souhaités. Avantageusement, deux solutions tampons de type ou de densité différentes sont introduites dans les orifices d'entrée 1430b et 1430b'.

Quel que soit le mode de réalisation du dispositif selon l'invention, le dispositif peut comprendre, autour du canal d'acheminement vers un orifice de sortie contenant l'échantillon décomplexifié, des supports d'aimants mobiles permettant la capture d'échantillon mise en présence de particules magnétiques tel que de la silice magnétique. Comme représenté sur la figure 7a en sortie du dispositif 300, à proximité de l'orifice 360a, deux supports d'aimants mobiles 2000 sont disposés. Les aimants ainsi disposés de part et d'autres du canal d'acheminement vers l'orifice de sortie 360a permettent de capturer en flux continu des microorganismes par déplacement de particules de silice magnétiques 2100 de manière perpendiculaire au flux de l'échantillon décomplexifié. Le mouvement M des supports d'aimant s'effectue par exemple perpendiculairement au canal et de façon alternative entre les deux supports afin de favoriser le mélange de l'échantillon et des particules.

Quel que soit le mode de réalisation du dispositif selon l'invention, le dispositif peut comprendre, autour du canal d'acheminement vers un orifice de sortie contenant l'échantillon décomplexifié, des électrodes de Di ElectroPhorese (DEP) déposées sur la surface du dispositif au niveau de l'orifice de sortie. Comme représenté sur la figure 7b en sortie du dispositif 300, à proximité de l'orifice 360a, deux électrodes de Di ElectroPhorese (DEP) 3000 sont disposées afin de permettent la réalisation d'une étape de capture spécifique. Ces électrodes peuvent être soient fonctionnalisées avec des antigènes de captures dans le cas d'une capture sur un panel d'espèces recherchées, ou non fonctionnalisées utilisant alors uniquement les propriétés bien connues de capture par la charge de la technique DEP. L'identification des espèce(s) d'intérêt capturée(s) peut être dans les deux cas de figure confirmée ou effectuée par exemple par identification Raman 3100.

Comme représenté sur la figure 8, un sixième dispositif 500 comprenant deux parties fluidiques, un séparateur 522 et une plaque de recouvrement 521, ces deux parties étant sensiblement planes. Ces deux parties sont assemblées de façon hermétique. Des canaux fluidiques sont réalisés dans le séparateur 522. Le dispositif 500 comprend deux orifices d'entrée 530a, 530b, réalisés dans la plaque de recouvrement 521, en communication fluidique avec au moins un canal de séparation 550 par le biais de canaux d'acheminement respectivement 540a, 540c, pour l'orifice 530a et 540b pour l'orifice 530b. Le canal de séparation 550 est également en communication fluidique avec deux orifices de sortie 560a, 560b, par le biais de canaux d'acheminement, respectivement 570a et 570c pour l'orifice 560a, 570b pour l'orifice 560b. Les orifices d'entrée et de sortie forment des réservoirs de manière à pouvoir réaliser des opérations de dépose d'un échantillon à décomplexifier par pipetage et de la solution tampon ainsi que des opérations de connexion à un système de mise en pression ou vide asservi et régulé sur la mesure de débit et permettant de piloter de manière reproductible l'introduction et l'écoulement des échantillons biologique et du tampon dans le canal de séparation. Le dispositif 500 est agencé pour qu'un transducteur à ultrasons, non représenté, puisse être intégré dans ou accolé à une paroi dudit canal de séparation 550. Le transducteur à ultrasons ainsi intégré ou accolé est apte à transmettre des oscillations mécaniques en ondes acoustiques multiples pouvant agir sur le contenu du canal de séparation et y générer une onde acoustique stationnaire. Un générateur de courant alternatif non représenté peut être connecté électriquement au transducteur afin de générer un signal de commande du transducteur dont la fréquence, la forme d'onde et l'amplitude sont connues.

Dans un mode de réalisation particulier du dispositif 500, Le séparateur 522 est réalisé dans une plaquette de verre de 1mm d'épaisseur revêtue d'une couche de chrome et d'une couche de résine photosensible. Après développement de la couche photosensible, le verre est gravé par de l'acide fluorhydrique (HF). Les canaux d'acheminement 540a et 540c, 570a, 570c, présentent une profondeur P de 125 $\mu$m, une largeur inférieure Lmin, au fond des canaux, de 300 $\mu$m, et une largeur supérieure Lmax de 550 $\mu$m dans le plan du séparateur en contact avec la plaque de recouvrement. Les canaux d'acheminement 540b, 570b ainsi que le canal de séparation 550 présentent une profondeur P de 125 $\mu$m, une largeur inférieure Lmin, au fond des canaux, de 375 $\mu$m, et une largeur supérieure Lmax de 625$\mu$m dans le plan du séparateur en contact avec la plaque de recouvrement. La plaque de recouvrement est percée pour réaliser les orifices d'entrée et de sortie puis collée thermiquement avec le séparateur afin de réaliser un assemblage étanche. Les canaux d'acheminement des orifices d'entrée (540a, 540b, 540c) présentent un angle de respectivement de 45°, 0° et moins 45° par rapport au canal de séparation 550 dans le plan du séparateur, de manière à ralentir le débit des fluides provenant des canaux d'acheminement présentant un angle de 45 ° ou -45°, 540a, 540c, par rapport au canal de séparation. Le canal de séparation 550 rectiligne, présente une longueur de 80 mm, permettant aux cellules, particules ou molécules de devenir acoustiquement concentrés dans le canal d'acheminement 570b lors du fonctionnement du système. L'axe du canal d'acheminement 570b est identique à l'axe du canal de séparation 550.

Alternativement, le séparateur 522 du dispositif 500 comprend deux évidements, 590a, 590b, réalisés le long du canal de séparation 550, et susceptibles de recevoir chacun un transducteur à ultrasons (non représentés). Ce mode de réalisation permet l'obtention d'une onde stationnaire dans le séparateur 522 si celui-ci réalisé dans un matériau peu réfléchissants aux ondes acoustiques, le matériau étant souple et fin tel que des matériaux plastiques, polymères, silicones. Un silicone préférentiellement utilisé est le Polydiméthylsiloxane (PDMS). Préférentiellement, chaque transducteur à ultrasons comprend une lame ¼ onde adaptatrice dont l'impédance acoustique est calculée pour minimiser les pertes d'énergie, et donc l'échauffement, entre le transducteur à ultrasons et les parois du dispositif.

Préférentiellement la plaque de recouvrement (non représentée) est réalisée en PDMS ou en matériau plastique moulé tel que du Polycarbonate (PC), poly(méthacrylate de méthyle) (PMMA), polypropylène (PP), PolyStyrene (PS), Acrylonitrile butadiène styrène (ABS), Copolymère d'Oléfine Cyclique (COC), Polymère d'Oléfine Cyclique (COP), Polyoxyméthylène (POM).

Ce dispositif 500 est susceptible de mettre en œuvre des procédés de préparation d'échantillons biologiques par acoustophorèse . Pour cela, tout ou partie de l'échantillon biologique à traiter est introduit dans l'orifice d'entrée 530a du dispositif 500. Une solution tampon est introduite dans l'orifice d'entrée 530b, le tampon et l'échantillon étant introduits à des débits respectifs aptes à générer un flux laminaire dans le canal de séparation. Un transducteur ultrasonore, tel qu'un transducteur piézoélectrique, accolé au canal de séparation est alors activé par un signal de commande, de manière à réaliser une étape de séparation dudit échantillon biologique par acoustophorèse. Cette séparation permet de favoriser la concentration des particules non-spécifiques, tel que des éléments figurés du sang, présents dans l'échantillon, dans l' orifice de sortie 560b dudit dispositif d'acoustophorèse. L'échantillon décomplexifié étant obtenu dans l'orifice de sortie 560a. L'échantillon concentré étant obtenu dans l'orifice de sortie 560b (zone poubelle)..

La figure 9 présente un support 600 pour dispositifs avec un dispositif 500 disposé et maintenu sur le support.. Ce support permet par exemple de traiter un même échantillon biologique simultanément sur plusieurs dispositifs ou plusieurs échantillons différent simultanément en connectant un ou des réservoir(s) contenant l'(les) échantillon(s) tel qu'une seringue aux différents orifices d'entrée des dispositifs disposés sur le support. Alternativement, le support peut comprendre un capot articulé, non représenté, permettant de connecter directement les orifices d'entrée et de sortie du dispositif à des moyens de mise en pression et/ou vide, à la fermeture du capot. Ce support comprend un ou plusieurs socles de réception (non représentés) de dispositifs, permettant de placer et maintenir lesdits dispositifs. Le maintien des dispositifs peut être réalisé mécaniquement ou par mise en aspiration des dispositifs sur le(s) socle(s) de réception. Des transducteurs ultrasonores 690 peuvent être disposés dans ces socles de manière à pouvoir créer un onde stationnaire dans chacun des dispositifs disposés sur le support. Le transducteur étant accolé au dispositif quand celui-ci est disposé dans ledit socle. Alternativement un seul transducteur ultrasonore recouvrant plusieurs dispositif peut être disposé de manière à pouvoir créer un onde stationnaire dans chacun des dispositifs disposés sur le support.

Un espace de réception 610 permet notamment d'accoler un système de chauffage (non représenté) à au moins un des orifices de sortie du dispositif disposé sur le support. Ledit système de chauffage permettant d'incuber l'échantillon biologique traité et contenu dans au moins un des orifices de sortie 560a, 560b. De plus, l'espace de réception 610 peut comprendre un moyen de mesure de la densité optique de l'échantillon contenu dans au moins un des orifice de sortie 560a, de manière à éviter des manipulations supplémentaires pour réaliser cette opération. D'autre part, le support peut être disposé directement sur un agitateur ou comprendre un moyen d'agitation de manière à réaliser une étape d'agitation de l'échantillon biologique contenu dans un des orifices de sortie, avant, pendant ou suite à une étape d'incubation.

Ce support 600 permet également de disposer et maintenir différents dispositifs 200, 300, 400, 500, 800, l'espace de réception 610 pouvant être facilement adapté par l'homme du métier.

La figure 10 présente un support 700 pour dispositifs représenté avec quatre dispositifs 500 disposés et maintenus sur le support. Ce support permet par exemple de traiter un même échantillon biologique simultanément sur plusieurs dispositifs en connectant un réservoir contenant l'échantillon biologique tel qu'une seringue aux différents orifices d'entrée 530a des dispositifs disposés sur le support. Alternativement, le support permet de disposer les dispositifs de manière à présenter chacun des orifices d'entrée et de sortie alignés sur un même axe et selon un écartement défini. Cet écartement peut avantageusement être équivalent ou multiple à celui du pas d'une microplaque connue de l'homme du métier. De cette manière, les opérations de pipetage, automatiques ou manuelles, peuvent être réalisées directement dans plusieurs dispositifs simultanément, notamment à l'aide d'une pipette ou un distributeur à microplaques. Ce support comprend plusieurs socles de réception (non représentés) de dispositifs , permettant de placer et maintenir lesdits dispositifs. Des transducteurs ultrasonores (non représentés), ou un transducteur commun à plusieurs dispositifs, peuvent être disposés dans ces socles de manière à pouvoir créer un onde stationnaire dans chacun des dispositifs disposés sur le support. Le transducteur étant accolé au dispositif quand celui-ci est disposé dans ledit socle. De façon identique au support 600, le support 700 peut comprendre plusieurs espaces de réception (non représentés) permettant notamment d'accoler à au moins un des orifices de sortie de chacun des dispositifs disposés sur le support, un système de chauffage (non représentés) permettant d'incuber l'échantillon biologique traité et contenu dans au moins un des orifices de sortie 560a, 560b. De plus, chacun des espaces de réception peut comprendre un moyen de mesure de la densité optique de l'échantillon contenu dans au moins un des orifices de sortie 560a, de manière à éviter des manipulations supplémentaires pour réaliser cette opération. D'autre part, le support 700 peut être disposé directement sur un agitateur ou comprendre un moyen d'agitation de manière à réaliser une étape d'agitation de l'échantillon biologique contenu dans un des orifices de sortie, avant, pendant ou suite à une étape d'incubation. Ce support 700 permet également de disposer parallèlement plusieurs des différents dispositifs 200, 300, 400, 500, 800, le nombre de socles de réception pouvant être facilement adapté par l'homme du métier. Les différents dispositifs disposés sur le support 700 peuvent être de même type ou de types différents.

Comme représenté sur les figures 11, 12a et 12b, un dispositif multiplexe 800 comprenant trois parties fluidiques, un séparateur 822, une plaque de recouvrement 821 et un socle 823, ces trois parties étant sensiblement planes. Ces trois parties sont assemblées de façon hermétique. Des canaux fluidiques sont réalisés dans le séparateur 822. Le dispositif 800 comprend deux orifices d'entrée 830a, 830b, en communication fluidique avec huit canaux de séparation 850, par le biais d'un réseau de canaux introduction 841, 842, et de canaux d'acheminement. Ainsi, les canaux de séparation 850 communiquent avec l'orifice 830a via le réseau de canaux d'introduction 841 ainsi que par les canaux d'acheminement 840a, 840c. De la même manière, les canaux de séparation 850 communiquent avec l'orifice 830b via le réseau de canaux d'introduction 842 ainsi que par le canaul d'acheminement 840b . Les canaux de séparation 850 sont également en communication fluidique avec deux orifices de sortie 860a, 860b, par le biais d'un réseau de canaux d'aspiration 843, 844 et de canaux d'acheminement. Ainsi, les canaux de séparation 850 communiquent avec l'orifice 860a via le réseau de canaux d'aspiration 843 ainsi que par les canaux d'acheminement, 870a, 870c.De la même manière, les canaux de séparation 850 communiquent avec l'orifice 860b via le réseau de canaux d'aspiration 844 ainsi que par le canal d'acheminement 870b. Huit canaux de séparation 850 sont représentés, chacun des canaux 850 et des canaux d'acheminement associés formant un motif fluidique répété huit fois.

Le dispositif 800 est agencé pour qu'un transducteur à ultrasons, non représenté, puisse être intégré dans ou accolé à une paroi desdits canaux de séparation 850. Le transducteur à ultrasons ainsi intégré ou accolé est apte à transmettre des oscillations mécaniques en ondes acoustiques multiples pouvant agir sur le contenu de chaque canal de séparation et y générer une onde acoustique stationnaire. Un générateur de courant alternatif non représenté peut être connecté électriquement au transducteur afin de générer un signal de commande du transducteur dont la fréquence, la forme d'onde et l'amplitude sont connues. Alternativement, des ouvertures 880 traversantes ou des gravures d'une profondeur supérieure ou égale à celle des canaux de séparation sont effectuées dans le séparateur.

Ces ouvertures ou gravures sont réparties de chaque côté des canaux de séparations 850. Ces ouvertures 880 peuvent être utilisées entre les différents canaux de séparation et permettent de mieux séparer acoustiquement les résonances de chaque canal de séparation.

Les réseaux de canaux d'introduction 841, 842 permettent de diviser le flux, par exemple d'échantillon biologique ou de tampon introduit dans les orifices d'entrée 830a et 830b. Cette parallélisation des canaux de séparation permet de traiter un volume d'échantillon par acoustophorèse à des débits plus important que sur un dispositif classique sans dégrader les performances d'extraction par l'augmentation du débit d'échantillon (en $\mu$L/mn). Ainsi si les réseaux divisent par deux le flux d'échantillon introduit, acheminant l'échantillon vers deux canaux de séparation, un débit deux fois supérieur peut être atteint pour un même taux de décomplexification. De la même manière, les réseaux d'aspiration 843, 844, permettent de collecter les échantillons ou tampon traités dans un seul orifice de sortie. Ces réseaux permettent également d'assurer un équilibre de pression en aval de l'étape de séparation par acoustophorèse.

Ce dispositif 800 est susceptible de mettre en œuvre des procédés de préparation d'échantillons biologiques par acoustophorèse. Pour cela, tout ou partie de l'échantillon biologique à traiter, est introduit dans l'orifice d'entrée 830a du dispositif 800. Une solution tampon est introduite dans l'orifice d'entrée 830b, le tampon et l'échantillon étant introduits à des débits respectifs aptes à générer un flux laminaire dans les canaux de séparation. Un transducteur ultrasonore, tel qu'un transducteur piézoélectrique, accolé aux canaux de séparation est alors activé par un signal de commande, de manière à réaliser une étape de séparation dudit échantillon biologique par acoustophorèse. Cette séparation permet de favoriser la concentration des particules non-spécifiques, tel que des éléments figurés du sang présents dans l'échantillon, dans l'orifice de sortie 860b. L'échantillon décomplexifié étant obtenu dans l'orifice de sortie 860a. L'échantillon concentré étant obtenu dans l'orifice de sortie 860a. Ce dispositif présentant huit canaux de séparations, un débit de traitement d'échantillon biologique huit fois supérieur à un dispositif de l'état de l'art peut être atteint sur un même dispositif. De la même manière, un volume d'échantillon (en mL) huit fois plus important peut être traité dans un temps identique sans modification de débit ($\mu$L/min) conservant ainsi les performances d'extraction intactes.

De façon plus générale, ce cinquième dispositif multiplexe comprend deux orifices d'entrée en communication fluidique avec au moins deux canaux de séparation, par le biais d'un réseau de canaux introduction et de canaux d'acheminement. Lesdits canaux de séparation communiquent également avec deux orifices de sortie par le biais d'un réseau de canaux d'aspiration et de canaux d'acheminement. Ce motif de réalisation associé à chacun des canaux de séparation permet d'adapter le débit de traitement de l'échantillon en fonction de l'application souhaitée, en multipliant le nombre de canaux de séparation parallélisés et de canaux d'acheminement associés.

Comme représenté sur les figures 13a et 13b, dispositif de connexion 900 permettant de connecter des orifices d'un dispositif microfluidique à un moyen d'introduction ou d'aspiration. Ce dispositif est représenté connecté à un dispositif 10 de l'état de l'art.

Le dispositif de connexion 900 comprend un connecteur d'entrées 910, et un connecteur de sorties 920. Le connecteur d'entrées 910 comprend trois canaux d'acheminement 930a, 930b, 930c aptes à coopérer avec les orifices d'entrée du dispositif 10, respectivement 30a, 30b, 30c. Le connecteur d'entrée 910 permet ainsi de venir connecter des moyens d'introduction d'échantillon biologique ou de solution tampon sans manipuler directement le dispositif microfluidique 10. Par exemple, un tube d'introduction d'une seringue 931b, visible sur la figure 13b, est connecté au canal d'acheminement 930b afin de pouvoir introduire une solution dans l'orifice 30b. Un arrangement similaire est réalisé en regard des orifices d'entrée 30a et 30c afin de pouvoir introduire l'échantillon biologique dans les orifices d'entrée 30a et 30c.

Le connecteur de sorties 920 comprend trois canaux d'acheminement 960a, 960b, 960c aptes à coopérer avec les orifices de sortie du dispositif 10, respectivement 60a, 60b, 60c. Le connecteur de sorties 920 permet ainsi de venir connecter des moyens d'aspiration ou des moyens de collecte d'échantillon biologique ou de solution tampon sans manipuler directement le dispositif microfluidique 10. Par exemple, un tube de collecte 961b visible sur la figure 13b, tel qu'un tube « Eppendorf », est disposé en regard du canal d'acheminement 960b afin de pouvoir collecter la solution en provenance de l'orifice de sortie 60b. Un arrangement similaire est réalisé en regard des orifices de sortie 60a et 60c afin de collecter l'échantillon décomplexifié directement dans des tubes de collecte.

Le dispositif 10 est disposé entre les connecteurs 910 et 920 et un support, non représenté. Les connecteurs 910 et 920 sont maintenus sur ledit support par tout moyen, notamment par vissage, par montage en force ou par des ressorts de rappels, de manière à assurer une connexion hermétique avec les orifices d'entrée et de sortie du dispositif. Avantageusement, les connecteurs comprennent des joints toriques 990 afin de garantir cette étanchéité quand le connecteur est maintenu.

Bien entendu, la géométrie de ce dispositif de connexion 900, des connecteurs 910 et 920 et le nombre de canaux d'acheminement des connecteurs pourront aisément être adaptés pour être utilisé avec un ou des dispositifs 200, 300, 400, 500, 800.

Comme représenté sur la figure 14, un système de régulation d'air 1000 et des dispositifs de connexion 1100 associés permettant la réalisation de la méthode selon l'invention par asservissement de l'introduction et de l'aspiration des liquides introduits. Le système est ici représenté avec un dispositif 10 de l'état de l'art représenté ici selon la coupe A-A de la figure 1. Le système de régulation 1000 comprend un générateur de pression ou de vide 1010. Le générateur 1010 comprend deux sorties de pression 1020a et 1020b susceptibles de délivrer la pression d'air nécessaire à l'introduction de l'échantillon biologique ou de la solution tampon d'une méthode selon l'invention à un débit régulé dans un dispositif microfluidique. Le générateur 1010 comprend également deux entrées de vide 1040a et 1040b susceptibles de générer un différentiel de pression d'air suffisant pour aspirer l'échantillon biologique et la solution tampon d'une méthode selon l'invention à un débit régulé dans un dispositif microfluidique. A ces fins, les entrées de vide 1040a et 1040b sont connectées au dispositif via deux capteurs de débits d'air 1050a et 1050b afin de mesurer et d'asservir la régulation de pression d'air introduit ou le vide d'aspiration de l'échantillon et de la solution tampon d'une méthode selon l'invention. Cette régulation permet ainsi d'obtenir un débit constant de séparation et de décomplexification de l'échantillon biologique en limitant les risques de perte de charge.

Dans ce but, le générateur est connecté aux orifices d'entrée et de sortie du dispositif par l'intermédiaire du dispositif de connexion 1100. Ce dispositif comprend un connecteur d'entrée par orifice d'entrée ainsi qu'un connecteur de sortie par orifice de sortie. Seul les connecteurs d'entrée 1120b et 1140b sont représentés par souci de clarté de la figure.

Le dispositif de connexion 1100 comprend également un capot 1110 disposé et maintenu sur le dispositif 10. Le capot 1110 comprend un réservoir en regard de chacun des orifices d'entrée et de sortie du dispositif 10. Seul le réservoir 1130b coopérant avec l'orifice 30b et le réservoir 1160b coopérant avec l'orifice 60b sont représentés par souci de clarté de la figure. Le capot 10 peut être réalisé en matière plastique, par exemple par injection et permet de capoter un dispositif afin de présenter en regard de chaque orifice d'entrée et de sortie du dispositif maintenu des réservoirs. Ces réservoirs peuvent notamment être utilisés pour la dépose et la collecte d'échantillons et de solutions tampons. Le capot est maintenu sur un support, non représenté, de manière à réaliser une connexion hermétique avec les orifices du dispositif 10. Le capot peut être maintenu sur le support par tout moyen, notamment par vissage, par montage en force ou par la pression d'un ressort de rappel.

Un connecteur d'entrée 1120b est apte à être disposé et maintenu sur le capot 1110 de manière à coopérer avec le réservoir 1130b dudit capot. Un connecteur de sortie 1140b est également apte à être disposé et maintenu sur le capot 1110 de manière à coopérer avec le réservoir 1160b dudit capot. Les connecteurs présentent avantageusement des joints toriques d'étanchéité et sont maintenus sur le capot par des vis ou des ressorts. Les connecteurs peuvent être maintenus sur le capot par tout moyen, notamment par vissage, par montage en force ou par la pression d'un ressort de rappel.

Bien entendu, la géométrie de ce dispositif de connexion 1100 et notamment des connecteurs pourra aisément être adaptée pour être utilisé avec un dispositif 200, 300, 400, 500, 800.

Les exemples ci-après permettront de mieux appréhender la présente invention. Toutefois, ces exemples ne sont donnés qu'à titre illustratif et ne doivent en aucun cas être regardés comme limitant la portée de ladite invention d'une quelconque manière.

Exemple : traitement d'échantillons sanguins selon la méthode

A) Assemblage d'un dispositif et d'un système

Un dispositif microfluidique est réalisé conformément au dispositif représenté sur la figure 1.

Comme illustrés sur la figure 3, les trois orifices d'entrées 30a, 30b, 30c, et les trois orifices de sorties 60a, 60b, 60c, sont connectés à des moyens d'introduction, tel que des seringues (Becton Dickinson Plastipak TM, Espagne) via un assemblage comprenant un embout de pipette (Eppendorf, Royaume-Uni) connecté à un tube de Tygon (0,03 en ID, 0,09 en OD, Cole-Parmer, Royaume-Uni). Un embout de pipette est collé hermétiquement à chacun des orifices, un tube de tygon est alors inséré puis collé dans l'embout de pipette et enfin assemblé avec l'embout de la seringue. De cette manière, trois seringues d'introduction 130a, 130b, 130c, permettent d'introduire un fluide dans chacun des orifices d'entrée 30a, 30b, 30c, tandis que trois moyens d'aspiration tel que des seringues 140a, 140b, 140c, permettent d'aspirer le fluide dans chacun des orifices de sortie 60a, 60b, 60c. Alternativement, un dispositif de connexion 900 peut être utilisé pour connecter chaque seringue au dispositif.

Les trois seringues d'aspiration 140a, 140b, 140c, permettent d'assurer un équilibre de pression, et de débit, dans les trois canaux d'acheminement aux orifices de sorties, en aval du canal de séparation. De cette manière, le dispositif est moins sujet à d'éventuels bouchage pouvant être dus à des particules de taille importante entravant la circulation du fluide dans un des canaux.

Des pompes à seringues (Harvard Apparatus, Royaume-Uni), non représentées, sont utilisés pour contrôler les débits d'introduction des échantillons et tampon.

Un transducteur piézo-électrique d'une dimension de 10mm × 30 mm en céramique (Pz26, Ferroperm Piezoceramics, USA) avec une plage de fréquences de résonance de 1 MHz et 2 MHz est fixé au dispositif 10 par un gel à ultrasons (Anagel™ Royaume-Uni). Ce transducteur permet de générer des ultrasons utilisés pour induire une onde stationnaire entre les parois du canal de séparation. Un mouvement des particules induit par le phénomène d'acoustophorèse peut ainsi être obtenu par l'application d'un courant alternatif (AC) au transducteur. Ce signal de commande est introduit dans le circuit par un générateur de fonction (Agilent, modèle 33210A, 10 MHz sur la fonction / générateur de forme d'onde arbitraire, Royaume-Uni) en mode de fonctionnement sinusoïdal. Un amplificateur (Amplifier research, modèle 1W1000B, 1-1000 MHz, AR) est utilisé pour amplifier le signal provenant du générateur de fonction et obtenir l'amplitude nécessaire pour entraîner les particules. Un oscilloscope (Tektronix, modèle 1042 SCT, USA) est connecté en parallèle avec le capteur pour mesurer la tension de fonctionnement.

**B) Préparation d' échantillons sanguins inoculés.**

Afin d'éviter des problèmes de bouchage du dispositif microfluidique employé, du sang de cheval défibriné a été employé. 9mL de sang de cheval (Oxoid, ThermoScientific, UK) défibriné par un filtre Porex (porosité de 25 $\mu$m) puis mélangés avec 1ml de citrate trisodique à 3,3 p/v% .

Afin de préparer un échantillon inoculé contenant un inoculum contrôlé d'organismes pathogène, une colonie isolée de Salmonella typhimurium (NCTC 12023/ATCC® 14028, Pro-lab Diagnostics, UK) isolée à partir d'une culture sur milieu de culture Trypticase Soja Agar (TSA ; bioMérieux, France) est dilué dans 9 ml de bouillon Bouillon Trypticase Soja (TSB ; bioMérieux, France) puis incubée à 37 ° C pendant 18 h.

Environ 0,4 ml de bouillon enrichi ainsi obtenu est dilué dans 9 ml de BPW et son absorbance à 600 nm mesurée (appareil CECIL 1011, série 1000) pour obtenir une lecture de 0,100 correspondant à environ $1.0 \times 10^9$ UFC mL-1. A partie de ce bouillon enrichi, 6 séries de dilutions sont ensuite réalisées et numérotées de d-1 à d-6 correspondant à des concentrations de $10^8$ à $10^3$ UFC. mL$^{-1}$, (UFC signifiant Unité Formant Colonies) .

Afin de déterminer la quantité de pathogènes présents dans le lots d'échantillon contenant un inoculum contrôlé ainsi obtenu, un milieu de culture TSA (inoculums contrôlé; TSA x2) est ensemencé avec 100 $\mu$l de d-6 et incubée à 37 ° C pendant une nuit. Cette étape est réalisée en doublon. Le nombre réel de microorganismes présents dans l'échantillon initial a été calculé en fonction de la dilution et du nombre de colonies comptées sur le milieu.

0,1 ml de $10^9$ UFC.mL$^{-1}$ de Salmonella typhimurium est par la suite inoculé dans le mélange de sang, préparé comme indiqué ci-dessus, pour obtenir une concentration finale de Salmonella typhimurium de $10^7$ UFC.mL$^{-1}$. Le mélange inoculé de sang à $10^7$ UFC.mL$^{-1}$ de Salmonella typhimurium a ensuite été dilué avec une solution tampon de phosphatase (PBS ou « Phosphate Buffered Saline » en langue anglaise) (1: 1 en rapport de volume) et utilisé pour les exemples suivants.

Cette préparation équivaut à une concentration classique en microorganismes dans le cas d'une hémoculture positive.

**C) Etape de décomplexification par acoustophorèse**

La récupération des cellules sanguines après leur traitement dans un dispositif microfluidique par un procédé selon l'invention a été déterminée en utilisant un hémocytomètre. La suspension recueillie auprès de chaque orifice de sortie a été dilué avec une solution de PBS jusqu'à ce que le nombre de cellules comptées à partir de chaque carré d'une cellule de comptage (type Thoma ou Malasse) se situe entre 50 et 100. Le taux de réjection des cellules sanguines dans l'orifice de sortie disposé dans l'axe du canal de séparation est calculé par:

$$\text{Taux de réjection (\%)} = 100x \frac{\text{(nombre de cellules sanguines dans l'orifice de sortie considéré)}}{\text{(nombre total de cellules sanguines dans tous les orifices de sorties)}}$$

D'autres indicateur sont :

- la qualité de l'extraction, qui sera donnée par la composition de chaque sortie du dispositif (méthode d' analyse par cytométrie en flux).

- Le rendement d'extraction qui représente le pourcentage de bactéries séparées par rapport à la concentration de l'échantillon de départ (méthode d' analyse par étalement sur boîte et comptage).

- La croissance bactérienne indique si le tri acoustique permet de conserver la viabilité des micro-organismes sans leur imposer un stress trop important (méthode d'analyse :mesure de Densité Optique de l'échantillon décomplexifié après extraction et après incubation à 37°C)

Le mélange inoculé de sang à $10^7$ UFC.mL$^{-1}$ de Salmonella typhimurium est dilué dans du PBS dans un rapport de 1 :1, préparé selon la description ci-dessus est filtré par un filtre Porex puis introduit dans les orifices d'entrés 30a et 30c, d'un dispositif 10 de l'état de l'art, tel que décrit précédemment et représenté sur la figure 1. Les orifices d'entrée 30a et 30c sont également appelés entrées latérales Le dispositif 10 est connecté à des seringues d'introduction 130a, 130b, 130c, et d'aspiration 140a, 140b, 140c, tel que décrit précédemment et selon le figure 3. Un transducteur ultrasonore 90 est accolé au canal de séparation 50. Un générateur d'onde 110 permet de transmettre au transducteur ultrasonore 90, ici un piézoélectrique, un signal de commande. Un tampon phosphate salin (PBS) est introduit dans l'orifice d'entrée 30b, également appelé entrée centrale. L'étape de filtration permet de limiter la présence de particules pouvant boucher ou encombrer les canaux du dispositif et perturber l'écoulement laminaire des fluides dans le canal de séparation.

L'échantillon sanguin est introduit à un débit de 10 μL.min$^{-1}$ dans les entrées latérales. Le tampon phosphate salin (PBS) est introduit à un débit de 70 μL.min$^{-1}$ dans l'entrée centrale. Un débit d'aspiration de 30 μL.min$^{-1}$ est assuré par les seringues d'aspiration dans les orifices de sortie 60a, 60b, 60c.

Dans le cas où aucun signal de commande n'est transmis au transducteur ultrasonore 90, l'écoulement est laminaire tout au long du canal de séparation est toutes les particules de l'échantillon introduit dans l'orifice d'entrée 30a se déplacent totalement vers l'orifice de sortie 60a. De même toutes les particules de l'échantillon introduites dans l'orifice d'entrée 30c se déplacent totalement vers l'orifice de sortie 60c. Cet écoulement est réalisé pendant une durée de 40 min. Ainsi, tout au long de l'expérience, une quantité très faible de cellules sanguines est observée dans l'orifice de sortie 60b. Cet écoulement est visible sur la figure 15a, les cellules sanguines étant plus sombres sur l'image et concentrées uniquement dans les canaux d'acheminements 70a et 70c, vers les orifices de sorties,respectivement 60a et 60c.

Dans un second temps, l'expérience est renouvelée selon le même protocole, en appliquant un signal de commande d'une fréquence de 1,300 MHz, et d'amplitude de 36,6 V crête à crête (Vp-p) au transducteur piézoélectrique. L'écoulement est alors laminaire à l'entrée du canal de séparation puis on observe une migration des cellules sanguines vers le centre du canal de séparation. Ainsi, toutes les cellules sanguines de l'échantillon introduit dans les orifices d'entrées 30a et 30c se déplacent vers l'orifice de sortie 60b. Cet écoulement est réalisé pendant une durée de 40 min. Cet écoulement est visible sur la figure 15b, les cellules sanguines étant plus sombres sur l'image est concentrées uniquement dans le canal d'acheminement 70b vers l'orifice de sortie 60b. Seuls restent dans les canaux d'acheminements 70a et 70c un mélange de tampon PBS et de plasma, susceptible de contenir les microorganismes présents dans l'échantillon introduit dans le dispositif.

Cette expérience est effectué deux fois et permet de démontrer un taux de réjection de 99,82% de cellules sanguines dans l'orifice de sortie 60b (% de coefficient de variation = 0,043). Cet expérience démontre la capacité du procédé a séparer efficacement les cellules sanguines du plasma a un débit constant.

## D) récupération de microorganismes présents dans l'échantillon sanguin

5 ml de l'échantillon de sang ensemencé à 10$^7$ UFC.mL$^{-1}$est dilué avec du PBS (1: 1 de rapport en volume). Le protocole décrit dans la partie C est reproduit et un dénombrement est effectué sur les échantillons collectés dans les orifices de sorties (avec et sans ultrasons). Ce dénombrement est réalisé par ensemencement de milieux chromID®-Salmonella (bioMérieux, France) afin d'estimer le nombre de colonies viables après la préparation de l'échantillon. Les résultats de ces dénombrement sont illustrés sur les figures 16a et 16b. Un ordre de grandeur plus faible de récupération de l'agent pathogène est observé sur les sorties 60a et 60c en raison de l'acoustophorèse. En effet, les tailles des cellules de Salmonella typhimurium (diamètre: 0,7 -1,5 μm et longueur: 2-5 μm) et la taille des globules rouges (ca. 7 μm), font qu'ils sont tous les deux susceptibles d'être concentré par acoustophorèse dans les canal central, vers l'orifice de sortie 60b.

Cet expérience permet cependant d'obtenir une décomplexification forte de l'échantillon, 99,8% des cellules sanguines, et notamment des globules rouges étant rejetés, tout en garantissant un niveau de pathogènes viables et suffisamment concentrés pour être détectable par des techniques conventionnelles. Cette décomplexification permet également une analyse plus aisée des échantillons collectés dans les orifices de sortie 60a et 60c, notamment par cytométrie de flux en ligne, directement à la sortie du dispositif d'acoustophorèse.

## E) séparation par acoustophorèse de billes fluorescentes

Une première solution contenant des billes de 1 μm (référence 17154, Polysciences) à une concentration de 4,55×10$^{10}$ billes/mL est diluée par 1000. Une seconde solution contenant des billes de 10 μm (référence 18140, Polys-ciences) à une concentration de 4,55×10$^7$ billes/mL est diluée par 50. Un mélange de billes est obtenu par mélange 1 :1 de ces solutions diluées afin d'obtenir des concentrations finales dans la solution de 5×10$^7$ billes de 1 μm/mL et 1×10$^6$ billes de 10 μm /mL.

Le mélange de billes de 1 et 10 μm est injecté dans les entrées latérales 30a et 30c d'un dispositif 10 de l'état de l'art, et de l'eau (Suspension Medium bioMerieux) est introduite dans l'orifice central 30b. Le dispositif est utilisé dans un système tel que décrit au point A.

Les échantillons présents dans les sorties 60a et 60c sont mélangés et analysés par un cytomètre. Pour cela, de l'eau (Suspension Médium, bioMerieux) est introduite dans le canal central d'un appareil de cytométrie en flux (Partec), le débit d'analyse du cytomètre est réglé sur 1 μL/min et l'acquisition se fait sur 60s.

Afin de régler le cytomètre pour permettre l'analyse des mélanges de billes : les échantillons de billes sont dilués par 100 avant analyse. Le trigger est réglé sur le signal FL1, pour ne détecter que les éléments fluorescents (et ainsi pouvoir discriminer les billes de 1 μm du bruit de fond). Le cytomètre est ainsi réglé selon les paramètres du tableau 1 suivant.

**Tableau 1 : Valeurs des gains des PMT pour l'analyse des billes au cytomètre**

| FSC | 100 |
|-----|-----|
| SSC | 180 |
| FL1 | 225 |
| FL2 | 350 |

Deux régions peuvent ainsi être délimitées sur les représentations FL1-FSC pour discriminer les billes 1 et 10$\mu$m (voir figure 17).

Le nombre d'éléments détectés dans chacune de ces régions peut ensuite être exporté pour déterminer la composition de l'échantillon. Cette méthode permet également de vérifier et de caler les paramètres du système d'acoustophorèse sans passer par des échantillons biologiques.

La figure 18 illustre ainsi la possibilité de séparer des billes de 1 et 10$\mu$m à l'aide des forces acoustiques. Lorsque le transducteur piézoélectrique n'est pas activé, près de 90% des billes 1 et 10$\mu$m sont récupérées dans les sorties latérales 60a et 60c du dispositif, tandis que lorsqu'il est alimenté par un signal sinusoïdal d'amplitude 1 V crête à crête (Vpp (correspondant à 34V après amplification) et de fréquence 1.44 MHz, les ondes acoustiques transmises dans le canal permettent la séparation des billes 1 et 10$\mu$m : environ 85% des billes de 10$\mu$m sont récupérées dans la sortie centrale 60b alors que 90% des billes de 1$\mu$m sont récupérées dans les sorties latérales 60a et 60c.

Cette exemple permet de valider la capacité de la méthode à décomplexifier des particules fluorescentes de tailles similaires à des microorganismes et des cellules sanguines et à les analyser par cytométrie de flux.

**F) Traitement d'hémocultures inoculées**

Trois bouteilles d'hémoculture BacT/ALERT® (bioMérieux, France) ayant été déclarées comme positives par l'instrument BacT/ALERT® (bioMérieux, France) sont utilisées. Chacune de ces bouteilles contient une espèce de microorganisme, à savoir Citrobacter freundii (cette bouteille a été stockée durant 14 jours à 4°C), Escherichia coli et Enterococcus faecalis.

La concentration de microorganismes présente dans ces hémocultures est d'environ $10^9$UFC/mL. L'hémoculture ensemencée est injectée à 40$\mu$L/mn dans les entrées latérales 30a et 30c d'un dispositif 10 de l'état de l'art, tel que décrit au point A. Du tampon (Suspension Medium 0.85%, bioMerieux) est injecté à 100$\mu$L/mn dans l'entrée centrale 30b. Le dispositif est disposé sur un système tel que décrit au point A.

Les figure 19a (sans excitation du transducteur) et 19b (avec excitation) illustrent l'effet des ondes acoustiques sur la focalisation des globules rouges au centre au canal lorsque le transducteur piézoélectrique est actionné et au débits précités. Cela illustre la possibilité de décomplexifier l'échantillon en éliminant une grande partie des éléments figurés du sang présent dans l'hémoculture.

La figure 20a illustre que sous l'effet des forces acoustiques (cette étape est réalisée à une fréquence d'excitation de 1.51MHz), il est possible de rejeter de près de 95% des cellules sanguines. Néanmoins seulement 20% (36% sur le réplica) des bactéries sont récupérées dans les sorties latérales 60a et 60c. En effet, les bactéries, bien qu'ayant un volume plus faible que les cellules sanguines, subissent aussi les effets des forces acoustiques ainsi que l'effet d'entraînement des hématies et se concentrent en partie au centre du canal.

Tel qu'illustré sur la figure 20b, en augmentant le débit des échantillons en entrée (en passant de 2$\times$20$\mu$L/min à 2x40$\mu$L/min), on limite cette focalisation des bactéries, et on récupère plus de 60% des bactéries dans les canaux latéraux.

Afin d'analyser les hémocultures par cytométrie de flux, celles-ci sont diluées par $10^3$ dans du PBS (solution tampon de phosphatase).

Le cytomètre est réglé selon les paramètres du tableau 2 suivant.

**Tableau 2: Valeurs des gains des PMT pour l'analyse des hémocultures au cytomètre**

| FSC | 150 |
|-----|-----|
| SSC | 257 |
| FL1 | 490 |
| FL2 | 490 |

Les échantillons présents dans les sorties 60a et 60c sont mélangés puis analysés par un cytomètre. Pour cela, de l'eau (Suspension Médium, bioMerieux) est introduite dans le canal central d'un appareil de cytomètrie en flux (Partec), le débit d'analyse du cytomètre est réglé sur 1µL/min et l'acquisition se fait sur 60s.

Le résultat de cette analyse par le cytomètre permet d'observer des régions pouvant ainsi être délimitées pour distinguer les différents composants des hémocultures ensemencées (figure 21). Le nombre d'éléments détectés dans chacune de ces régions peut ensuite être exporté pour déterminer la composition de l'échantillon. Des marqueurs spécifiques des globules rouges et des plaquettes couplés à des fluorophores détectables sur FL1 et FL2 peuvent donc être utilisés pour obtenir une meilleure discrimination entre les éléments figurés du sang et les micro-organismes.

Cet exemple montre que donc que les bactéries sont viables après séparation acoustique, que l'hémoculture peut être traitée sans dilution sans problème majeur de coagulation dans le dispositif ou les connexion fluidiques.

Les débits d'échantillons situés entre 10 et 40µL/mn par entrée pour l'hémoculture et pour 1 V crête à crête (Vpp), soit 34 V crête à crête (Vpp) après amplification, pour le transducteur piézoélectrique permettent de conserver une bonne focalisation des hématies dans le canal.

On constate également qu'à chaque essai d'extraction, la fréquence est légèrement ajustée (+/-50kHz environ autour d'une résonance à 1,44MHz), afin d'optimiser la focalisation des hématie dans le canal de séparation.

## G) Traitement d'hémocultures inoculées

Des ampoules de sang total (10 mL) sont obtenues auprès de l'établissement français du sang. Dans le cas d'une hémoculture négative (i.e. sans contamination artificielle), le sang est introduit dans une bouteille BTA 40mL bacT/Alert (bioMérieux, référence 259789) puis incubé 24h à 35,5°C (homogénéisation sur agitateur « roller »).

Dans le cas d'une hémoculture positive, sont ajoutés en plus du sang 400µL de suspension du microorganisme d'intérêt (suspension en tryptone sel), calibrée à $10^3$ CFU/mL. La bouteille est ensuite incubée à 35,5°C pendant 18h sous homogénéisation au roller pour une culture bactérienne. Pour une culture de levures, la bouteille est incubée à 30°C pendant 36h sous homogénéisation au roller. Ceci permet d'obtenir une concentration en microorganismes équivalente à celle d'une bouteille d'hémoculture positive.

Les trois modèles de micro-organismes étudiés sont les suivants :

- E. coli ATCC 25922 (Gram négatif)

- S. aureus ATCC 29213 (Gram positif)

- C. albicans ATCC 18804 (levure)

Deux pousse-seringues (Pump Elite 11, Harvard Apparatus) sont utilisés de manière indépendante pour réguler les débits en entrée d'un dispositif 10 de l'état de l'art (un pousse-seringue pour les canaux latéraux 30a et 30c et un pousse-seringue pour la voie centrale 30b). L'hémoculture est chargée dans deux seringues plastique Becton Dickinson de 5mL mise en place sur le premier pousse-seringue et le tampon extracteur dans une seringue plastique Becton Dickinson de 25mL dans le second pousse-seringue. Le traitement de l'échantillon est réalisé en utilisant un tampon introduit dans le canal central de sérum physiologique NaCl 0.85% (bioMérieux) ajusté en densité par 1% v/v de Percoll® (Sigma). Ce milieu isotonique et biocompatible garantit la viabilité des cellules focalisées au centre du canal lors de l'étape de séparation par acoustophorèse. Ce tampon est injecté dans l'entrée centrale 30b. Dans le cas où l'isotonie n'est pas souhaitée, le tampon de traitement peut être remplacé par du milieu de suspension (bioMérieux).

L'alimentation peut également être réalisée au moyen d'un contrôleur en pression (Elvesys), l'alimentation des canaux 30a et 30c est alors dédoublée, de sorte qu'elle n'est contrôlée que par un seul étalon de pression.

Le traitement de l'échantillon, également appelé extraction, par acoustophorèse est réalisée selon le protocole suivant :

1) Mouillage de l'ensemble du système fluidique par les solutions de tampon et d'échantillon, à haut débit (2 mL/min - 1mL échantillon, 1mL tampon). Cette étape vise entre autre à évacuer les éventuelles bulles d'air présentes dans le système.

2) Réduction du débit au point d'optimum (60µL/min pour l'échantillon, 70µL/min pour le tampon) et alimentation du transducteur piézoélectrique. Cette étape vise à obtenir une focalisation acoustique optimale en appréciant par exemple de manière visuelle la concentration des hématies dans la sortie centrale.

3) En maintenant l'actuateur alimenté, augmentation du débit de tampon (autour de 2 mL/min) de manière à nettoyer les trois sorties avant traitement / extraction.

4) Réduction du débit de tampon à 70µL/min quand les trois sorties débitent une solution transparente.

L'extraction commence quand la sortie centrale débite visuellement une solution dense rouge.

Dénombrement des espèces en solution :

Trois types de cellules sont à dénombrer : les micro-organismes, les hématies et les plaquettes.

Les micro-organismes sont dénombrés par étalement sur gélose nutritive COS ou SDA10 (bioMérieux). La solution subit une succession de dilutions en milieu de suspension, aboutissant à une concentration de quelques $10^3$ CFU/mL. 50μL sont alors déposés sur la gélose, étalés et laissés à incuber pendant la nuit. Les colonies sont ensuite dénombrées à la main ou de façon automatique.

Les cellules sanguines sont quant à elles dénombrées en cytométrie en flux (Cyflow, Partec). Leur identification précise est assurée par l'adjonction de marqueurs spécifiques « CD41a-PerCP-Vio700 human » pour les hématies (Miltenyi Biotec) et « CD235a - FITC human » pour les plaquettes (Miltenyi Biotec). Sauf exception, 10 μL de chaque marqueur sont mis en contact de 80μL d' échantillon et laissé à l'obscurité pendant 15min avant passage au cytomètre.

L'étape de dénombrement des micro-organismes peut également être réalisée grâce à un cytomètre. Un post-traitement informatique (FCS express) permet alors d'identifier et de dénombrer du même coup les trois populations d'intérêt au moyen de portes d'exclusion et d'inclusion sur les canaux de fluorescence.

Le dénombrement de micro-organismes peut enfin être effectué en mesurant la densité optique de l'échantillon (λ=640 nm) de façon manuelle (Densimat - bioMérieux) ou automatique (lecteur Tecan - Tecan Group Ltd). Ces dénombrements permettent, dans le cas de l'acoustophorèse, la quantification de trois paramètres discriminants :

| Efficacité | Rendement | Pureté |
|---|---|---|
| $\dfrac{^n\text{CFU.périphérique}}{^n\text{CFU.central} + {^n\text{CFU.périphérique}}}$ | $\dfrac{^n\text{CFU.périphérique}}{^n\text{CFU.source} + {^n\text{CFU.périphérique}}}$ | $\dfrac{^n\text{.CFU}}{^n\text{cellules sanguines} + {^n\text{CFU}}}$ |

Par nCFU,périphérique, on entend le nombre total de UFC (unité formant colonie) dénombré à partir des sorties latérales du dispositif d'acoustophorèse. Par nCFU,central, on entend le nombre total de UFC (unité formant colonie) dénombré à partir de la ou des sortie(s) centrale(s) du dispositif d'acoustophorèse. Un des critères de comparaison entre les différentes méthodes d'extraction est la reprise de croissance des micro-organismes post-traitement. Ces derniers sont placés en suspension dans le milieu d'hémoculture (les différentes solutions sont calibrées en densité optique pour partir de concentrations de départ égales), puis dilués par cinq dans du milieu BHI (bioMérieux) avant d'être incubés sous agitation orbitale (300 tpm) à 35,5°C pour les bactéries, 30°C pour les levures. Le dénombrement est effectué heure par heure en cytométrie en flux .

L'identification post-acoustophorèse est réalisée en spectrométrie de masse (Vitek MS - bioMérieux). Brièvement, quatre type de dépôts sont réalisés : échantillon concentré brut, échantillon concentré centrifugé (14000 tpm/10mn), échantillon concentré centrifugé (14000 tpm/10mn) et resuspendue en eau distillée, resuspension centrifugée (14000 tpm/10mn).

Pour les phases liquides, 1μL est déposé sur une cible MALDI, suivi d'1μL de matrice HCCA (bioMérieux). Pour les phases solides, le culot de centrifugation est prélevé à l'aide d'un cône de pipette 0.1 - 10μL et étalé sur cible, suivi d'1μL de matrice. Dans le cas des levures, 0.5μL d'acide formique à 10% est déposé avant l'ajout de matrice HCCA. Une fois préparée, la plaque est introduite dans un appareil Vitek MS (bioMérieux) qui délivre le résultat d'identification ou non de chaque spot.

Pour ce test, seule E. coli est étudiée. Des résultats d'identification par spectrométrie de masse sont notamment obtenus sur E. coli en phase solide (après centrifugation), et ce malgré la complexité de certains dépôts. De plus, la bactérie a également pu être identifiée en phase liquide, dans des concentrations égales à celles d'une sortie brute, justifiant la validité du procédé comme outil de préparation d'échantillon de spectrométrie de masse.

Un test de susceptibilité aux antibiotiques est réalisé au moyen d'un automate VITEK2. Brièvement, 3mL de suspension saline 0.45% (bioMérieux) calibrée à 0.5 Mac Farland sont préparés à partir des micro-organismes traités, et placées dans l'automate (cartes VITEK N°503, bioMérieux).

Pour ce test, seule E. coli est étudiée. Toutefois, l'examen est fait sur une souche sensible (ATCC 25922) à l'un des antibiotiques testés dans l'appareil. Les Concentrations Minimales Inhibitrices (CMI) sont calculées et analysées suivant la base de donnée CAST (Europe) et CLSI (USA). Aucune différence d'interprétation n'est à reporter sur E. coli ATCC 25922 entre la solution extraite par acoustophorèse, la solution extraite par un protocole classique de préparation d'échantillon et un témoin (culture fraîche).

Caractérisation de l'acoustophorèse sur hémoculture ensemencée :

Optimum d'extraction (en débit) sur E. coli :

Le débit optimal de tampon pour une hémoculture ensemencée à E. coli a été déterminé en termes d'efficacité d'extraction. Le débit d'échantillon est fixé à 60μL/min, et la fréquence est ajustée manuellement pour conserver visuellement la focalisation des hématies. Les performances issues des différents débits de tampon sont présentées sur la Figure 22 (extractions sur trois hémocultures différentes, toutes au premier jour après le virage du culot de la bouteille d'hémoculture). Selon la Figure 22, un débit de tampon de 70$\mu$L/min permet une efficacité moyenne d'extraction des microorganismes de 62%, significativement supérieure aux autres points expérimentaux étudiés. Cette efficacité d'extraction sur hémoculture d'E. Coli est largement supérieure aux procédés de l'état de l'art utilisant la technique d'acoustophorèse et ce à partir d'une hémoculture non diluée préalablement à l'introduction dans un dispositif d'acoustophorèse. Suivant ces résultats, le point expérimental [débit échantillon = 60$\mu$L/min ; débit de tampon = 70$\mu$L/min] est adopté comme un point optimum d'extraction sur une hémoculture ensemencée d'E. coli.

Ce réglage permet de plus une grande pureté des canaux périphériques (latéraux). En effet, comme présenté à titre indicatif en Figure 23, plus de 99% des événements détectés correspondent à des bactéries (dénombrement en cytométrie en flux). Très peu de fuites sont observées dans les canaux latéraux, ce qui est confirmé par la faible représentation des cellules sanguines dans le scattergramme de la Figure 23. Il est à noter que ce point expérimental de fonctionnement limite la dilution lors de la séparation, permettant la récupération de sorties latérales concentrées. La concentration en E. Coli est ainsi estimée entre 6,25.10$^8$ et 7,75.10$^8$ UFC/ml.

Avantageusement, l'ajustement de la fréquence peut être résolu par l'emploi d'un système de détection automatique de la résonance du canal permettant la régulation du signal de commande du transducteur piézoélectrique en fonction des variations de température du dispositif.

De la même manière, différents tests de dénombrement en cytométrie en flux sont effectués en modifiant le ratio volumique de Percoll dans le tampon d'extraction au sérum physiologique à 10% v/v, 5% v/v et 1% de v/v. On observe que l'efficacité diminue avec l'augmentation du ratio volumique de Percoll. L'utilisation d'un tampon du sérum physiologique ajusté à 1% (v/v) de Percoll est donc provilégiée.

Reprise de croissance post-extraction :
La reprise de croissance pour les trois espèces étudiées et équivalente voire plus rapide qu'une reprise de croissance suite à un isolement sur boîte classique. Pour chaque solution bactérienne, les micro-organismes subissent une phase de latence d'environ une heure, avant d'entrer en phase exponentielle jusqu'à 4-5h de culture, date à laquelle un plateau commence à être atteint. Pour chaque solution de levures, les micro-organismes subissent une phase de latence bien plus longue que pour les bactéries, allant de 3 à 4 heures, avant d'entrer en phase exponentielle.

Cet exemple montre la capacité du procédé selon l'invention à traiter des échantillons susceptible de contenir de microorganismes et provenant d' hémocultures ainsi que de fluides stériles mis en croissance dans un milieu de culture. Des milieux de cultures aptent à recevoir des fluides corporels stériles peuvent notamment être des bouteilles d'hémocultures. Il est également démontré que le procédé selon l'invention permet de réaliser une étape d'identification et d'antibiogramme sur l'échantillon concentré.

La méthode de traitement selon l'invention permet l'extraction de microorganismes sans utilisation de tampon de lyse sélective et sans dilution de l'échantillon préalablement à son introduction dans un dispositif d'acoustophorèse, pour l'identification et l'antibiogramme et ce quelle que soit la méthode d'identification ou d'antibiogramme utilisée.

La méthode de traitement selon l'invention permet l'utilisation directe d'hémoculture ou de bouteille d' hémoculture positive non diluée, en prélevant par exemple 1mL ou plus dans la bouteille positive pour extraction par le procédé de l'invention.

Il est également démontré qu'un débit de 60$\mu$L/min permet de traiter environ 1mL en 20mn.

Avantageusement un dispositif tel que décrit sur la figure 6b permet d'augmenter le rendement de collection des microorganismes en procédant à deux étapes successives de séparation par acoustophorèse.

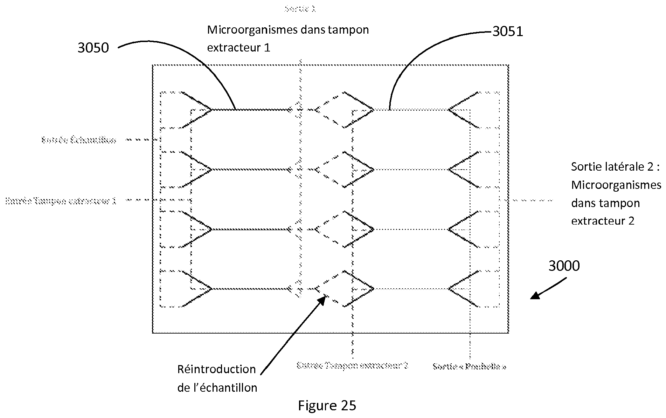

L'invention concerne l'utilisation d'un dispositif comprenant deux canaux successifs de séparation, tel qu'illustré sur les figures 24 et 25. Ces dispositifs peuvent être réalisés de la même manière que les dispositifs précédemment décrits. De même un ou plusieurs transducteurs peuvent être accolés aux dispositifs afin de procéder à deux étapes de séparations par acoustophorèse dans les canaux de séparations successifs.

La figure 24 illustre un dispositif 2000 comprenant deux canaux de séparation successifs 2050, 2051 et permettant le changement du tampon utilisé entre les deux étapes de séparation au sein de ces canaux. Le dispositif 2000 comprend une entrée pour l'échantillon, un entrée pour le tampon dit extracteur, à savoir le tampon utilisé pour la première étape de séparation par acoustophorèse. Il comprend également un première sortie « sortie centrale 1 » permettant la récupération des hématies extraites dans le tampon d'extraction. La première étape de séparation au sein du canal 2050 est donc le siège de l'extraction des microorganismes à proprement parler. Le dispositif comprend également une seconde entrée permettant l'introduction d'un second tampon d'extraction dans le second canal de séparation 2051. Ceci permet notamment la réalisation d'une deuxième étape permettant le changement de tampon d'extraction utilisé, par exemple pour de l'eau purifiée. Les sorties latérales du canal 2050 sont réintroduite dans les entrées latérales du canal de canal 2051. Avantageusement, le canal 2051 est plus long que le canal 2050 afin d'améliorer le rendement de la seconde étape de séparation. Cette seconde permet le transfert des microorganismes du tampon de la première étape de séparation vers le tampon de la seconde étape de séparation. La sortie centrale 2 du canal 2051 permet de récupérer des microorganismes dans de l'eau purifiée. Les sorties latérales permettent de récupérer le tampon extracteur, utilisé dans la première étape de séparation, avec une concentration en microorganismes plus faible qu'à la suite de la première étape de séparation. La succession des étapes de séparations à l'aide de deux tampons différents permet ainsi d'augmenter la concentration d'échantillon préparé et de disposer des microorganismes dans un tampon utilisable directement pour une étape d'identification, par exemple par spectrométrie de masse .

Avantageusement un dispositif tel que décrit sur la figure 6c permet de retraiter la sortie de récupération des microorganismes (provenant des canaux 1370a et 1370c) en concentrant les micro-organismes dans le canal de séparation 1350' afin de recapturer et d'augmenter le rendement de capture dans un tampon adhoc (par exemple de l'eau) permettant une identification par MALDI TOF ou LC-ESI-MS/MS. Le tampon adhoc est pour cela introduit à partir de l'orifice 1330b'. Alternativement, le tampon introduit dans l'orifice 1330b' peut être un tampon permettant une remise en croissance rapide, idéale pour la réalisation d'un antibiogramme.

La figure 25 illustre un dispositif 3000 selon l'invention comprenant deux canaux de séparation successifs 3050, 3051 et permettant le changement du tampon utilisé entre les deux étapes de séparation. La sortie centrale du canal de séparation 3050 est réintroduite dans les entrées latérales du canal 3051. Le dispositif 3000 comprend une entrée pour l'échantillon, un entrée pour le tampon dit extracteur 1, à savoir le tampon utilisé pour la première étape de séparation par acoustophorèse. Il comprend également un première sortie « sortie 1 » permettant la récupération de microorganismes extraits dans le tampon extracteur 1. La première étape de séparation au sein du canal 3050 est donc le siège d'une première étape d'extraction à proprement parler, les microorganismes étant focalisés vers les sorties latérales du canal 3050. Le dispositif 3000 comprend également une seconde entrée permettant l'introduction d'un second tampon d'extraction (tampon d'extraction 2) dans le second canal de séparation 3051. Ceci permet notamment la réalisation d'une deuxième étape d'extraction améliorant le rendement global de l'extraction des microorganismes. Avantageusement, le canal 3051 est plus long que le canal 3050 afin d'améliorer le rendement de la seconde étape de séparation. La sortie centrale du canal 3051 est alors une sortie poubelle et la seconde sortie latérale (sortie latérale 2) permet de récupérer des microorganismes dans de tampon extracteur 2.

Les dispositifs 2000 et 3000 présentent avantageusement plusieurs paires de canaux de séparations successifs tel que décrits et fonctionnant parallèlement, les entrées et sorties d'échantillon et de tampon étant distribuées dans les paires de canaux par des réseaux d'introduction, d'acheminement et d'aspiration tels que décrits précédemment. La parallélisation des séparations successives permet ainsi d'augmenter le volume d'échantillon préparé et de disposer des microorganismes dans un tampon utilisable directement pour une étape d'identification, par exemple par spectrométrie de masse .

Références :

[1] Ai, Y., Sanders, C. K., & Marrone, B. L. (2013). Separation of Escherichia coli bacteria from peripheral blood mononuclear cells using standing surface acoustic waves. Analytical chemistry, 85(19), 9126-9134.

**Revendications**

1. Méthode de traitement d'un échantillon biologique susceptible de contenir une ou des espèce(s) biologique(s) d'intérêt(s) comprenant une étape de décomplexification, ladite étape de décomplexification comprenant les étapes suivantes

   - optionnellement, la réalisation d'une étape d'enrichissement de l'échantillon biologique, préférentiellement par incubation en présence d'un milieu de culture,
   - l'introduction de tout ou partie de cet échantillon dans un premier orifice d'entrée (1330a, 1430a) d'un dispositif d'acoustophorèse (1300, 1400, 2000, 3000),
   - l'introduction d'un tampon dans un second orifice d'entrée (1330b, 1430b) du dispositif d'acoustophorèse
   - lesdits orifices d'entrées étant connectés fluidiquement à au moins deux orifices de sortie (1360a, 1360b, 1360b', 1460a, 1460b, 1460b') par au moins un canal de séparation (1350, 1350', 1450, 1450'),
   - le tampon et l'échantillon étant introduit à des débits respectifs aptes à générer un flux laminaire dans le canal de séparation,
   - la réalisation d'une étape de séparation dudit échantillon biologique par acoustophorèse de manière à favoriser la concentration des particules non-spécifiques présentes dans l'échantillon dans au moins un des orifices de sortie dudit dispositif d'acoustophorèse
   - optionnellement et à la suite de cette étape de décomplexification, la réalisation d'une étape de détermination de la présence, avantageusement d'identification, d'une ou plusieurs espèce(s) biologique(s) dans l'échantillon,
   - optionnellement et à la suite de cette étape de décomplexification et d'une étape d'identification de/des espèce(s) biologique(s), la réalisation d'une étape de détermination du profil d'antibiogramme de(s) l'espèce(s) biologique(s) identifiée(s)

ladite méthode comprenant une seconde étape de décomplexification comprenant la réalisation d'une seconde étape de séparation dudit échantillon concentré par acoustophorèse , au moyen de l'introduction d'un tampon dans un troisième orifice d'entrée (1330b', 1430b') du dispositif d'acoustophorèse, de manière à favoriser un taux de récupération plus important de(s) espèce(s) biologique(s) encore présent(e)s dans l'échantillon concentré dans au moins un des orifices de (1360a, 1360b, 1360b', 1460a, 1460b, 1460b') dudit dispositif d'acoustophorèse,

**caractérisée en ce que** la première étape de séparation est réalisée à l'aide d'un premier tampon et **en ce que** la seconde étape de séparation est réalisée avec un second tampon, de densité différente de celle du premier tampon.

2. Méthode de traitement selon la revendication 1 dans laquelle la seconde étape de décomplexification comprend la réalisation d'une seconde étape de séparation dudit échantillon décomplexifié par acoustophorèse de manière à favoriser un taux de réjection plus important des éléments non-spécifiques, tel que les éléments figurés du sang, présents dans l'échantillon décomplexifié dans au moins un des orifices de sortie dudit dispositif d'acoustophorèse

3. Méthode de traitement selon la revendication 1 ou 2 , **caractérisée en ce que** la seconde étape de séparation est réalisée avec un unique dispositif d'acoustophorèse comprenant au moins deux canaux de séparation successifs

4. Méthode de traitement selon l'une des revendications 1 à 3, **caractérisée en ce que** les tampons utilisés sont de différente nature et/ou de différente densité par rapport à l'échantillon biologique

5. Méthode de traitement selon l'une des revendications 1 à 4 comprenant une étape supplémentaire d'identification de l'espèce biologique suite à l'obtention d'un échantillon enrichi et décomplexifié, préférentiellement par une méthode de spectrométrie de masse

6. Méthode de traitement selon l'une des revendications 1 à 5 comprenant un test d'antibiogramme de l'espèce biologique suite à l'obtention d'un échantillon enrichi et décomplexifié, préférentiellement réalisée en cytométrie de flux, par imagerie précoce des zones inhibitrices obtenues sur milieu de culture à l'aide de disque d'antibiotique ou de bandelette antibiotique, ou par test biochimique

7. Méthode de traitement selon l'une des revendications 1 à 6 comprenant

- la mesure de la densité optique de l'échantillon enrichi et décomplexifié pendant ou à la suite d'une étape de séparation par acoustophorèse de manière à obtenir un échantillon d'une densité optique définie, préférentiellement comprise entre 0,025 et 0,5 Mc Farland, la mesure pouvant être réalisée dans l' orifice de sortie

8. Méthode de traitement selon la revendication 1 à 7 comprenant une étape ultérieure d'incubation de l'échantillon décomplexifié et enrichi, préférentiellement entre 30°C et 37°C, préférentiellement pendant une durée de 1 à 5 heures plus préférentiellement de 2 heures, l'incubation pouvant être réalisée directement dans l'orifice ou le tube de collection de sortie

9. Méthode de traitement selon la revendication 8 comprenant une étape d'agitation de l'échantillon décomplexifié et enrichi durant l'incubation

10. Méthode de traitement selon l'une des revendications 8 ou 9 comprenant la mesure de la densité optique de l'échantillon enrichi et décomplexifié pendant ou à la suite de l'étape d'incubation, préférentiellement après 2 heures d'incubation, de manière à stopper ou prolonger l'étape de d'incubation ou à ajuster la dilution de l'échantillon lorsqu'un seuil de densité optique défini est mesuré, ledit seuil étant préférentiellement compris entre 0,025 et 0,63 Mc Farland, plus préférentiellement entre 0,025 et 0,5 Mc Farland, alternativement entre 0,5 et 0,63 Mc Farland, la mesure pouvant être réalisé dans l' orifice de sortie

11. Méthode de traitement selon la revendication 5, comprenant une étape d'analyse de susceptibilité de l'échantillon à un ou plusieurs antibiotique(s), préférentiellement réalisée en cytométrie de flux, par imagerie précoce des zones inhibitrices obtenues sur milieu de culture à l'aide de disque d'antibiotique ou de bandelette antibiotique, ou par test biochimique, l(es) antibiotique(s) étant déterminé(s) par le résultat de l'étape d'identification de /des espèce(s) de microorganisme(s) présent(es) dans l'échantillon concentré

**12.** Méthode de traitement selon l'une des revendications 1 à 11, tout ou partie de l'échantillon enrichi et décomplexifié étant transféré dans les puits d'une microplaque ou dans différents puits en connexion fluidique avec au moins un des orifices de sortie contenant tout ou partie de l'échantillon, préférentiellement de 3 à 7 puits, chacun des puits pouvant contenir un antibiotique différent et/ou en concentrations différentes

**13.** Méthode de traitement selon la revendication 12, comprenant la réalisation d'une étape d'incubation de l'échantillon enrichi et décomplexifié directement dans la microplaque

**14.** Méthode de traitement selon la revendication 12 comprenant la réalisation d'une étape de d'ajustement de la densité optique de l'échantillon enrichi et décomplexifié directement dans la microplaque

**15.** Méthode de traitement selon l'une des revendications 12 à 14 chacun des puits étant, après un temps d'incubation égal ou supérieur à 1h en présence de(s) (l) antibiotique(s), aspiré successivement pour être analysé en cytométrie de flux

**16.** Méthode de traitement selon la revendication 15, comprenant la détection par l'analyse en cytométrie de flux d'un décalage du signal de fluorescence entre microorganismes sensible, intermédiaire ou résistant aux antibiotiques testés avec l'échantillon enrichi et décomplexifié

**17.** Méthode de traitement selon l'une des revendications 1 à 4 comprenant une étape supplémentaire de lyse mécanique ou enzymatique de l'échantillon décomplexifié, suivi d'une étape d'extraction des acides nucléiques et d'analyse desdits acides nucléiques par PCR, qPCR ou par séquençage

**18.** Méthode de traitement selon l'une des revendications précédentes comprenant une étape de capture des espèces présentes dans l'échantillon concentré à l'aide de particules magnétique suivi d'une séparation par magnétophorèse

**19.** Méthode de traitement selon la revendication 1 ou 2 , **caractérisée en ce que** la première étape de séparation est réalisée à l'aide d'un premier tampon, tel qu'un tampon isotonique et **en ce que** la seconde étape de séparation est réalisée avec un second tampon, distinct du premier tampon et tel qu'un tampon non salin ou adapté à une reprise de croissance rapide du ou des microorganismes

**20.** Méthode de traitement selon la revendication 19, **caractérisée en ce que** le premier tampon est isotonique et comprend du Percoll à un ratio volumique inférieur ou égal à 10% v/v, préférentiellement inférieur ou égal à 5% v/v, plus préférentiellement inférieur ou égal à l%v/v,

**Patentansprüche**

**1.** Verfahren zur Behandlung einer biologischen Probe, die ein oder mehrere biologische Spezies von Interesse enthalten kann, umfassend einen Dekomplexifikationsschritt, wobei der Dekomplexifikationsschritt die folgenden Schritte umfasst

- optional das Ausführen eines Schritts des Anreicherns der biologischen Probe, bevorzugt durch Inkubation in Gegenwart eines Nährmediums,
- Einführen der gesamten oder eines Teils dieser Probe in eine Einlassöffnung (1330a, 1430a) einer Akustophoresevorrichtung (1300, 1400, 2000, 3000),
- Einführen eines Puffers in eine zweite Einlassöffnung (1330b, 1430b) der Akustophoresevorrichtung
- wobei die Einlassöffnungen fluidisch mit mindestens zwei Auslassöffnungen (1360a, 1360b, 1360b', 1460a, 1460b, 1460b') durch mindestens einen Separationskanal (1350, 1350', 1450, 1450') verbunden sind,
- wobei der Puffer und die Probe mit jeweiligen Volumenströmen eingeführt werden, die geeignet sind, einen laminaren Strom im Separationskanal zu erzeugen,
- das Ausführen eines Schritts der Separation der biologischen Probe durch Akustophorese, so dass die Konzentration der in der Probe vorkommenden nicht-spezifischen Partikel in mindestens einer der Auslassöffnungen der Akustophoresevorrichtung begünstigt wird
- optional und nach diesem Dekomplexifikationsschritt das Ausführen eines Schritts des Bestimmens des Vorkommens, vorteilhafterweise des Identifizierens, von einer oder mehreren biologischen Spezies in der Probe,
- optional und nach diesem Dekomplexifikationsschritt und einem Schritt des Identifizierens von einer oder mehreren biologischen Spezies das Ausführen eines Schritts des Bestimmens des Antibiogrammprofils der einen oder mehreren identifizierten biologischen Spezies

wobei das Verfahren einen zweiten Dekomplexifikationsschritt umfasst, der das Ausführen eines zweiten Schritts der Separation der konzentrierten Probe durch Akustophorese mittels des Einführens eines Puffers in eine dritte Einlassöffnung (1330b', 1430b') der Akustophoresevorrichtung umfasst, so dass eine höhere Rückgewinnungsrate der in der konzentrierten Probe noch vorkommenden einen oder mehreren biologischen Spezies in mindestens einer der Öffnungen (1360a, 1360b, 1360b', 1460a, 1460b, 1460b') der Akustophoresevorrichtung begünstigt wird,

**dadurch gekennzeichnet, dass** der erste Separationsschritt mithilfe eines ersten Puffers ausgeführt wird und dass der zweite Separationsschritt mit einem zweiten Puffer ausgeführt wird, dessen Dichte von der des ersten Puffers verschieden ist.

2. Behandlungsverfahren nach Anspruch 1, bei dem der zweite Dekomplexifikationsschritt das Ausführen eines zweiten Schritts der Separation der dekomplexifizierten Probe durch Akustophorese umfasst, so dass eine höhere Ausstoßrate der nicht-spezifischen Elemente begünstigt wird wie etwa der Blutbestandteile, die in der dekomplexifizierten Probe in mindestens einer der Auslassöffnungen der Akustophoresevorrichtung vorkommen.

3. Behandlungsverfahren nach Anspruch 1 oder 2, **dadurch gekennzeichnet, dass** der zweite Separationsschritt mit einer einzigen Akustophoresevorrichtung ausgeführt wird, die mindestens zwei aufeinanderfolgende Separationskanäle umfasst.

4. Behandlungsverfahren nach einem der Ansprüche 1 bis 3, **dadurch gekennzeichnet, dass** die verwendeten Puffer von unterschiedlicher Art sind und/oder in Bezug auf die biologische Probe von unterschiedlicher Dichte sind.

5. Behandlungsverfahren nach einem der Ansprüche 1 bis 4, umfassend einen zusätzlichen Schritt des Identifizierens der biologischen Spezies nach dem Erhalten einer angereicherten und dekomplexifizierten Probe, bevorzugt mit einem Massenspektrometrieverfahren.

6. Behandlungsverfahren nach einem der Ansprüche 1 bis 5, umfassend einen Antibiogrammtest der biologischen Spezies nach dem Erhalten einer angereicherten und dekomplexifizierten Probe, der bevorzugt durch Durchflusszytometrie, durch frühzeitige Bildgebung der auf Nährmedium mithilfe einer Antibiotikascheibe oder eines Antibiotikastreifens erhaltenen Hemmzonen oder durch einen biochemischen Test ausgeführt wird.

7. Behandlungsverfahren nach einem der Ansprüche 1 bis 6, umfassend

   - das Messen der optischen Dichte der angereicherten und dekomplexifizierten Probe während oder nach einem Separationsschritt durch Akustophorese, so dass eine Probe mit einer definierten optischen Dichte erhalten wird, die bevorzugt zwischen 0,025 und 0,5 McFarland beträgt, wobei die Messung in der Auslassöffnung ausgeführt werden kann.

8. Behandlungsverfahren nach Anspruch 1 bis 7, umfassend einen weiteren Schritt der Inkubation der dekomplexifizierten und angereicherten Probe, bevorzugt zwischen 30°C und 37°C, bevorzugt während einer Dauer von 1 bis 5 Stunden, noch bevorzugter von 2 Stunden, wobei die Inkubation direkt in der Auslassöffnung oder dem Auslasssammelrohr ausgeführt werden kann.

9. Behandlungsverfahren nach Anspruch 8, umfassend einen Schritt des Rührens der dekomplexifizierten und angereicherten Probe während der Inkubation.

10. Behandlungsverfahren nach einem der Ansprüche 8 oder 9, umfassend das Messen der optischen Dichte der angereicherten und dekomplexifizierten Probe während oder nach dem Inkubationsschritt, bevorzugt nach 2 Stunden Inkubation, so dass der Inkubationsschritt gestoppt oder verlängert wird oder die Verdünnung der Probe angepasst wird, wenn ein definierter Schwellenwert der optischen Dichte gemessen wird, wobei der Schwellenwert bevorzugt zwischen 0,025 und 0,63 McFarland, noch bevorzugter zwischen 0,025 und 0,5 McFarland, alternativ zwischen 0,5 und 0,63 McFarland beträgt, wobei die Messung in der Auslassöffnung ausgeführt werden kann.

11. Behandlungsverfahren nach Anspruch 5, umfassend einen Schritt des Analysierens der Empfindlichkeit der Probe gegenüber einem Antibiotikum oder mehreren Antibiotika, der bevorzugt durch Durchflusszytometrie, durch frühzeitige Bildgebung der auf Nährmedium mithilfe einer Antibiotikascheibe oder eines Antibiotikastreifens erhaltenen Hemmzonen oder durch einen biochemischen Test ausgeführt wird, wobei das Antibiotikum oder die Antibiotika durch das Ergebnis des Schritts des Identifizierens der einen oder mehreren in der konzentrierten Probe vorkommenden Mikroorganismenspezies bestimmt werden.

**12.** Behandlungsverfahren nach einem der Ansprüche 1 bis 11, wobei die gesamte oder ein Teil der angereicherten und dekomplexifizierten Probe in die Näpfe einer Mikrotiterplatte oder in verschiedene Näpfe transferiert wird, die in Fluidverbindung mit mindestens einer der Auslassöffnungen sind, die die gesamte oder einen Teil der Probe enthalten, bevorzugt 3 bis 7 Näpfe, wobei jeder der Näpfe ein anderes Antibiotikum und/oder ein Antibiotikum in unterschiedlichen Konzentrationen enthalten kann.

**13.** Behandlungsverfahren nach Anspruch 12, umfassend das Ausführen eines Schritt der Inkubation der angereicherten und dekomplexifizierten Probe direkt in der Mikrotiterplatte.

**14.** Behandlungsverfahren nach Anspruch 12, umfassend das Ausführen eines Schritt des Anpassens der optischen Dichte der angereicherten und dekomplexifizierten Probe direkt in der Mikrotiterplatte.

**15.** Behandlungsverfahren nach einem der Ansprüche 12 bis 14, wobei jeder der Näpfe nach einer Inkubationszeit größer oder gleich 1 Stunde in Gegenwart des Antibiotikums oder der Antibiotika nacheinander ausgesaugt wird, um mit Durchflusszytometrie analysiert zu werden.

**16.** Behandlungsverfahren nach Anspruch 15, umfassend die Detektion durch die Analyse mit Durchflusszytometrie einer Verlagerung des Fluoreszenzsignals zwischen Mikroorganismen, die sensibel, intermediär oder resistent gegenüber den Antibiotika sind, die mit der angereicherten und dekomplexifizierten Probe getestet werden.

**17.** Behandlungsverfahren nach einem der Ansprüche 1 bis 4, umfassend einen zusätzlichen Schritt der mechanischen oder enzymatischen Lyse der dekomplexifizierten Probe, gefolgt von einem Schritt der Extraktion der Nukleinsäuren und des Analysierens der Nukleinsäuren durch PCR, qPCR oder durch Sequenzierung.

**18.** Behandlungsverfahren nach einem der vorhergehenden Ansprüche, umfassend einen Schritt des Fangens der in der konzentrierten Probe vorkommenden Spezies mithilfe von magnetischen Partikeln, gefolgt von einer Separation durch Magnetophorese.

**19.** Behandlungsverfahren nach Anspruch 1 oder 2, **dadurch gekennzeichnet, dass** der erste Separationsschritt mithilfe eines ersten Puffers wie etwa eines isotonischen Puffers ausgeführt wird und dass der zweite Separationsschritt mit einem zweiten Puffer ausgeführt wird, der vom ersten Puffer verschieden ist, wie etwa einem nicht salzigen oder für eine schnelle Wiederaufnahme des Wachstums des oder der Mikroorganismen geeigneten Puffer.

**20.** Behandlungsverfahren nach Anspruch 19, **dadurch gekennzeichnet, dass** der erste Puffer isotonisch ist und Percoll in einem Volumenverhältnis kleiner oder gleich 10 % v/v, bevorzugt kleiner oder gleich 5 % v/v, noch bevorzugter kleiner oder gleich 1 % v/v umfasst.

**Claims**

**1.** Method for treating a sample from a blood culture, or a sterile bodily fluid sample likely to contain one or more microorganisms of interest, comprising a decomplexification step, said decomplexification step comprising the following steps :

- Optionally, performing a sample enrichment step by incubation, preferably in the presence of a culture medium,
- introducing all or part of this sample into a first inlet orifice (1330a, 1430a) of a device of acoustophoresis (1300, 1400, 2000, 3000),
- introducing a buffer into a second inlet orifice of the acoustophoresis device
- the said inlet orifices being fluidically connected to at least two outlet orifices (1360a, 1360b, 1360b', 1460a, 1460b, 1460b') by at least one separation channel (1350, 1350', 1450, 1450'),
- the buffer and the sample being introduced at respective flow rates capable of generating a laminar flow in the separation channel,
- the performance of a step of separating said biological sample by acoustophoresis so as to promote the concentration of non-specific particles present in the sample in at least one of the outlet orifices of said acoustophoresis device

- optionally and following the decomplexification step, the performance of a step for determination of the presence and advantageously of identification, of one or more biological species in the said sample,
- optionally and following the decomplexification step and an identification step of biological species , performing a step of determining the antibiogram profile of the identified biological species,

the method comprising a second step of decomplexification comprising carrying out a second step of separation of said sample decomplexified by acoustophoresis by introduction of a buffer in a third inlet (1330b', 1430b') of acoustophoresis device so as to favor a greater rate of recovery of biological species still present in the decomplexified sample in at least one orifices (1360a, 1360b, 1360b', 1460a, 1460b, 1460b') of said acoustophoresis device, wherein the first step of separation is carried out with a first buffer and wherein the second step of separation is carried out with a second buffer having a density different from the one of the first buffer.

2. Treatment method according to claim 1, wherein the second step of decomplexification comprises performing a second step of separation of said decomplexified sample by acoustophoresis so as to favor a greater rate of rejection of non-specific elements, such as the formed elements of blood, present in the decomplexified sample in at least one of the outlet orifices of said acoustophoresis device.

3. Treatment method according to any of the claims 1 or 2, wherein the second step of separation is carried out with a single acoustophoresis device comprising at least two successive separation channels.

4. Treatment method according to any of the claims 1 to 3, wherein the buffers used are of a different nature and/or of different density with respect to the biological sample.

5. Treatment method according to any of the claims 1 to 4, wherein the step of identifying biological species after obtaining an enriched and decomplexified sample is carried out preferably by a mass spectrometry method.

6. Treatment method according to any of the claims 1 to 5, comprising an antibiogram test of biological species following the obtaining of an enriched and decomplexified sample, preferably carried out by flow cytometry, by early imaging of the areas inhibitors obtained on culture medium using an antibiotic disc or antibiotic strip, or by biochemical test

7. Treatment method according to any of the claims 1 to 6, comprising :

- measuring the optical density of the enriched and decomplexified sample during or following a step of separation by acoustophoresis so as to obtain a sample with a determined optical density, preferably between 0.025 and 0.5 Mc Farland, the measurement being able to be carried out in the outlet orifice.

8. Treatment method according to any of the claims 1 to 7, comprising a subsequent step of incubation of the decomplexified and enriched sample, preferably between 30°C and 37°C, preferably for a period of 1 to 5 hours, more preferably 2 hours, the incubation can be carried out directly in the outlet orifice or in the collection tube outlet.

9. Treatment method according to claim 8, comprising a step of agitation of the decomplexified and enriched sample during the incubation.

10. Treatment method according to any of the claims 8 or 9, comprising measuring the optical density of the enriched and decomplexified sample during or following the incubation step, preferably after 2 hours of incubation, so as to stop or prolonging the incubation step or adjusting the sample by dilution when a defined optical density threshold is measured, said threshold preferably being between 0.025 and 0.63 Mc Farland, more preferably between 0.025 and 0.5 Me Farland, alternatively between 0.5 and 0.63 Mc Farland, the measurement being able to be carried out in the outlet orifice

11. Treatment method according to claim 5, comprising a step of analyzing the susceptibility of the sample to one or more antibiotic(s), preferably carried out by flow cytometry, by early imaging of the inhibitory zones obtained on culture medium using an antibiotic disc or antibiotic strip that, or by biochemical test, the antibiotic(s) being determined by the result of the step of identifying the species of microorganism(s) present in the decomplexified sample.

12. Treatment method according to any of the claims 1 to 12, wherein all or part of the enriched and decomplexified sample being transferred into the wells of a microplate or into different wells in fluidic connection with at least one of the outlet orifices containing all or part of the sample, preferably from 3 to 7 wells, each of the wells possibly containing a different antibiotic and/or in different concentrations.

13. Treatment method according to claim 12, comprising carrying out a step of incubating the enriched and decomplexified sample directly in the microplate.

14. Treatment method according to claim 12, comprising performing a step of adjusting the optical density of the enriched and decomplexified sample directly in the microplate.

15. Treatment method according to any of the claims 12 to 14, wherein each of the wells being, after an incubation time equal to or greater than 1 hour in the presence of antibiotic(s), successively aspirated to be analyzed by flow cytometry flow.

16. Treatment method according to claim 15, comprising the detection by flow cytometry analysis of a shift in the fluorescence signal between microorganisms sensitive, intermediate or resistant to the antibiotics tested with the enriched and decomplexified sample.

17. Treatment method according to any of the claims1 to 4, comprising an additional step of mechanical or enzymatic lysis of the decomplexified sample, followed by a step of extraction of the nucleic acids and analysis of the said nucleic acids by PCR, qPCR or by sequencing.

18. Treatment method according to any of the preceding claims, comprising a step of capturing the species present in the concentrated sample using magnetic particles followed by separation by magnetophoresis.

19. Treatment method according to any of the claims 1 or 2, wherein the first separation step is carried out using a first buffer, such as an isotonic buffer and in that the second separation step is carried out with a second buffer, distinct from the first buffer and such as a non-saline buffer or adapted to rapid resumption of growth of the microorganism(s).

20. Treatment method according to claim 19, wherein the first buffer is isotonic and comprises Percoll at a volume ratio less than or equal to 10% v/v, preferably less than or equal to 5% v/v, more preferably less than or equal to 1% v/v.

Figure 1

Coupe A-A

Figure 2a

Lmax

P

Lmin

50

**Coupe B-B**

Figure 2b

Oscilloscope

120

10

110

130a

140a

Echantillon
biologique

130b

Tampon / Milieu

Echantillon
biologique

130c

Générateur
d'ondes

Echantillon
décomplexifié

140b

Particules non
spécifiques

Echantillon
décomplexifié

140c

Détail A

90

Figure 3

Figure 4

Figure 5a – Coupe B-B

230a

P2

P

241a

240a

z

Figure 5b

300

340a

322

370a

330a 330b

370b

340b

350

370c 360b

360a

340c

Figure 6a

Figure 6b

Figure 6c

1400

1470a

1440a

1430a    1430b    1422    1440a'    1470d

1470b    1470f

1440b    1430b'    1440b'    1470e    1460c    1460b    1460a

1450    1440c    1470c    1440c'    1450'    1460b

Figure 6d

370a    2000    300    3100    300

M    370a    Raman ID

2100    M

2000    3000

360c    360b    360a    360c    360b    360a

Figure 7a    Figure 7b

Figure 8

Figure 9

44

Figure 10

840b 840a 840c 850 870b 870a 870c 800

B

B

841

830b

830a

A

A

842

844

860a

860b

843

880

Figure 11

830a 830b 821

841 822

842 823

Figure 12a - Coupe A-A

850 840b 840c 821

840a 822

823

Figure 12b - Coupe B-B

Figure 13a

Figure 13b

Figure 14

Figure 15a

Figure 15b

Figure 16a

Figure 16b

Figure 17

sans pousse-seringue en sortie
piezo OFF
ext : 100µL/min
central : 200µL/min

sans pousse-seringue en sortie
piezo ON
f = 1.44 MHz
V = 1 Vpp
ext : 100µL/min
central : 200µL/min

Figure 18

1040

| | |
|---|---|
| Débit hémoculture | 2x20µL/min |
| Débit tampon | 100µL/min |
| Piezo | OFF |

70a

50

1040

70b

70c

Figure 19a

| | |
|---|---|
| Débit hémoculture | 2x20µL/min |
| Débit tampon | 100µL/min |
| Piezo | Vpp = 1V |
| | f = 1.51MHz |

70a

1040

50

70b

70c

Figure 19b

sans pousse-seringue en sortie
piezo OFF
ext : 20μL/min
central : 100μL/min

sans pousse-seringue en sortie
piezo ON
Vpp = 1V
f = 1.51 MHz
ext : 20μL/min
central : 100μL/min

Fig 20a

sans pousse-seringue en sortie
piezo ON
Vpp = 1V
f = 1.513 MHz
ext : 40μL/min
central : 50μL/min

Fig 20b

Figure 21

Figure 22

Population spécifique
des bactéries

Figure 23

Figure 24

Sortie 1

Microorganismes dans tampon
extracteur 1

3050

3051

Sortie Échantillon

Entrée Tampon extracteur 1

Sortie latérale 2 :
Microorganismes
dans tampon
extracteur 2

3000

Réintroduction
de l'échantillon

Entrée Tampon extracteur 2      Sortie « Poubelle »

Figure 25

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2008122051 A1

**Littérature non-brevet citée dans la description**

- **THOMAS LAURELL et al.** Chip integrated stratégies for acoustic séparation and manipulation of cells and particles. *CHEMICAL SOCIETY REVIEWS,* 01 Mars 2007, vol. 36 (3), ISSN 0306-0012, 492-506
- **PER AUGUSTSSON et al.** Decomplexing biofluids using microchip based acoustophoresis. *LAB ON A CHIP,* 01 Janvier 2009, vol. 9 (6), ISSN 1473-0197, 810-818
- **AI, Y. ; SANDERS, C. K. ; MARRONE, B. L.** Separation of Escherichia coli bacteria from peripheral blood mononuclear cells using standing surface acoustic waves. *Analytical chemistry,* 2013, vol. 85 (19), 9126-9134